(19) Europäisches Patentamt / European Patent Office / Office européen des brevets

(11) EP 3 940 076 A1

(12) **EUROPEAN PATENT APPLICATION**
published in accordance with Art. 153(4) EPC

(43) Date of publication:
**19.01.2022 Bulletin 2022/03**

(21) Application number: **20772639.9**

(22) Date of filing: **03.03.2020**

(51) Int Cl.:
***C12N 15/62*** *(2006.01)* ***C07K 19/00*** *(2006.01)*
***C12N 15/867*** *(2006.01)* ***C12N 5/10*** *(2006.01)*
***A61K 35/17*** *(2015.01)* ***A61P 35/00*** *(2006.01)*

(86) International application number:
**PCT/CN2020/077582**

(87) International publication number:
**WO 2020/187016 (24.09.2020 Gazette 2020/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.03.2019 CN 201910196531**

(71) Applicant: **Asclepius (Suzhou) Technology Company Group Co., Ltd.**
**Suzhou**
**Jiangsu 215123 (CN)**

(72) Inventors:
- **HAN, Kunkun**
  **Suzhou, Jiangsu 215123 (CN)**
- **FU, Jianmin**
  **Suzhou, Jiangsu 215123 (CN)**
- **ZHOU, Min**
  **Suzhou, Jiangsu 215123 (CN)**
- **ZENG, Fanjun**
  **Suzhou, Jiangsu 215123 (CN)**
- **WANG, Qiang**
  **Suzhou, Jiangsu 215123 (CN)**
- **LI, Huashun**
  **Suzhou, Jiangsu 215123 (CN)**

(74) Representative: **ABG Intellectual Property Law, S.L.**
**Avenida de Burgos, 16D**
**Edificio Euromor**
**28036 Madrid (ES)**

(54) **ROBO1 CAR-NK CELL CARRYING SUICIDE GENE, PREPARATION METHOD THEREFOR AND APPLICATION THEREOF**

(57) Provided is a ROBO1 CAR-NK cell carrying a suicide gene, and a preparation method and application thereof. In order to increase the safety and controllability of a CAR-NK therapy, on the basis of a present ROBO1 CAR-NK cell, a suicide gene switch element is integrated into a genome by means of a lentiviral transfection technology to form a CAR-NK carrying a suicide gene. By adding the suicide gene, the CAR-NK cell can be better controlled, and the clinical safety is further improved.

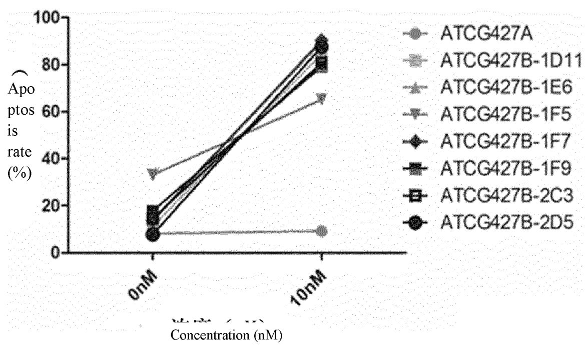

Fig. 9

EP 3 940 076 A1

**Description**

## FIELD OF THE INVENTION

The present invention belongs to the technical field of biopharmaceuticals, and relates to a NK cell and a preparation method and application thereof, and particularly to a ROBO1 CAR-NK cell carrying a suicide gene and a preparation method and application thereof.

## BACKGROUND OF THE INVENTION

In recent years, CAR-T cell-based immunotherapy has received extensive attention in the industry. As a "living" medicament, CAR-T therapy is quite different from traditional medicament. First, this therapy needs to isolate T cells from patients, and uses chimeric antigen receptors (CAR) to modify the T cells in vitro so that the T cells can specifically recognize cancer cells, and then the modified T cells are expanded and reinfused into the patients. For example, the successful application of CD19 CAR-T cells in patients with CD19 positive malignant tumors proves the feasibility of this method for cancer immunotherapy (Grupp et al., 2013). Moreover, CAR-T cells targeting a plurality of different tumor antigens are being actively developed clinically (Kalos et al., 2013).

Although CAR-T therapy has shown great potential, it has obvious limitations. First, the cells have to be isolated from the body (this process is time-consuming and costly). Moreover, because the T cells are modified for specific patients, some patients may not be able to collect the T cells or have insufficient time to wait for the preparation process of the T cells. Although CAR-T is developing towards universal CAR-T, it actually increases clinical risks and operational difficulties. Moreover, because the T cells are modified for specific patients, some patients may not be able to collect the T cells or have insufficient time to wait for the preparation process of the T cells. In addition, faced with the high cost of CAR-T (referring to two listed companies, Novartis and Kite), these limitations may cause some patients who are expected to benefit to fail to accept the CAR-T immunotherapy. In addition, the CAR-T therapy still has serious toxicity or side effects, including cytokine release syndrome CRS, neurotoxicity, and the like.

Natural killer (NK) cells are an important effector cell type for adoptive cancer immunotherapy. Similar to the T cells, the NK cells may be modified to express chimeric antigen receptors (CARs) to enhance anti-tumor activity. The NK cells play an important role in cancer immune surveillance and represent an important effector cell type for adoptive cancer immunotherapy. Compared with the T cells, the NK cells do not need to activate and recognize peptide antigens presented by complex MHC molecules in advance. On the contrary, the NK cells can show killing activity under appropriate stimulation by coupling coded cell surface receptors with a CD3ζ molecules. Therefore, the CAR-NK therapy is expected to become an important development orientation of tumor cell therapy.

By modifying the NK92 cells and loading CAR, the targeting ability of the cells is greatly increased. Meanwhile, a suicide gene switch element is installed to further improve the safety and controllability of the CAR-NK cells. Moreover, pre-clinical experiments also prove that our CAR-NK cells do not have obvious toxic and side effects. However, in order to further increase the safety and effectiveness of the CAR-NK therapy, further research and improvement are needed.

## BRIEF DESCRIPTION OF THE INVENTION

In view of the above technical problems, one object of the present invention is to provide a nucleotide sequence carrying a suicide gene, another object of the present invention is to provide a ROBO1 CAR-NK cell carrying a suicide gene, and one another object of the present invention is to provide an application of the ROBO1 CAR-NK cell carrying the suicide gene, which can better control the CAR-NK cell by adding a suicide gene switch element on the basis of the ROBO1 CAR-NK cell, thus further increasing the clinical safety.

The present invention provides a nucleotide sequence carrying a suicide gene, including a gene encoding a chimeric antigen receptor, and further including a suicide inducing gene, wherein the chimeric antigen receptor includes an antigen binding domain, a transmembrane domain and a costimulatory signal transduction region, the antigen binding domain is capable of specifically binding to a tumor specific antigen, and activating a NK cell through the transmembrane domain and the costimulatory signal transduction region, and the tumor specific antigen is ROBO1.

In a preferred embodiment of the present invention, the suicide inducing gene is iCaspase9, EGFRt, CD20, rapamycin and/or RQR8.

In a preferred embodiment of the present invention, the suicide inducing gene is iCaspase9, the iCaspase9 includes FKBP12-F36V and ΔCaspase9, a nucleotide sequence of the FKBP12-F36V is shown in SEQ ID NO: 1, and a nucleotide sequence of the ΔCaspase9 is shown in SEQ ID NO: 2.

In a preferred embodiment of the present invention, the FKBP12-F36V and the ΔCaspase9 are connected through a Linker, and a nucleotide sequence of the Linker is shown in SEQ ID NO: 3. The design of a Linker sequence is one of the key technologies for the success of a gene fusion technology. Different target genes are connected by appropriate Linker sequences, making the target genes express as a single peptide chain in an appropriate organism, wherein an amino acid which plays a connecting role is called Linker. The Linker does not affect respective functions of target proteins, and enables a fusion protein to form a correct spatial structure and gives better play to biological activity.

In a preferred embodiment of the present invention, the suicide gene iCaspase9 is further provided with a flag gene, and the flag gene is preferably CD19, Myc, Flag, HA or His, and the flag gene is tested to prove whether the target protein is correctly expressed.

In a preferred embodiment of the present invention, the flag gene may be CD19, Myc, Flag, HA, or His, and is preferably CD19; the suicide gene iCaspase9 is further provided with a splicing gene, and the splicing gene is T2A or P2A, and preferably T2A; a nucleotide sequence of the flag gene CD19 is shown in SEQ ID NO: 4, and a nucleotide sequence of the T2A is shown in SEQ ID NO: 5. Through the flag gene CD19, the Caspase9 protein expressed in cells can be recognized. In addition, a flag of the fusion protein after mRNA translation can be successfully spliced from iCaspase9 under the action of the splicing gene. CD19 is a truncated CD19, including an extracellular segment and a transmembrane region of CD19 and an intracellular truncated sequence (excluding an intracellular signal transduction region), which is only used as a label for testing the suicide genes, and has no other functions. Moreover, as a test label of the suicide genes, CD19 has also shown good test results in clinical patients, and has higher safety than other labels. Two genes are connected into one ORF by T2A polypeptide, and become one fusion protein after mRNA translation. However, this fusion protein will be splied into two proteins by a protease recognizing 2A. The two proteins are expressed separately, and the functions thereof do not affect each other. T2A has the advantage of very short sequence, which basically has no influence on protein functions.

In a preferred embodiment of the present invention, the nucleotide sequence of the iCaspase9 is shown in SEQ ID NO: 6.

In a preferred embodiment of the present invention, the suicide inducing gene is EGFRt, and the EGFRt includes an extracellular domain III, an extracellular domain IV and a transmembrane region of EGFR.

In a preferred embodiment of the present invention, a nucleotide sequence of the EGFRt is shown in SEQ ID NO: 12.

In a preferred embodiment of the present invention, the antigen binding domain is capable of specifically binding to one or more of Ig1, Ig2, Ig3, Ig4, Ig5, FN1, FN2 and FN3 domains of the tumor specific antigen ROBO1.

In a preferred embodiment of the present invention, the antigen binding domain is capable of specifically binding to the FN3 domain of the tumor specific antigen ROBO1.

In a preferred embodiment of the present invention, the antigen binding domain is an antibody or an antigen binding fragment thereof specifically binding to the FN3 domain of ROBO1, and the antigen binding fragment is Fab or ScFV.

In a preferred embodiment of the present invention, the transmembrane domain is selected from one or more of CD28, CD3ε, CD45, CD4, CD5, CD8, CD9, CD16, CD22, CD33, CD37, CD134, CD137, ICOS and CD154; and preferably, the transmembrane domain is a CD8 transmembrane domain; and/or,
the costimulatory signal transduction region contains an intracellular domain of a costimulatory molecule, and the costimulatory molecule is selected from one or more of CD3ζ, CD3γ, CD3δ, CD3ε, CD5, CD22, CD79a, CD79b, CD66d, CD2, CD4, CD5, CD28, CD134, CD137, ICOS, CD154, 4-1BB and OX40; and preferably, the costimulatory signal transduction region contains intracellular domains of 4-1BB and CD3ζ.

In a preferred embodiment of the present invention, the chimeric antigen receptor includes a fusion protein with a structure of ScFV-CD8-4-1BB-CD3ζ, and the ScFv is capable of specifically binding to the FN3 domain of the tumor specific antigen ROBO1.

In a preferred embodiment of the present invention, an amino acid sequence of the fusion protein ScFv-CD8-4-1BB-CD3ζ is shown in SEQ ID NO: 8 or SEQ ID NO: 9.

In a preferred embodiment of the present invention, an encoding nucleotide sequence of the fusion protein ScFv-CD8-4-1BB-CD3ζ is shown in SEQ ID NO: 10 or SEQ ID NO: 11.

The present invention further provides a construct carrying a suicide gene, including the nucleotide sequence mentioned above.

In a preferred embodiment of the present invention, the construct is a lentiviral construct; and preferably, when the suicide gene is iCaspase9, a nucleotide sequence of the construct is shown in SEQ ID NO: 7; or
when the suicide gene is EGFRt, the nucleotide sequence of the construct is shown in SEQ ID NO: 13.

The present invention further provides a chimeric antigen receptor carrying a suicide gene, encoded by the above-mentioned nucleotide sequence.

The present invention further provides a ROBO1 CAR-NK cell carrying a suicide gene, wherein the ROBO1 CAR-NK cell expresses the chimeric antigen receptor.

In a preferred embodiment of the present invention, the ROBO1 CAR-NK cell carrying the suicide gene is capable of effectively destroying or killing a lung cancer cell, a pancreatic cancer cell, a hepatoma cell, a breast cancer cell, a colon cancer cell, a prostate cancer cell or a gastric cancer cell.

The present invention further provides a preparation method of the above-mentioned ROBO1 CAR-NK cell carrying the suicide gene, including the following steps of:

(1) synthesizing and amplifying the suicide gene in the above-mentioned nucleotide sequence, and cloning the nucleotide sequence into a lentiviral expression vector to obtain a lentiviral vector carrying the suicide gene; and
(2) packaging a lentivirus through a lentivirus packaging cell line and a three-plasmid system including the lentivirus vector carrying the suicide gene obtained in step (1) to obtain a lentivirus carrying the suicide gene, and then infecting the ROBO1 CAR-NK cell with the lentiviral carrying the suicide gene to integrate the suicide gene into a genome of the ROBO1 CAR-NK cell to obtain the ROBO1 CAR-NK cell carrying the suicide gene.

In a preferred embodiment of the present invention, the ROBO1 CAR-NK cell is prepared through a method including the following steps of:

a. synthesizing and amplifying a nucleotide sequence encoding the chimeric antigen receptor, and cloning the nucleotide sequence into a lentiviral expression vector; and preferably, synthesizing the above-mentioned encoding nucleotide sequence of the fusion protein ScFv-CD8-4-1BB-CD3ζ to obtain a lentiviral vector containing the encoding nucleotide sequence of the fusion protein ScFv-CD8-4-1BB-CD3ζ;
b. packaging through a plasmid required by lentiviral packaging and the lentivirus expression vector obtained in step a in a packaging cell line to prepare a lentivirus; and
c. infecting a NK cell with the lentivirus obtained in step b to obtain the ROBO1 CAR-NK cell.

The present invention further provides a pharmaceutical composition including the above-mentioned ROBO1 CAR-NK cell carrying the suicide gene or the ROBO1 CAR-NK cell carrying the suicide gene prepared by the above-mentioned method; and preferably, the pharmaceutical composition further including an inducer, wherein when the suicide gene is iCaspase9, the inducer is preferably AP1903 or AP20187; and a concentration of the small molecule chemical inducer is 0 nM to 50 nM; or
when the suicide gene is EGFRt, the inducer is preferably cetuximab with an action concentration of 1 μg/ml.
In a preferred embodiment of the present invention, when the ROBO1 CAR-NK cell carrying the suicide gene is used for killing a tumor, an effector-to-target ratio of the ROBO1 CAR-NK cell carrying the suicide gene to a tumour cell is 0.5:1 to 5:1, and the effector-to-target ratio is preferably 0.5:1 to 1:1.
In a preferred embodiment of the present invention, the pharmaceutical composition further includes pharmaceutically acceptable salts, carriers, excipients and adjuvants.
According to some general methods in the technical field of the present invention, the medicament of the present invention may be prepared into various pharmaceutically acceptable dosage forms, such as tablets, capsules, oral liquids, lozenges, injections, ointments, granules or various sustained and controlled release preparations, or the like, and preferably injection, which may be administered by subcutaneous injection.
The carriers of the medicament of the present invention may be common types available in the pharmaceutical field, including adhesives, lubricants, disintegrants, cosolvents, diluents, stabilizers, stabilizers or matrix, and the like.
The present invention further provides an application of the above-mentioned ROBO1 CAR-NK cell carrying the suicide gene or the ROBO1 CAR-NK cell carrying the suicide gene prepared by the above-mentioned preparation method in the preparation of a medicament for treating and/or preventing a cancer; and preferably, the cancer is a tumor with high expression of ROBO1 and related diseases.
In a preferred embodiment of the present invention, the cancer is liver cancer, breast cancer, colon cancer, pancreatic cancer, prostate cancer, gastric cancer, or lung cancer.
In a preferred embodiment of the present invention, the medicament is a medicament in an intratumoral administration form, such as a medicament in an intratumoral injection form or a medicament in an intravenous infusion form.
The present invention further provides a method for treating and/or preventing a cancer by using the above-mentioned ROBO1 CAR-NK cell carrying the suicide gene, or the ROBO1 CAR-NK cell carrying the suicide gene prepared, or the pharmaceutical composition, wherein the method includes administering an effective amount of the pharmaceutical composition containing the ROBO1 CAR-NK cell carrying the suicide gene into a patient.
In a preferred embodiment of the present invention, the cancer is a tumor with high expression of ROBO1 and related diseases.
In a preferred embodiment of the present invention, the cancer is liver cancer, breast cancer, colorectal cancer, pancreatic cancer, prostate cancer, gastric cancer, or lung cancer; and preferably, the cancer is breast cancer, colorectal cancer or liver cancer.
In a preferred embodiment of the present invention, a dosage of the ROBO1 CAR-NK cell carrying the suicide gene is $0.5\times10^9$ cells/times to $5\times10^9$ cells/times.
In a preferred embodiment of the present invention, an administration mode of the ROBO1 CAR-NK cell carrying the suicide gene is intratumoral injection, intravenous injection, intrathoracic injection or local intervention.

In a preferred embodiment of the present invention, the administration mode of the ROBO1 CAR-NK cell carrying the suicide gene is intravenous injection.

The present invention has the beneficial effects that: firstly, the ROBO1 CAR-NK cells may be used as a therapeutic medicament for tumor diseases, used for treating tumors with high expression of ROBO1 molecules, and have no undesirable phenomena such as cytokine storm and the like, thus providing a new treatment method for tumors ineffective by traditional surgery, chemotherapy and radiotherapy; moreover, the targeting ability of the cells is greatly increased by modifying the NK92 cells and loading CAR, and the target in CAR is an anti-tumor target ROBO1, which greatly improves the anti-tumor performance thereof while improving the targeting ability; in addition, upon preclinical experiments, it is found that the toxic and side effects of the ROBO1CAR-NK currently used by us are smaller than those of CAR-T, and the killing effect is better; that is to say, the targeting ability of the ROBO1 CAR-NK cell constructed by the present invention is improved by firstly modifying the NK92 cells and loading CAR; secondly, the NK92 cells are isolated from a peripheral blood of a lymphoma patient and a cell line is successfully established; although the NK92 cells can identify target cells without MHC limitation, can kill tumor cells without pre-sensitization, and can also produce a series of cytokines, so as to regulate the acquired immunity of the body, the ROBO1 CAR-NK also has the function of regulating secondary immunity, but still has some tumor formation risks after being used in a human body.

Therefore, in order to increase the safety and controllability of the CAR-NK therapy, on the basis of the present ROBO1 CAR-NK cell, the suicide gene switch element is integrated into the genome by means of the lentiviral transfection technology to form the CAR-NK carrying the suicide genes with different mechanisms of action. When the small molecule chemical inducer, such as AP1903, is added in vitro, the small molecule chemical inducer can combine with the domain on FKBP12-F36V in the suicide gene, and then form dimeric iCaspase9, thus activating Caspases 3, 6 and 7 and inducing apoptosis; when the inducer cetuximab is added in vitro, the ROBO1 CAR-NK carrying the suicide gene is under the action of killer/effector cells (NK92-CD16, i.e., the NK92 cell line with high expression of CD16 molecule) or PBMC, the cetuximab is combined with EGFRt, and the Fc segment thereof is combined with FcR on the surface of NK92-CD16 or PBMC, thereby initiating antibody-dependent cell-mediated cytotoxicity (ADCC), mediating the killer/effector cells to directly kill the target cells, thus better controlling the CAR-NK cells, further increasing clinical safety and controllability, and promoting the development of the CAR-NK therapy.

## BRIEF DESCRIPTION OF THE DRAWINGS

The present invention can be more clearly understood from the following detailed description with reference to the accompanying drawings, wherein:

Fig. 1 is a schematic structural diagram of a lentiviral plasmid vector pRRLSIN-ScFv (anti ROBO1-FN3) provided in Example 1 of the present invention;

Fig. 2 is a schematic structural diagram of a lentiviral plasmid vector (1942-3-IC9) carrying a suicide gene iCaspase9 in Example 4 of the present invention;

Figs. 3a-3b show results of CAR positive rates of ROBO1 CAR-NK cells by flow cytometry provided in Example 3 of the present invention;

Figs. 4a-4b show results of CD56 molecule positive rates of the ROBO1 CAR-NK cells using flow cytometry provided in Example 3 of the present invention;

Fig. 5 is a diagram showing iCaspase9 and ROBO1 positive rates of the ROBO1 CAR-NK cells carrying a suicide gene using flow cytometry provided in Example 4 of the present invention, wherein PE is a channel for testing Flag, and the positive rate in the Flag-PE channel indicates the result of the positive rate of ROBO1 CAR, APC is a channel for testing CD19, the positive rate in the APC-CD19 channel indicates the result of the positive rate of iCaspase9, Flag/CD19 indicates the double positive result tested, and the positive rate of the double positive indicates the result of the positive rate of simultaneous expression of iCaspase9 and ROBO1;

Fig. 6 shows a test result of an mRNA level of the ROBO1 CAR-NK cell carrying the suicide gene provided in Example 4 of the present invention by RT-PCR, wherein 1 is a NK-92 cell, 2 is a ROBO1 CAR-NK cell, 3 is a ROBO1-iCaspase9 cell, and iCas9 in the figure indicates iCaspase9;

Fig. 7 shows a whole genome sequencing result of a ROBO1 CAR-NK cell carrying a suicide gene in Example 5 of the present invention, and the result is the analyzed information of inserted genome sites;

Fig. 8 shows a stability test result of a ROBO1 CAR-NK cell carrying a suicide gene in Example 6 of the present invention;

Fig. 9 shows a result of inducing apoptosis of the ROBO1 CAR-NK cell carrying the suicide gene in Example 6 of the present invention with AP1903;

Fig. 10a shows a result of a positive rate of the ROBO1 CAR-NK cell ATCG427B-1F7 carrying the suicide gene by flow cytometry in Example 6 of the present invention;

Fig. 10b shows a result of a positive rate of the ROBO1 CAR-NK cell ATCG427B-2D5 carrying the suicide gene by flow cytometry in Example 6 of the present invention;

Fig. 11 shows a result of inducing apoptosis of a ROBO1 CAR-NK cell carrying a suicide gene in Example 7 of the present invention with different concentrations of AP1903;

Fig. 12 shows a result of counting a cell survival rate after the cells treated with different concentrations of AP1903 for 2 hours, 4 hours and 24 hours are continuously cultured in fresh media for three days in Example 7 of the present invention;

Fig. 13 is a result of culturing an experimental group treated for 4 hours in Example 7 of the present invention in a fresh medium for 24 hours.

Fig. 14a shows test results of ROBO1 protein expression in different breast cancer cells in Example 8 of the present invention, wherein HCC1187, HCC1937, HCC38, MDAMB-231, MDAMB-453 and MDAMB-468 are triple-negative breast cancer cell lines;

Fig. 14b shows a result of a ratio of an optical density of ROBO1 to an optical density of GAPDH determined and tested in Fig. 14a;

Fig. 15 shows a killing result of the ROBO1 CAR-NK cell carrying a suicide gene in Example 9 of the present invention used for breast cancer cells;

Figs. 16a-c show killing results of the ROBO1 CAR-NK cell carrying the suicide gene in Example 9 of the present invention used for different cancer cells;

Fig. 17a shows a test result of ROBO1 expression in a normal breast cell and PBMC in Example 9 of the present invention;

Fig. 17b shows an experimental result of killing the normal breast cell and PBMC by the ROBO1 CAR-NK cell carrying the suicide gene in Example 9 of the present invention;

Fig. 18 shows a killing result of PBMC on the ROBO1 CAR-NK cell carrying the suicide gene in Example 9 of the present invention;

Fig. 19 is is a schematic structural diagram of a lentiviral plasmid vector 1942-3-hEGFRt provided in Example 10 of the present invention;

Fig. 20 is a schematic structure diagram of EFGRt in Example 10 of the present invention;

Fig. 21a shows a result of a positive rate of the ROBO1 CAR-NK cell ROBO1-EGFRt-3C10-2D10 carrying the suicide gene using flow cytometry in Example 10 of the present invention;

Fig. 21b shows a result of a positive rate of the ROBO1 CAR-NK cell ROBO1-EGFRt-3C10-1F11 carrying the suicide gene using flow cytometry in Example 10 of the present invention;

Fig. 22 shows test results of ROBO1 protein expression levels in different types of cancer cells in Example 11 of the present invention;

Fig. 23a is an experimental diagram showing killing results of the ROBO1 CAR-NK cell carrying the suicide gene in Example 11 of the present invention for killing experiments;

Fig. 23b is an experimental diagram for determining specific killing results of the ROBO1 CAR-NK cell carrying the suicide gene in Example 11 of the present invention for killing experiments by using a ROBO1-overexpressed monoclonal cell strain (HCT116-ROBO1) model;

Fig. 23c is an experimental diagram for determining specific killing results of the ROBO1 CAR-NK cell carrying the suicide gene in Example 11 of the present invention for killing experiments by using a ROBO1-overexpressed monoclonal cell strain (MDAMB-231-ROBO1) model;

Fig. 24 is an experimental result diagram of evaluating effectiveness of a suicide switch in the ROBO1-EGFRt-CAR-NK cell carrying a suicide gene by a NK92-CD16 (high affinity) effector cell constructed in vitro in Example 12 of the present invention; and

Fig. 25 is an experimental result diagram of evaluating the effectiveness of the suicide switch in the ROBO1-EGFRt-CAR-NK cell carrying the suicide gene by a PBMC effector cell in Example 12 of the present invention;

wherein, ATCG427A-2h, ATCG427A-4h and ATCG427A-24h respectively represent results of inducing the ATCG427A with AP1903 for 2 hours, 4 hours and 24 hours; ATCG427B-1F7-2h, ATCG427B-1F7-4h and ATCG427B-1F7-24h respectively represent results of inducing the ATCG427B-1F7 with AP1903 for 2 hours, 4 hours and 24 hours respectively; while ATCG427B-2D5-2h, ATCG427B-2D5-4h and ATCG427B-2D5-24h respectively represent results of inducing the ATCG427B-2D5 with AP1903 for 2 hours, 4 hours and 24 hours; and

T47D-2h, HCC1187-2h, MCF-2h, HepG2-2h, Huh7-2h, HCT116-2h, HCT116-Flag.ROBO1.Full-1C11-2h, HCT116-Flag.ROBO1.Full-1D11-2h, MDAMB-231-2h, MDAMB-231-Flag.ROBO1.Full-1C11-2h, and MDAMB-231-Flag.ROBO1.Full-1D11-2h all indicate the corresponding cancer cells that are induced to kill for 2 hours, wherein HCT116-Flag.ROBO1.Full-1C11-2h and HCT116-Flag.ROBO1.Full-1D11-2h respectively indicate of being induced to kill for 2 hours by different clones of the ROBO1-overexpressed HCT116-ROBO1 model; while MDAMB-231-Flag.ROBO1.Full-1C11-2h and MDAMB-231-Flag.ROBO1.Full-1D11-2h respectively represent of being induced to kill for 2 hours by different clones of the ROBO1-overexpressed MDAMB-231-ROBO1 model.

## DETAILED DESCRIPTION OF INVENTION

Various exemplary examples of the present invention will now be described in detail with reference to the accompanying drawings. It should be noted that the parameter values of the methods and steps illustrated in these examples do not limit the scope of the present invention unless otherwise specified.

The following description of at least one exemplary example is merely illustrative in nature and in no way serves as any limitation on the present invention and application or use thereof.

Techniques and methods known to those of ordinary skills in the related arts may not be discussed in detail, but under appropriate circumstances, such techniques and methods should be regarded as a part of the specification.

Unless otherwise specified, "NK cells" herein include "peripheral blood NK cells, NK92 cells and other NK cells".

Unless otherwise specified, the "NK-92 cells" and "NK cells" herein share the same meaning.

Unless otherwise specified, "ROBO1 CAR-NK" herein refers to "a chimeric antigen receptor cell targeting ROBO1, especially an enhanced CAR-T cell and CAR-NK cell targeting ROBO1", and the English names appearing herein are not case-sensitive, such as ROBO1, Robo1, robo1, and the like, which share the same meaning. Robo1 CAR-NK and Robo1-CAR NK share the same meaning, the specific preparation processes of which are referred to the following examples.

Unless otherwise specified, "ATCG427A" herein refers to "ROBO1 CAR-NK cell" or "ROBOIMCAR-NK".

Unless otherwise specified, "iCaspase9" and "iCas9" herein both refer to "suicide inducing gene Caspase9".

Unless otherwise specified, "ROBO1-iCaspase9 CAR-NK cell" herein refers to "ROBO1 CAR-NK cell carrying suicide gene".

Unless otherwise specified, "ATCG427B" herein refers to "ROBO1 CAR-NK cell carrying suicide gene iCaspase9, i.e., ROBO1-iCaspase9 CAR-NK cell".

Unless otherwise specified, "FKBP12" herein refers to "FKBP12-F36V".

Unless otherwise specified, "CMV" herein refers to "CMV promoter".

Unless otherwise specified, "ATCG427E" herein refers to "ROBO1 CAR-NK cell carrying suicide gene EGFRt, i.e., ROBO1-EGFRt CAR-NK cell".

Unless otherwise specified, "ATCG427A-KO" herein refers to "knocking out the gene sequence of a ROBO1-scFV target in CAR in the form of Crisp Cas9 gene editing, keeping other signal transduction sequences, and then knocking the CAR into a NK92 cell, that is, the NK cell in which the CAR sequence without the ROBO1 target is knocked on the same genome insertion site as CAR in 427A".

Unless otherwise specified, "NK" described in the present invention is a human normal NK cell or NKT cell, or NK cell line, including NK92 cell, YT cell, NKL cell, HANK-1 cell, NK-YS cell, KHYG-1 cell, SNK-6 cell, IMC-1 cell, and the like". In the specific embodiment of the present invention, NK-92 cell is taken as an example.

Unless otherwise specified, "vector" herein is a physical composition, which includes isolated nucleic acids, and can be used to transfer isolated nucleic acids into a cell. Many vectors are known in this field, including, but not limited to, linear polynucleotides, and the polynucleotides related to ions or amphiphilic compounds, plasmids and viruses. Therefore, the term "vector" includes autonomously replicating plasmids or viruses. The term should also be interpreted as including non-plasmid and non-viral compounds that facilitate transferring nucleic acids into cells, such as polylysine compounds, liposomes and the like. Examples of viral vectors include, but not limited to, adenovirus vectors, adeno-associated virus vectors, retroviral vectors, and so on.

Unless otherwise specified, "antibody" herein includes a whole antibody and any antigen binding fragment (i.e., "antigen binding part") or single chain thereof.

Unless otherwise specified, all reagents herein are analytical-grade pure and may be purchased from regular channels.

Material sources in the following examples:

NK-92 cell (CC® CRL-2407), breast cancer cells BT474, T47D, HCC1187, HCC1937, MCF7, MDAMB-231, MDAMB-453, MDAMB-468 and ZR-75-1, lung cancer cells A549 and H1299, hepatoma cells Huh7, SMMC7721 and HEPG2, pancreatic cancer cells BxPC3, PANC1 and Capan-2, and Lenti-X-293T cell, and colorectal cancer cells such as HT-29, LoVo and HCT116 were all purchased from the Cell Resource Center of Shanghai Institutes for Biological Sciences, Chinese Academy of Sciences.

### **Example** 1. Preparation of lentiviral vector

A sequence of a ScFV (Anti ROBO1-FN3)-CD8(CD8TM)-4-1BB-CD3ζ fusion gene (with an amino acid sequence shown in SEQ ID NO: 8 and a gene sequence shown in SEQ ID NO: 10) and a sequence of a mutant ScFV (Anti ROBO1-FN3)-CD8(CD8TM)-4-1BB-CD3ζ fusion gene (with an amino acid sequence shown in SEQ ID NO: 9, a gene sequence shown in SEQ ID NO: 11, and a mutation site of GCC or GCG) were synthesized separately. The ScFV (Anti ROBO1-FN3)-CD8TM-4-1BB-CD3ζ fusion gene was taken as an example to illustrate the preparation process of ROBO1 CAR-NK cells, while the preparation process of ROBO1M CAR-NK cells using the mutant ScFV (Anti ROBO1-FN3)-CD8TM-4-1BB-CD3ζ fusion gene was identical.

The sequence of the ScFV (Anti ROBO1-FN3)-CD8-4-1BB-CD3ζ fusion gene was transformed and ligated into a pRRLSIN vector by enzyme digestion, wherein the upstream of the gene was EP-Iα promoter. The vector was transformed into Stbl3 Escherichia coli strain, which was screened by ampicillin to obtain positive clones. Then plasmid was extracted and the clone was identified by enzyme digestion, so that a pRRLSIN-ScFv (anti ROBO1-FN3) lentiviral transfection vector was obtained (as shown in Fig. 1).

Example 2. Preparation of lentiviral

(1) 24 hours before transfection, Lenti-X-293T cells were inoculated into 15cm petri dishes at about $8\times10^6$ per dish. It was ensured that the convergence degree of the cells was about 80% and the cells were evenly distributed in the petri dishes during the transfection.
(2) Preparation of solution A and solution B

Solution A: 6.25 mL of 2×HEPES buffer (the effect was better when the quantity was a package of 5 dishes together)
Solution B was a mixture by adding the following plasmids respectively: 112.5 μg of pRRLSIN-ScFv (anti ROBO1-FN3) (target plasmid); 39.5 μg of pMD2.G (VSV-G envelop); 73 μg of pCMVR8.74 (gag, pol, tat and rev); and 625 μl of 12M calcium ion solution. The total volume of the solution B was 6.25 mL.

Solution B was mixed completely, and dropwise added into solution A while gently swirling solution A, so that a mixture of solution A and solution B was obtained, which was then rested to stand for 5 minutes to 15 minutes. The mixture of the above-mentioned solution A and solution B was gently swirled and dropwise added into petri dishes containing Lenti-X-293T cells, with 2.5 ml per dish. The obtained petri dish was gently shaken back and forth to evenly distribute a mixture of DNA and calcium ions. Then the petri dish (without being rotated) was incubated for 16 hours to 18 hours in an incubator. A fresh medium was changed and the cultivation was continued. The supernatants containing virus were collected after 48 hours and 72 hours separately. The supernatants were centrifuged at 500g for 10 minutes at 25°C and then filtered by PES membrane (0.45 μm). The filtered supernatants containing lentivirus were transferred into ultra-clear virtus centrifuge tubes. The volume of the supernatants in each tube did not exceed 2/3 of the tube volume, and the supernatants were centrifuged at 25000 rpm at 4°C for 2 hours. The centrifuge tubes were taken out carefully, and the supernatants were poured out, and then the centrifuge tubes were inverted to remove the residual liquid. Then the centrifuge tubes were added by 100 μl of fresh medium, sealed, stood at 4°C for 2 hours, swirled gently every 20 minutes, and finally centrifuged at 500g for one minute (25°C) to collect the supernatant containing virus. The collected supernatant containing virus was cooled on ice and then stored at -80°C.

**Example 3. Preparation of ROBO1 CAR-NK cells**

The density of the NK-92 cells was adjusted to $2\times10^5$/ml to $3\times10^5$/ml. The virus obtained from Example 2 was added to the NK-92 cells according to a volume ratio (V/V) of virus: cell culture medium = 1:5, while 8 μg/mL polyamine was added at the same time. 4 hours later, equivalent amount of fresh complete medium was added to adjust the cell density to $1\times10^5$/ml for further cultivation. The next day, all the cells were centrifuged and added with fresh medium for further cultivation. Fresh medium was added every 1 to 2 days to maintain the cell density at $2\times10^5$/ml to $3\times10^5$/ml. CAR antibody staining was performed after 72 hours, and ROBO1 CAR NK-92 positive cells were sorted by flow cytometry for culture expansion. The color change of the medium, cell density and cell morphology were observed daily and recorded accordingly.

After sorting the cells by flow cytometry, positive ROBO1 CAR NK-92 cells were continuously cultured in a GMP workshop, and then expanded to the required measurement for clinical use. After that, the cells were centrifugated and washed thrice (by PBS solution), then the obtained ROBO1 CAR-NK 92 cells were resuspended in normal saline for clinical treatment.

Before clinical treatment, the quality of ROBO1 CAR-NK 92 cells was tested with reference to the test methods of Pharmacopoeia to guarantee the safety of the cells. The test results are shown in Table 1.

Table 1 Quality test of ROBO1 CAR-NK 92 cells

| Release parameters | GMP release criteria | Actual test results |
|---|---|---|
| Sterility test (liquid cultivation) | Negative | Negative |
| Sterility test (gram staining) | Negative | Negative |
| Cell viability (trypan blue staining) | >95% | 98% |
| Endotoxin (limulus reagent) | <5EU/kg/h | <5EU/kg/h |
| CAR positive rate (flow cytometry) | >90% | 96.31% (shown in Figs. 3a-b) |
| Mycoplasma DNA (PCR method) | Negative | Negative |
| CD56+ (flow cytometry) | Positive | Positive (shown in Figs. 4a-b) |

The positive rate of the CAR NK-92 cells was tested by flow cytometry, and the flow cytometry results were shown in Fig. 3a and Fig. 3b. In Fig. 3a and Fig. 3b, the antibodies used were shown as APC fluorescence label and represented on the abscissa. If the NK-92 cells successfully expressed CAR molecules, the signal value would increased significantly. It could be seen from Fig. 3a and Fig. 3b that the signal value of the APC fluorescence label increased significantly, indicating that the CAR molecules were successfully expressed by the NK-92 cells. The positive rate of CAR-NK92 was 98.89%.

Fig. 4a and 4b showed results of positive rates of CD56 molecules of ROBO1 CAR-NK by flow cytometry. Fig. 4a was the control group, and Fig. 4b was the experimental group. It could be seen from Fig. 4a and 4b that the CD56 molecule was positive, indicating that the prepared CAR-NK92 cells did not lose the CD56 molecules, and there were no other forms of differentiation, which means the basic characteristics of the NK cells had been preserved.

ROBOIM CAR-NK cells were prepared in the same way.

**Example 4. Preparation of ROBO1-iCaspase9 CAR-NK cells**

Suicide gene iCaspase9 [SEQ ID NO: 6: ATGGGAGTGCAGGTGGAAACCATCTCCCCAGGAGACGGGCGCACCTTCCCCAAGCG CGGCCAGACCTGCGTGGTGCACTACACCGGGATGCTTGAAGATGGAAAGAAAGTTG ATTCCTCCCGGGACAGAAACAAGCCCTTTAAGTTTATGCTAGGCAAGCAGGAGGTGA TCCGAGGCTGGGAAGAAGGGGTTGCCCAGATGAGTGTGGGTCAGAGAGCCAAACTG ACTATATCTCCAGATTATGCCTATGGTGCCACTGGGCACCCAGGCATCATCCCACCACA TGCCACTCTCGTCTTCGATGTGGAGCTTCTAAAACTGGAA (FKBP12-F36V: SEQ ID NO: 1) *TCCGGAGGAGGATCCGGAGTCGAC* (Linker: SEQ ID NO: 3)

GGATTTGGTGATGTCGGTGCTCTTGAGAGTTTGAGGGGAAATGCAGATTTGGCTTACA TCCTGAGCATGGAGCCCTGTGGCCACTGCCTCATTATCAACAATGTGAACTTCTGCCG TGAGTCCGGGCTCCGCACCCGCACTGGCTCCAACATCGACTGTGAGAAGTTGCGGCG TCGCTTCTCCTCGCTGCATTTCATGGTGGAGGTGAAGGGCGACCTGACTGCCAAGAA AATGGTGCTGGCTTTGCTGGAGCTGGCGCAGCAGGACCACGGTGCTCTGGACTGCTG CGTGGTGGTCATTCTCTCTCACGGCTGTCAGGCCAGCCACCTGCAGTTCCCAGGGGC TGTCTACGGCACAGATGGATGCCCTGTGTCGGTCGAGAAGATTGTGAACATCTTCAAT GGGACCAGCTGCCCCAGCCTGGGAGGGAAGCCCAAGCTCTTTTTCATCCAGGCCTGT GGTGGGGAGCAGAAAGACCATGGGTTTGAGGTGGCCTCCACTTCCCCTGAAGACGA GTCCCCTGGCAGTAACCCCGAGCCAGATGCCACCCCGTTCCAGGAAGGTTTGAGGAC CTTCGACCAGCTGGACGCCATATCTAGTTTGCCCACACCCAGTGACATCTTTGTGTCC TACTCTACTTTCCCAGGTTTTGTTTCCTGGAGGGACCCCAAGAGTGGCTCCTGGTACG TTGAGACCCTGGACGACATCTTTGAGCAGTGGGCTCACTCTGAAGACCTGCAGTCCC TCCTGCTTAGGGTCGCTAATGCTGTTTCGGTGAAAGGGATTTATAAACAGATGCCTGG TTGCTTTAATTTCCTCCGGAAAAAACTTTTCTTTAAAACATCA (ΔCasepase9: SEQ ID NO: 2)

GAATTCGGCAGTGGAGAGGGCAGAGGAAGTCTGCTAACATGCGGTGACGTCGAGGA GAATCCTGGCCCA (T2A: SEQ ID NO: 5)

ATGCCACCTCCTCGCCTCCTCTTCTTCCTCCTCTTCCTCACCCCCATGGAAGTCAGGC CCGAGGAACCTCTAGTGGTGAAGGTGGAAGAGGGAGATAACGCTGTGCTGCAGTGC CTCAAGGGGACCTCAGATGGCCCCACTCAGCAGCTGACCTGGTCTCGGGAGTCCCCG CTTAAACCCTTCTTAAAACTCAGCCTGGGGCTGCCAGGCCTGGGAATCCACATGAGG CCCCTGGCCATCTGGCTTTTCATCTTCAACGTCTCTCAACAGATGGGGGGCTTCTACC TGTGCCAGCCGGGGCCCCCCTCTGAGAAGGCCTGGCAGCCTGGCTGGACAGTCAAT GTGGAGGGCAGCGGGGAGCTGTTCCGGTGGAATGTTTCGGACCTAGGTGGCCTGGG CTGTGGCCTGAAGAACAGGTCCTCAGAGGGCCCCAGCTCCCCTTCCGGGAAGCTCAT GAGCCCCAAGCTGTATGTGTGGGCCAAAGACCGCCCTGAGATCTGGGAGGGAGAGC CTCCGTGTCTCCCACCGAGGGACAGCCTGAACCAGAGCCTCAGCCAGGACCTCACCA TGGCCCCTGGCTCCACACTCTGGCTGTCCTGTGGGGTACCCCCTGACTCTGTGTCCAG GGGCCCCCTCTCCTGGACCCATGTGCACCCCAAGGGGCCTAAGTCATTGCTGAGCCT AGAGCTGAAGGACGATCGCCCGGCCAGAGATATGTGGGTAATGGAGACGGGTCTGTT GTTGCCCCGGGCCACAGCTCAAGACGCTGGAAAGTATTATTGTCACCGTGGCAACCT GACCATGTCATTCCACCTGGAGATCACTGCTCGGCCAGTACTATGGCACTGGCTGCTG AGGACTGGTGGCTGGAAGGTCTCAGCTGTGACTTTGGCTTATCTGATCTTCTGCCTGT GTTCCCTTGTGGGCATTCTTCATCTTCAAAGAGCCCTGGTCCTGAGGAGGAAAAGAA AGCGAATGACTGACCCCACCAGGAGATTCTTCAAAGTGACGCCTCCCCCAGGAAGC

GGGTGA (CD19: SEQ ID NO: 4)] was synthesized, and then cloned into a lentiviral vector to obtain a lentiviral plasmid vector (1942-3-IC9) (shown in Fig. 2, with a nucleotide sequence shown in SEQ ID NO: 7) carrying the suicide gene iCaspase9, and then the virus was packaged by a three-plasmid system (aided by plasmids pMD2.G and pCMVR8.74) (a nucleotide sequence of the packaged virus was shown in SEQ ID NO: 7). Then, the ROBO1 CAR-NK cells (or ROBO1MCAR-NK cells, wherein the ROBO1MCAR-NK cells were used in the following examples) prepared in Example 3 were infected by means of virus infestation, and then the ROBO1 and iCaspase9 double-positive cells, i.e., the ROBO1 CAR-NK cells (ROBO1-iCaspase9 CAR-NK cells) carrying the suicide gene were sorted by flow cytometry. The screened cells were cultured, and then the positive rate of the ROBO1 CAR-NK cells carrying the suicide gene was tested from the cultured cells by flow cytometry. The results were shown in Fig. 5. It could be seen from the figure that the signal values of APC and PE fluorescence label were significantly increased, and the ROBO1 CAR-NK cells carrying the suicide genes (i.e., ROBO1-iCaspase9 CAR-NK cells) were successfully obtained.

In addition, the RNA of the cultured ROBO1-iCaspase9CAR-NK cells was extracted, and a mRNA level of the suicide gene fragment was tested by RT-PCR. Meanwhile, the NK-92 cells and the ROBO1 CAR-NK cell were used as controls, and the results were shown in Fig. 6, which showed that the ROBO1 CAR-NK cells (ROBO1-iCaspase9CAR-NK cells) carrying the suicide gene may be verified at the mRNA level.

**Example 5. Sequencing of ROBO1-iCaspase9 CAR-NK cells**

Whole genome sequencing was carried out on the ROBO1-iCaspase9CAR-NK cells prepared in Example 4, and the insertion sites of the ROBO1-iCaspase9CAR-NK cells in the genome of the NK-92 cell were tested. The sequencing results were shown in Fig. 7. After PCR verification, the sequencing results showed that No. 2 chromosome was inserted into the gene iCaspase9 and No. 10 chromosome was inserted into the gene ROBO1.

**Example 6. Stability screening of ROBO1-iCaspase9 CAR-NK cells**

The ROBO1-iCaspase9CAR-NK cells constructed in Example 4 were cultured. The ROBO1-iCaspase9CAR-NK cells of different clones were respectively named ATCG427B-1D11, ATCG427B-1E1, ATCG427B-1F5, ATCG427B-1F7, ATCG427B-1F9, ATCG427B-2C3 and ATCG427B-2D5. Then, the positive rates of flag antibody Flag and CD19 in these cells were tested by flow cytometry (specifically, the cells were stained with flow antibodies Flag and CD19 in dark for 15 minutes, then washed twice with PBS, and tested on the machine), which were used as a cell stability index. The results were shown in Fig. 8.

Different amounts of medicament AP1903 were respectively added into the ROBO1-iCaspase9CAR-NK cells prepared in Example 4 (the final concentration of the medicament AP1903 was 0 nM and 10 nM) for 4 hours to test the effect thereof on apoptosis of the ROBO1-iCaspase9CAR-NK cells. The results were shown in Fig. 9.

It could be seen from the above Figs. 8-9 that ATCG427B-1F7 and 2D5 were two subclones which are stable and safe in vitro.

Then, two best subclones of the ROBO1 CAR-NK cells carrying the suicide gene after passing the positive rate by flow cytometry were selected for subsequent experiment and continuous culture, and the two subclones cells were sequenced and verified at the same time, and the sequencing results were correct. Finally, the positive rates of ROBO1 and isapase9 of the verified subclones were tested respectively by flow cytometry, and the results were shown in Figs. 10a-10b. It could be seen from the figures that ATCG427B-1F7 and 2D5 were two stable subclones screened.

**Example 7. Detection of apoptosis of ROBO1-iCaspase9CAR-NK cells induced by medicament AP1903**

Different amounts of medicament AP1903 were respectively added into the ROBO1 CAR-NK cells carrying the suicide gene (ATCG427B-1F7 and 2D5) after screening in Example 6 to test the effect thereof on apoptosis of the ROBO1 CAR-NK cells carrying the suicide gene (denoted with ATCG427B). Meanwhile, the medicament AP1903 was added into the ROBO1 CAR-NK cells as control, and divided into three experimental groups, i.e., cells treated by the medicament AP1903 with different concentrations (0 nM, 10 nM and 50 nM) for 2 hours, 4 hours and 24 hours. Then, apoptosis was detected, and the results were shown in Figure 11. The experimental groups respectively treated for 4 hours and 24 hours were continuously cultured with fresh medium, and the cell viability was counted three days later, and the results were shown in Fig. 12. In addition, the 0 nM and 10 nM experimental groups treated for 4 hours were cultured in fresh medium for 24 hours and then photographed, and the results were shown in Fig. 13.

It could be seen from Fig. 11 that the apoptosis rate of the ROBO1-iCaspase9 cells could reach 60% when the cells were treated with the medicament AP1903 for 2 hours at the concentration of 10 nM in vitro, reach 80% when being treated for 4 hours, and more than 95% of the cells were apoptotic when being treated for 24 hours, while the ROBO1CAR-NK cells were not apoptotic basically.

It could be seen from Fig. 12 that, after being treated with the medicament AP1903, the survival rate of the ROBO1CAR-NK cells was over 80%, while all the ROBO1-iCaspase9 CAR-NK cells of the present invention were apoptotic basically.

It could be seen from Fig. 13 that, after being treated with the medicament AP1903 at 10 nM, the cells were basically apoptotic through observation after 24 hours.

The above results indicate that the medicament AP1903 can effectively kill the ROBO1-iCaspase9CAR-NK cells within 24 hours, but has no effect on the ROBO1-iCaspase9CAR-NK cells, indicating that applying the ROBO1-iCaspase9CAR-NK for treating cancer can induce cell apoptosis under the induction of the medicament AP1903, thus better controlling the CAR-NK cells and further increasing the clinical safety.

**Example 8. Detection of ROBO1 expression in different breast cancer cell strains**

After different breast cancer cells BT474, T47D, HCC1187, HH1937, HCC38, MCF7, MDAMB-231, MDAMB-453, MDAMB-468 and ZR-75-1 were cultured, the expression of ROBO1 in different breast cancer cells was tested by Western Blot, and the results were shown in Fig. 14a. Moreover, a ratio of an optical density of ROBO1 determined by Western Blot to that of control GAPDH was analyzed by ImageJ software, and the results were shown in Fig. 14b.

The results showed that the expression of ROBO1 was different in different breast cancer cell strains, and the highest expression level was found in T47D.

**Example 9. Killing test of ROBO1-iCaspase9 CAR-NK cells**

1. ROBO1 CAR-NK cells carrying a suicide gene for killing breast cancer cells

A CFSE staining method (referring to: A Mathematical Model of Natural Killer Cell Activity, Anna Scherbakova, 1, 2 Helen Lust, 1, 2 Hele Everaus, 1, 2, 3 Alar Aints1, 2, 4*) was used to test the killing effect of the ROBO1 CAR-NK cells carrying the suicide gene (ATCG427B-1F7, ATCG427B-2D5) screened in Example 5, and the ROBO1 CAR-NK and NK-92 cells were used as controls at the same time. Different breast cancer cells included BT474, T47D, HCC1187, HCC1937, MCF7, MDAMB-231, MDAMB-453, MDAMB-468 and ZR-75-1.

The experimental operation method was as follows:

(1) Target cells were treated in advance, and 1 μl of CFSE was added for $1\times10^6$ cells, then the cells were incubated in a dark incubator for 15 minutes, and stopped with 10% BSA, washed with PBS twice, and then 500 μl of tumor cell suspension ($8\times10^4$ cells/well) was added into a 24-well plate. The culture plate was pre-cultured in an incubator for 12 hours.

(2) 500 μl of effector cells were added according to a ratio of the effector cells to the target cells of 0.5:1 and 1:1, three complex wells were placed in each experiment, and the effector cells and the target cells were co-incubated for 2 hours.

(3) After 2 hours, the supernatant was transferred to a 1.5 EP tube, and 200 μl of pancreatin was added to each hole. After digestion for 1 minute, the cells were beaten with the corresponding transferred supernatant, centrifuged, resuspended in PBS, added with 1 μl of 7-AAD, incubated in dark for 15 minutes, and tested on a machine.

(4)

(4) Killing rate = (killing rate of target cells - spontaneous mortality) / (1 - spontaneous mortality) × 100%.

The results as shown in Fig. 15, It could be seen from the figure that different cancer cells have different killing effects in vitro, and cells with high ROBO1 expression, such as T47D cells, have better killing effects; However, cells that do not express ROBO1, such as HCC1937, have poor killing effect; while the killing effect of MDAMB-231 cells was relatively poor even through the ROBO1 was expressed therein, and the reasons might be as follows: firstly, the ROBO1 proteins expressed by the MDAMB-231 was relatively low; secondly, PDL1 molecules were highly expressed in the MDAMB-231 cells, and a PD1-PD-L1 signaling pathway would inhibit the killing of the effector cells in the killing process; and thirdly, MICA/B was basically not expressed in MDAMB-231 itself, while the NK cells partly depended on the combination of activated receptors such as NKG2D and ligand MICA/B to play a killing role, so the non-expression of MICA/B would also weaken the killing of the MDAMB-231 cells. In addition, there might be other immune mechanisms regulating the killing of MDAMB-231 by the effector cells. In a word, the mechanism of the ROBO1-iCaspase9CAR-NK cells killing and controlling the MDAMB-231 cells needs further verification.

Therefore, the above experimental results showed that the specific killing effect of the ROBO1 CAR-NK cells carrying the suicide gene of the present invention was positively correlated with the expression level of ROBO1.

2. ROBO1 CAR-NK cells carrying a suicide gene for killing different cancer cells

Other types of tumor cells were killed by the same method as in the above 1 for 2 hours, and the killing effects of the ROBO1 CAR-NK cells (ATCG427B-1F7, ATCG427B-2D5) carrying the suicide gene screened in Example 5 on different tumor cells were tested. Different tumour cells included lung cancer cells A549 and H1299; hepatoma cells Huh7 and SMMC7721; and pancreatic cancer cells BxPC3, PANC1 and Capan-2, which were cultured and then killed. The results were shown in Fig. 16a-c, from which it could be seen that the ROBO1-iCaspase9 cells had better killing effects on the lung cancer and the liver cancer.

3. Killing of the ROBO1 CAR-NK cells carrying the suicide gene on normal breast cells and PBMC

Three normal people were selected randomly, and 50 ml of fresh blood was taken from each, then the blood was diluted with PBS at a ratio of 1:1; a new centrifuge tube was taken and added with Ficoll (lymphocyte separation solution), and tilted at 45 degrees. The diluted blood was slowly added above the liquid level of Ficoll according to a ratio of Ficoll to blood of 2:1, and centrifuged at 2000 rpm. After 30 minutes, a pipette was directly inserted into a mist layer to suck out the mist layer (i.e., mononuclear cell layer) gently, put into a new centrifuge tube, and washed; then the supernatant was discarded, and 1 ml of RPMI-1640 culture medium was added, blew and blended to prepare PBMC cell suspensions which were respectively denoted with PBMC-1, PBMC-2 and PBMC-3. The ROBO1 expression in normal breast cells and PBMC was tested. The results were shown in Fig. 17a. In addition, the killing potency of the ROBO1-iCaspase9 cells on PBMC-1, PBMC-2, PBMC-3 and MDA-kb2 normal breast cells was tested by the same method as above 1, and the killing rates of NK92 and ROBO1 CAR-NK on PBMC-1, PBMC-2, PBMC-3 and MDA-kb2 cells were compared at the same time in the killing process. The results were shown in Figc. 17b. It could be seen from the figure that the ROBO1-iCaspase9CAR-NK cells have no significant killing effect on the normal breast cells and PBMC in vitro. Thus, the safety of the ROBO1 CAR-NK cell medicament carrying the suicide gene was further proved.

4. Killing of PBMC on ROBO1 CAR-NK cells carrying the suicide gene

Considering that the ROBO1 CAR-NK cells were infused into human body as allogeneic cells, PBMC immune cells in vivo might kill the allogeneic cells. To verify whether the ROBO1 CAR-NK cells would be cleared by PBMC, in vitro, three normal people were randomly selected to collect blood, and PBMC cells were obtained after separation (the preparation method was the same as above), which were denoted with PBMC-4, PBMC-5 and PBMC-6 respectively. In addition, the killing of PBMC-4, PBMC-5 and PBMC-6 on the ROBO1-iCaspase9CAR-NK cells was tested in the same way as in the above 1, and the killing of PBMC on the NK92 and ROBO1 CAR-NK cells was compared in each group of experiments. The results were shown in Fig 18. It could be seen from Fig. 18 that PBMC had no significant killing effect on the ROBO1-iCaspase9 cells in vitro. Therefore, it was further proved that the ROBO1 CAR-NK cell medicament carrying the suicide gene could play a better role in specific killing in vivo.

The above experimental results showed that the killing effect of the ROBO1-iCaspase9 CAR-NK cells of the present invention was equivalent to that of the ROBO1 CAR-NK cells, which could effectively kill tumor cells, and had less cytotoxicity and better killing effect than the CAR-T cells. Moreover, due to the addition of the suicide gene (iCaspase9 switch element), the safety could be further improved. In addition, experiments showed that this cells did not kill normal cells, and the normal cells do not kill this cells, which further illustrated the safety of the ROBO1-iCaspase9 CAR-NK cells.

**Example 10. Preparation of ROBO1-EGFRt-CAR-NK cells and screening of monoclonal cell strains**

Suicide gene EGFRt *TCTAGA*ATGTTGCTGCTTGTAACTTCTCTCCTTCTTTGCGAGTTGCCCCATCCTGCGTTC CTCCTTATTCCCAGGAAGGTATGCAATGGGATCGGTATAGGAGAGTTCAAGGATTCCC TTTCTATCAACGCTACGAATATAAAGCACTTCAAGAACTGTACGTCCATCAGTGGAGA CCTGCATATATTGCCGGTGGCGTTCCGAGGGGACAGTTTTACCCACACGCCCCCTCTC GACCCACAGGAGCTGGATATCTTGAAGACCGTGAAGGAGATAACTGGCTTTCTTCTC ATTCAGGCGTGGCCGGAAAATAGGACAGACTTGCACGCCTTTGAAAACTTGGAAATT ATACGAGGGCGGACAAAACAACACGGTCAATTCAGCCTGGCCGTTGTATCCCTCAAT ATCACTAGCTTGGGTCTCCGAAGTCTGAAAGAAATAAGTGACGGGGACGTTATAATTT CTGGGAACAAGAACCTCTGCTACGCAAACACAATAAACTGGAAAAAATTGTTTGGAA CTAGCGGGCAGAAAACTAAGATCATTAGTAACAGAGGCGAGAATAGTTGCAAAGCCA CCGGACAAGTGTGCCATGCACTTTGCAGCCCCGAGGGTTGTTGGGGCCCTGAACCAC GGGATTGCGTGTCATGCAGAAACGTCTCACGAGGTCGCGAGTGTGTCGACAAATGTA ACCTGCTTGAAGGGGAGCCTCGCGAATTCGTAGAAAACAGCGAGTGCATTCAATGCC ACCCAGAGTGTCTCCCCCAGGCCATGAACATCACCTGTACAGGACGGGGGCCAGATA ACTGTATTCAATGCGCACACTATATAGATGGACCACATTGTGTGAAAACATGTCCCGC AGGGGTCATGGGTGAGAACAACACGCTCGTTTGGAAATATGCAGATGCCGGGCATGT ATGCCACCTCTGTCACCCGAACTGCACTTATGGGTGCACTGGGCCCGGCCTGGAAGG ATGCCCCACCAACGGACCCAAGATTCCCTCCATAGCGACCGGAATGGTTGGAGCCTT GCTTCTTCTTCTGGTAGTGGCGCTCGGGATCGGGTTGTTCATGTAA*GGATCC* (SEQ ID NO: 12, wherein the underlined parts in italics at the beginning and the end were restriction sites) (with a simplified structure shown in Fig. 20) was synthesized, and then cloned into a lentiviral vector to obtain a lentiviral vector (called 1942-3-hEGFRt) shown in Fig. 19 (with a nucleotide sequence shown in SEQ ID NO: 13), and then the virus was packaged by a three-plasmid system (aided by plasmids pMD2.G and pCMVR8.74). Then, the ROBO1 CAR-NK cells (or ROBO1M CAR-NK cells, wherein the ROBO1MCAR-NK cells were used in the following examples) prepared in Example 3 were infected by means of virus infestation, and then the ROBO1 and EGFRt double-positive cells, i.e., the ROBO1 CAR-NK cells (ROBO1-EGFRt CAR-NK cells, also called 427E) carrying the suicide gene were sorted by flow cytometry. The screened cells were cultured in a monoclonal screening manner, and then the positive rate of the ROBO1 CAR-NK cells carrying the suicide gene was tested from the cultured cells by flow cytometry. The results were shown in Figs. 21a-b. It could be seen from the figures that the signal values of APC and PE fluorescence label were significantly increased, and two ROBO1 CAR-NK monoclonal cells (i.e., ROBO1-EGFRt CAR-NK cells, i.e, 427E) carrying the suicide genes were successfully obtained by screening, which were respectively named ROBO1-EGFRt-3C10-2D10 and ROBO1-EGFRt-3C10-1F11 (also called 427E-3C10-2D10 and 427E-3C10-1F11 respectively). The mother clone of the two monoclonal cells was ROBO1-EGFRt-3C10 (427E-3C10).

## Example 11. Killing experiment of ROBO1-EGFRt-CAR-NK cells

Tumor-specific killing experiments were carried out on the monoclonal cell strain prepared in Example 10 and the mother clone of the two monoclonal cell strains. The experimental process was as follows: firstly, the expression level of ROBO1 protein in different types of cancer cells except the breast cancer cells was tested by Western, and the specific steps were as follows: collecting different cancer cell strains, extracting the total protein for lysis, quantifying the total protein with a BCA kit, then subjecting the ROBO1 protein to electrophoresis by SDS-PAGE gel and membrane transferring; and finally, testing the ROBO1 protein by developing. The expression of ROBO1 in some breast cancer cells was referred to the test results in Fig. 14a, while the test results of colorectal cancer cells such as HT-29, LoVo and HCT116 and liver cancer cells such as SMMC7721, Huh7 and HEPG2 were shown in Fig. 22. The results showed that the expression of the ROBO1 protein in different types of cancer cells was obviously different.

Different cancer cells including T47D, HCC1187, MCF7, HepG2 and Huh7 were taken for culture, and meanwhile, ROBO1-EGFRt-3C10, ROBO1-EGFRt-3C10-2D10 and ROBO1-EGFRt-3C10-1F11 were cultured and killed in vitro for 2 hours according to an effector-to-target ratio of 0.5: 1 and 1: 1, respectively, and NK92 and 427A were added as control groups, wherein a 427A-KO experimental group (427A-KO referred to "knocking out the gene sequence of a ROBO1-scFV target in CAR in the form of Crisp Cas9 gene editing, keeping other signal transduction sequences, and then knocking the CAR into a NK92 cell, that is, the NK cell in which the CAR sequence without the ROBO1 target is knocked on the same genome insertion site as CAR in 427A") was also added for the killing experiments of HCC1187, MCF7 and HepG2. According to "Methodology study of elvaluating immune cell therapeutic products cytotoxic potency in vitro" issued by National Institutes for Food and Drug Control, RTCA Systems real-time cell analysis system was a new cell testing system ACEA. The results were shown in Fig. 23a. Through killing analysis, it could be found that the ROBO1-EGFRt-CAR-NK cells showed strong killing effect, and the killing effect thereof on solid tumors was obvious, and the specific killing effect of the cells was positively correlated with the ROBO1 expression level, thus proving the effectiveness of the ROBO1-EGFRt CAR-NK cell medicament.

Meanwhile, in order to prove the specificity of ROBO1 CAR, a ROBO1-overexpressed monoclonal cell strain (HCT116-ROBO1) model was also constructed in vitro, and HCT116 was used as a control, and then the killing experiments were carried out according to the above method. The results were shown in Fig. 23b. It could be seen from the figure that in wild-type HCT116, the killing effect of 427E was slightly stronger than that of NK92, wherein the 427A-KO after knocking out ROBO1 target has no difference from NK92 and 427A. However, in the ROBO1-overexpressed HCT116 monoclonal cell strain, the killing effect of 427A-KO was obviously weakened compared with that of 427A and E; meanwhile, the killing activity of 427E in the overexpressed HCT116 cell strain was obviously enhanced compared with that of the wild type HCT116, thus effectively proving the targeting and specificity of ROBO1 in the ROBO1-EGFRt-CAR-NK cell of the invention.

In addition, a ROBO1-overexpressed monoclonal cell strain (MDAMB-231-ROBO1) model was constructed by the same method, and MDAMB-231 was used as a control group, and then the killing experiments were carried out according to the above method. The results were in Fig. 23c. Compared with the control group MDAMB-231, the killing activity and specificity ROBO1-overexpressed monoclonal cell strain (MDAMB-231-ROBO1) model were obviously improved.

## Example 12. Safety experiment of ROBO1-EGFRt-CAR-NK cells

Safety study was to investigate the sensitivity of the ROBO1-EGFRt CAR-NK cells to a suicide switch inducer (cetuximab) in the presence of killer/effector cells. The effectiveness of the suicide switch was evaluated by inducing apoptosis of the ROBO1-EGFRt CAR-NK cells (the monoclonal cell strains prepared in Example 10 and the mother clone of the two monoclonal cell strains) with cetuximab, and adding NK92-CD16 (high affinity) effector cells constructed in vitro. The concentration, target ratio, action time and action system of the effector cells were investigated respectively. Finally, optimal action conditions of the inductor cetuximab were as follows: 4 hours in vitro, the action concentration was 1 μg/ml, the effector-to-target ratio of NK92-CD16 (high affinity) to ROBO1-EGFRt CAR-NK cells was 25:1 (E:T). The test method was CFSE (referring to: A Mathematical Model of Natural Killer Cell Activity, Anna Scherbakova, 1, 2 Helen Lust, 1, 2 Hele Everaus,1, 2, 3 Alar Aints 1, 2, 4*), and the cell apoptosis potency was tested by 7-AAD/CFSE double staining. The specific method was as follows: the constructed NK92-CD16 effector cells were labeled with CFSE in advance, then the ROBO1-EGFR CAR-NK and NK92-CD16 cells were plated according to the effector-to-target ratio, and then cetuximab with the action concentrations of 0 μg/ml, 1 μg/ml and 10 μg/ml were added respectively. After 4 hours of action, the cells were stained with 7-AAD for 10 minutes, and then tested by flow cytometry (three parallel repetitions). The test results were shown in Fig. 24. It could be seen from the results that the apoptosis potency induced by cetuximab was between 40% and 50% in the selected subcloned cell strains.

In order to better simulate the mechanism of ADCC in vivo and evaluate the effectiveness of the suicide switch more accurately, in vitro, peripheral bloods from two normal people were extracted, and PBMC were separated, which were denoted with PBMC-ZF and PBMC-CQ. According to the above experimental methods and conditions, PBMC-ZF and PBMC-CQ were incubated with NK92/427A/427E cell strains, and cetuximab (with action concentrations of 0 μg/ml, 1 μg/ml and 10 μg/ml, respectively) were added at the same time, and the effector-to-target ratio was 25: 1. After 4 hours of action, the killing potency (referring to Fig. 25) of 427E cell strains by PBMC was tested by flow cytometry under the action of cetuximab. It could be seen from the results that under the same effector-to-target ratio and concentration, the potency of cell killing induced by PBMC of different people were different, which was caused by the differences of immune cell compositions and functions of different people. However, compared with the 427A and NK92 cells, the potency of the cell killing induced by 427E was obviously enhanced. On the whole, when PBMC was used as effector cells (the effector-to-target ratio was 25:1), the apoptosis potency of ATCG427E-3C10-1F11 and ATCG427E-3C10-2D10 induced by cetuximab (with a action concentration of 1 μg/ml) ranged from 30% to 40%, which was slightly worse than that induced by NK92-CD16 serving as effector cells, and this was consistent with the data reported in existing literatures and patents, and even, our effect was more obvious.

In conclusion, by the evaluation on the effect of ADCC in vitro, it can be seen that both NK92-CD16 and PBMC, as killer/effector cells, can be effectively induced by cetuximab to activate a suicide switch element, thus causing cell death and increasing cell controllability.

The above experimental results show that the killing effect of the ROBO1-iCaspase9 CAR-NK or ROBO1-EGFRt CAR-NK cells of the present invention is equivalent to that of the ROBO1 CAR-NK cells, and the specific killing effect is significantly enhanced compared with the NK92 cells, which can effectively target tumor cells for killing; secondly, the CAR-NK cells have less cytotoxicity, shorter action time and faster killing effect than the CAR-T cells; and finally, because the suicide gene (such as iCaspase9 or EGFRt induced suicide switch element) are also added in the CAR-NK cells of the present invention, the safety and controllability of the CAR-NK cells can be further improved, so that clinical medication is safer and more controllable.

The description of the present invention is given for the purpose of illustration and description, and is not intended to be exhaustive or to limit the present invention to the form disclosed. Many modifications and variations will be apparent to those of ordinary skills in the art. The embodiments are chosen and described in order to better explain the principles and practical applications of the present invention, and to enable those of ordinary skills in the art to understand the present invention, thus designing various embodiments with various modifications suitable for specific uses.

## Claims

1. A nucleotide sequence carrying a suicide gene, **characterized in that**: a gene encoding a chimeric antigen receptor, and further comprising a suicide inducing gene, wherein the chimeric antigen receptor comprises an antigen binding domain, a transmembrane domain and a costimulatory signal transduction region, the antigen binding domain is capable of specifically binding to a tumor specific antigen, and activating a NK cell through the transmembrane domain and the costimulatory signal transduction region, and the tumor specific antigen is ROBO1.

2. The nucleotide sequence carrying the suicide gene of claim 1, **characterized in that**: the suicide inducing gene is iCaspase9, EGFRt, CD20, rapamycin and/or RQR8.

3. The nucleotide sequence carrying the suicide gene of claim 2, **characterized in that**: the suicide inducing gene is iCaspase9, the iCaspase9 comprises FKBP12-F36V and ΔCaspase9, a nucleotide sequence of the FKBP12-F36V is shown in SEQ ID NO: 1, and a nucleotide sequence of the ΔCaspase9 is shown in SEQ ID NO: 2.

4. The nucleotide sequence carrying the suicide gene of claim 3, **characterized in that**: the FKBP12-F36V and the ΔCaspase9 are connected through a Linker, and a nucleotide sequence of the Linker is shown in SEQ ID NO: 3.

5. The nucleotide sequence carrying the suicide gene of claim 4, **characterized in that**: the iCaspase9 is further provided with a flag gene, and the flag gene is preferably CD19, Myc, Flag, HA or His.

6. The nucleotide sequence carrying the suicide gene of claim 5, **characterized in that**: the flag gene is CD19, the iCaspase9 is further provided with a splicing gene, and the splicing gene is preferably T2A; and a nucleotide sequence of the CD19 is shown in SEQ ID NO: 4, and a nucleotide sequence of the T2A is shown in SEQ ID NO: 5.

**7.** The nucleotide sequence carrying the suicide gene of claim 6, **characterized in that**: the nucleotide sequence of the iCaspase9 is shown in SEQ ID NO: 6.

**8.** The nucleotide sequence carrying the suicide gene of claim 2, **characterized in that**: the suicide inducing gene is EGFRt, and the EGFRt comprises an extracellular domain III, an extracellular domain IV and a transmembrane region of EGFR.

**9.** The nucleotide sequence carrying the suicide gene of claim 8, **characterized in that**: a nucleotide sequence of the EGFRt is shown in SEQ ID NO: 12.

**10.** The carrying the suicide gene of any one of claims 1 to 9, **characterized in that**: the antigen binding domain is capable of specifically binding to one or more of Igl, Ig2, Ig3, Ig4, Ig5, FN1, FN2 and FN3 domains of the tumor specific antigen ROBO1.

**11.** The carrying the suicide gene of claim 10, **characterized in that**: the antigen binding domain is capable of specifically binding to the FN3 domain of the tumor specific antigen ROBO1.

**12.** The nucleotide sequence carrying the suicide gene of claim 11, **characterized in that**: the antigen binding domain is an antibody or an antigen binding fragment thereof specifically binding to the FN3 domain of ROBO1, and the antigen binding fragment is Fab or ScFV.

**13.** The nucleotide sequence carrying the suicide gene of claim 12, **characterized in that**: the transmembrane domain is selected from one or more of CD28, CD3ε, CD45, CD4, CD5, CD8, CD9, CD16, CD22, CD33, CD37, CD134, CD137, ICOS and CD154; and preferably, the transmembrane domain is a CD8 transmembrane domain; and/or, the costimulatory signal transduction region contains an intracellular domain of a costimulatory molecule, and the costimulatory molecule is selected from one or more of CD3ζ, CD3γ, CD3δ, CD3ε, CD5, CD22, CD79a, CD79b, CD66d, CD2, CD4, CD5, CD28, CD134, CD137, ICOS, CD154, 4-1BB and OX40; and preferably, the costimulatory signal transduction region contains intracellular domains of 4-1BB and CD3ζ.

**14.** The carrying the suicide gene of claim 13, **characterized in that**: the chimeric antigen receptor comprises a fusion protein with a structure of ScFV-CD8-4-1BB-CD3ζ, and the ScFv is capable of specifically binding to the FN3 domain of the tumor specific antigen ROBO1.

**15.** The nucleotide sequence carrying the suicide gene of claim 14, **characterized in that**: an amino acid sequence of the fusion protein ScFv-CD8-4-1BB-CD3ζ is shown in SEQ ID NO: 8 or SEQ ID NO: 9.

**16.** The nucleotide sequence carrying the suicide gene of claim 15, **characterized in that**: an encoding nucleotide sequence of the fusion protein ScFv-CD8-4-1BB-CD3ζ is shown in SEQ ID NO: 10 or SEQ ID NO: 11.

**17.** A construct carrying a suicide gene, **characterized in that**: the nucleotide sequence of any one of claims 1 to 16.

**18.** The construct of claim 17, **characterized in that**: when the suicide gene is iCaspase9, a nucleotide sequence of the construct is shown in SEQ ID NO: 7; or
when the suicide gene is EGFRt, the nucleotide sequence of the construct is shown in SEQ ID NO: 13.

**19.** A chimeric antigen receptor carrying a suicide gene, encoded by the nucleotide sequence of any one of claims 1 to 16.

**20.** A ROBO1 CAR-NK cell carrying a suicide gene, **characterized in that**: the cell expresses the chimeric antigen receptor of claim 19.

**21.** The ROBO1 CAR-NK cell carrying the suicide gene of claim 20, **characterized in that**:
the ROBO1 CAR-NK cell carrying the suicide gene is capable of effectively destroying or killing a lung cancer cell, a pancreatic cancer cell, a hepatoma cell, a breast cancer cell, a colon cancer cell, a prostate cancer cell or a gastric cancer cell.

**22.** A preparation method of the ROBO1 CAR-NK cell carrying the suicide gene of claim 20 or 21, comprising the following steps of:

(1) synthesizing and amplifying the suicide gene in the nucleotide sequence carrying the suicide gene of any one of claims 1 to 16, and cloning the nucleotide sequence into a lentiviral expression vector to obtain a lentiviral vector carrying the suicide gene; and
(2) packaging a lentivirus through a lentivirus packaging cell line and a three-plasmid system comprising the lentivirus vector carrying the suicide gene obtained in step (1) to obtain a lentivirus carrying the suicide gene, and then infecting the ROBO1 CAR-NK cell with the lentiviral carrying the suicide gene to integrate the suicide gene into a genome of the ROBO1 CAR-NK cell to obtain the ROBO1 CAR-NK cell carrying the suicide gene.

**23.** The preparation method of the ROBO1 CAR-NK cell carrying the suicide gene of claim 22, **characterized in that**: the ROBO1 CAR-NK cell is prepared through a method comprising the following steps of:

a. synthesizing and amplifying a nucleotide sequence encoding the chimeric antigen receptor, and cloning the nucleotide sequence into a lentiviral expression vector; and preferably, synthesizing the encoding nucleotide sequence of the fusion protein ScFv-CD8-4-1BB-CD3ζ in any one of claims 14 to 16 to obtain a lentiviral vector containing the encoding nucleotide sequence of the fusion protein ScFv-CD8-4-1BB-CD3ζ;
b. packaging through a lentiviral packaging plasmid and the lentivirus expression vector obtained in step a in a packaging cell line to prepare a lentivirus; and
c. infecting a NK cell with the lentivirus obtained in step b to obtain the ROBO1 CAR-NK cell.

**24.** A pharmaceutical composition, comprising the ROBO1 CAR-NK cell carrying the suicide gene of claim 20 or 21, or the ROBO1 CAR-NK cell carrying the suicide gene prepared by the method of claim 22 or 23; and preferably, the pharmaceutical composition further comprising an inducer, **characterized in that**: when the suicide gene is iCaspase9, the inducer is preferably AP1903 or AP20187; and a concentration of the inducer is 0 nM to 50 nM; or

when the suicide gene is EGFRt, the inducer is preferably cetuximab with an action concentration of 1 μg/ml; and more preferably, an effector-to-target ratio of the ROBO1 CAR-NK cell carrying the suicide gene to a tumour cell is 0.5:1 to 5:1, and the effector-to-target ratio is preferably 0.5:1 to 1:1.

**25.** The pharmaceutical composition of claim 24, **characterized in that**: the pharmaceutical composition further comprises pharmaceutically acceptable salts, carriers, excipients and adjuvants.

**26.** An application of the ROBO1 CAR-NK cell carrying the suicide gene of claim 20 or 21, or the ROBO1 CAR-NK cell carrying the suicide gene prepared by the method of claim 22 or 23 in the preparation of a medicament for treating and/or preventing a cancer, wherein the cancer is preferably a tumor with high expression of ROBO1 and related diseases.

**27.** The application of claim 26, **characterized in that**: the cancer is liver cancer, breast cancer, colon cancer, pancreatic cancer, prostate cancer, gastric cancer, or lung cancer.

**28.** The application of claim 27, **characterized in that**: the medicament is a medicament in an intratumoral administration form, such as a medicament in an intratumoral injection form or a medicament in an intravenous infusion form.

**29.** A method for treating and/or preventing a cancer by using the ROBO1 CAR-NK cell carrying the suicide gene of claim 20 to 21, or the ROBO1 CAR-NK cell carrying the suicide gene prepared by the method of claim 22 or 23, or the pharmaceutical composition of claim 24 or 25, wherein the method comprises administering an effective amount of the pharmaceutical composition containing the ROBO1 CAR-NK cell carrying the suicide gene into a patient.

**30.** The method of claim 29, **characterized in that**: the cancer is a tumor with high expression of ROBO1 and related diseases.

**31.** The method of claim 29 or 30, **characterized in that**: the cancer is liver cancer, breast cancer, colorectal cancer, pancreatic cancer, prostate cancer, gastric cancer, or lung cancer; and preferably, the cancer is breast cancer, colorectal cancer or liver cancer.

**32.** The method of any one of claims 29 to 31, **characterized in that**: a dosage of the ROBO1 CAR-NK cell carrying the suicide gene is $0.5\times10^9$ cells/times to $5\times10^9$ cells/times.

**33.** The method of any one of claims 29 to 31, **characterized in that**: an administration mode of the ROBO1 CAR-NK cell carrying the suicide gene is intratumoral injection, intravenous injection, intrathoracic injection or local intervention.

**34.** The method of claim 33, **characterized in that**: the administration mode of the ROBO1 CAR-NK cell carrying the suicide gene is intravenous injection.

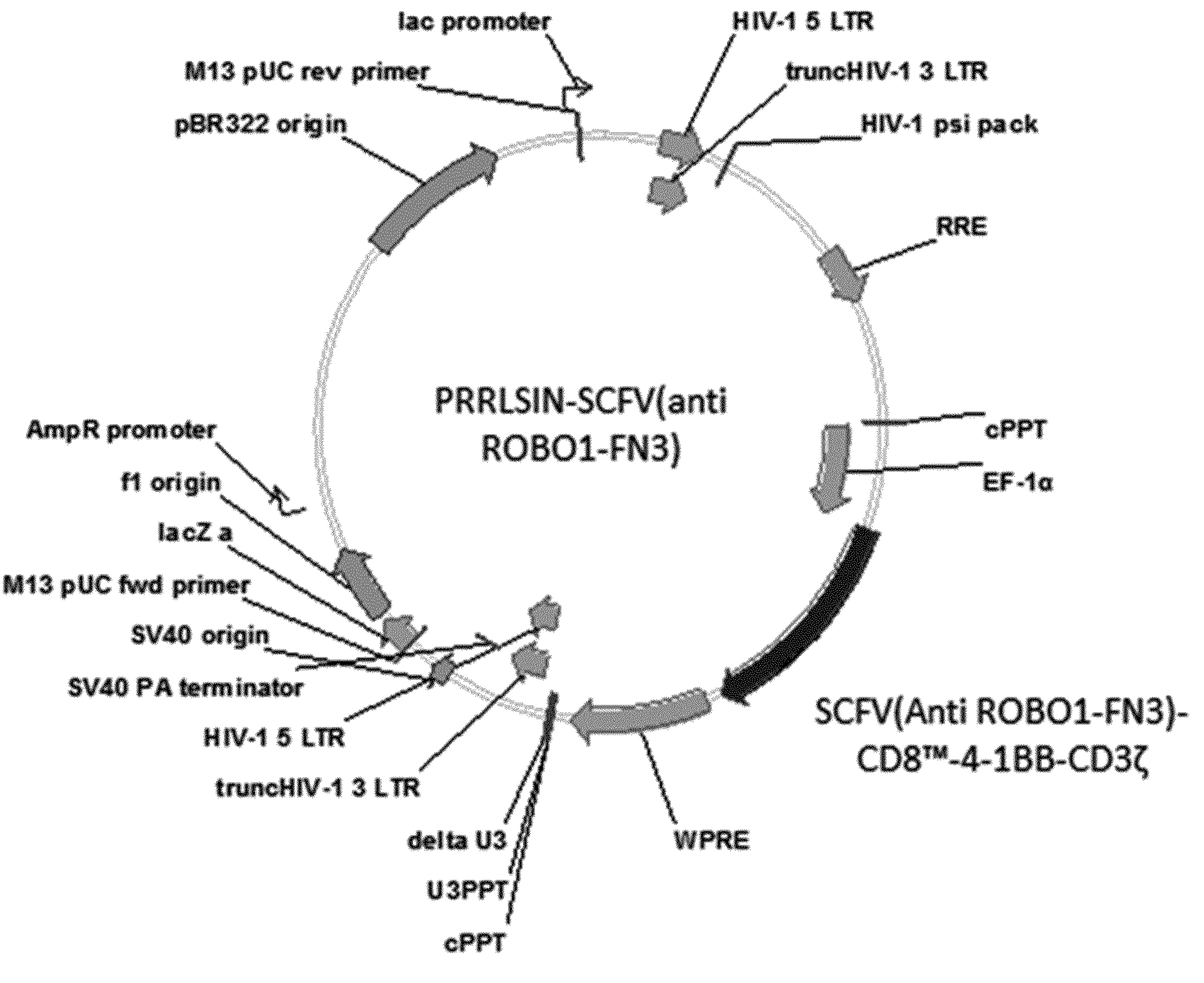
lac promoter
HIV-1 5 LTR
M13 pUC rev primer
truncHIV-1 3 LTR
pBR322 origin
HIV-1 psi pack
RRE
PRRLSIN-SCFV(anti
ROBO1-FN3)
AmpR promoter
cPPT
f1 origin
EF-1α
lacZ a
M13 pUC fwd primer
SV40 origin
SV40 PA terminator
SCFV(Anti ROBO1-FN3)-
CD8™-4-1BB-CD3ζ
HIV-1 5 LTR
truncHIV-1 3 LTR
delta U3
WPRE
U3PPT
cPPT

Fig. 1

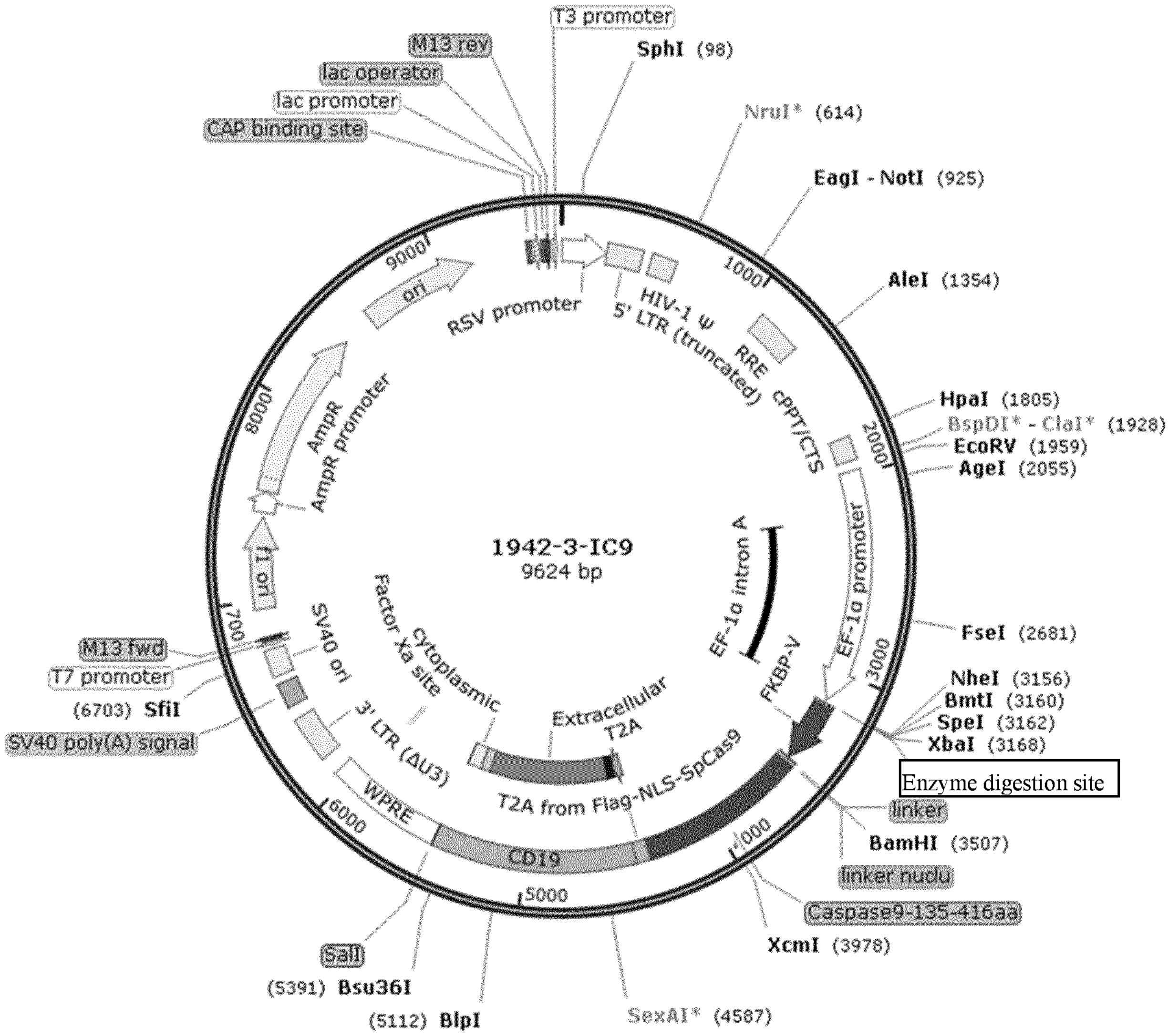

T3 promoter
SphI (98)
M13 rev
lac operator
lac promoter
CAP binding site
NruI* (614)
EagI - NotI (925)
AleI (1354)
HpaI (1805)
BspDI* - ClaI* (1928)
EcoRV (1959)
AgeI (2055)
FseI (2681)
NheI (3156)
BmtI (3160)
SpeI (3162)
XbaI (3168)
Enzyme digestion site
linker
BamHI (3507)
linker nuclu
Caspase9-135-416aa
XcmI (3978)
SexAI* (4587)
(5112) BlpI
(5391) Bsu36I
SalI
SV40 poly(A) signal
(6703) SfiI
T7 promoter
M13 fwd
RSV promoter
HIV-1 ψ
5' LTR (truncated)
RRE
cPPT/CTS
EF-1α promoter
EF-1α intron A
FKBP-V
T2A from Flag-NLS-SpCas9
Extracellular
T2A
Cytoplasmic
Factor Xa site
CD19
WPRE
3' LTR (ΔU3)
SV40 ori
f1 ori
AmpR promoter
AmpR
ori
1942-3-IC9
9624 bp
1000
2000
3000
4000
5000
6000
700
8000
9000


Fig. 2

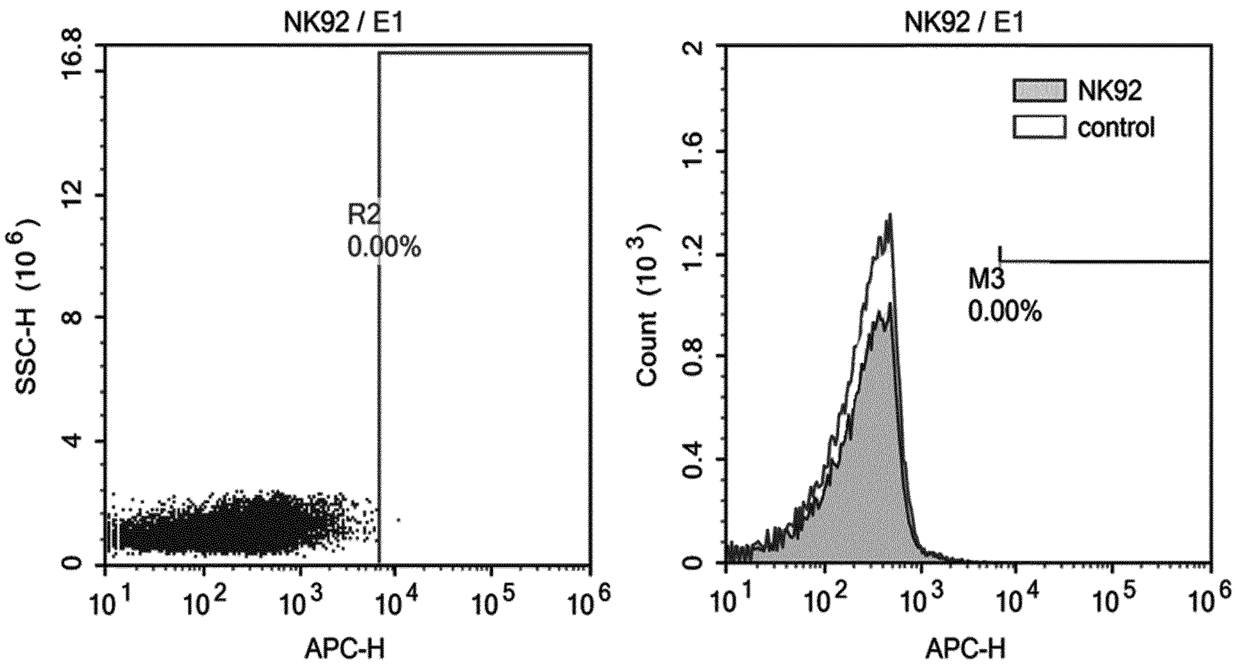
NK92 / E1
SSC-H (10 6)
16.8
12
8
4
0
R2
0.00%
10 1
10 2
10 3
10 4
10 5
10 6
APC-H
NK92 / E1
Count (10 3)
2
1.6
1.2
0.8
0.4
0
NK92
control
M3
0.00%
APC-H

Fig. 3a

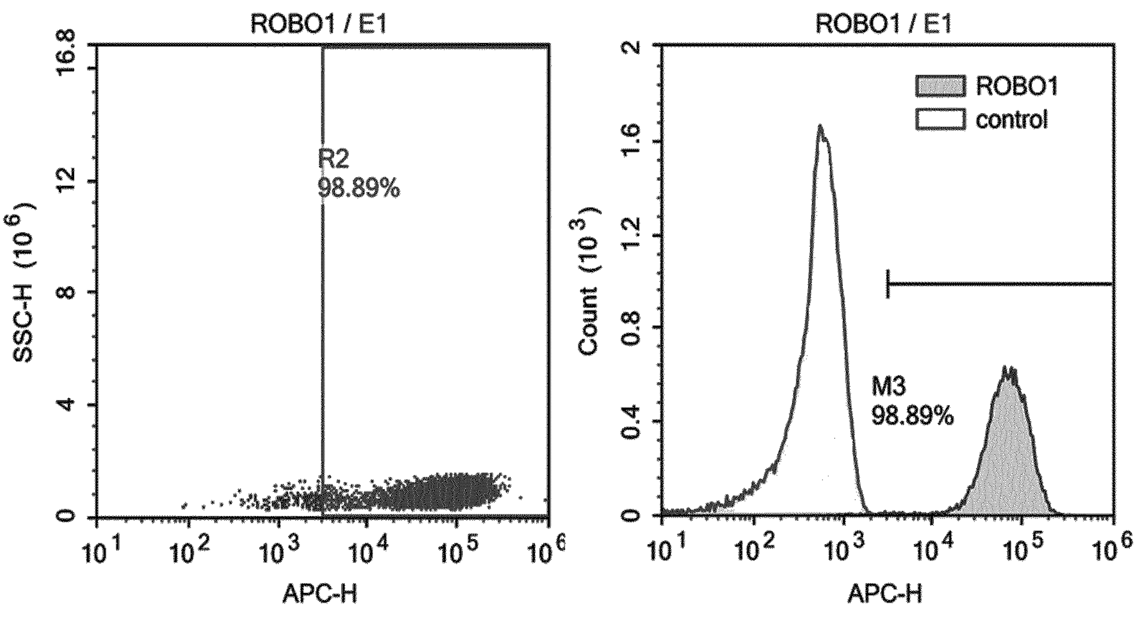
ROBO1 / E1
SSC-H (10 6)
R2
98.89%
APC-H
ROBO1 / E1
Count (10 3)
ROBO1
control
M3
98.89%
APC-H

Fig. 3b

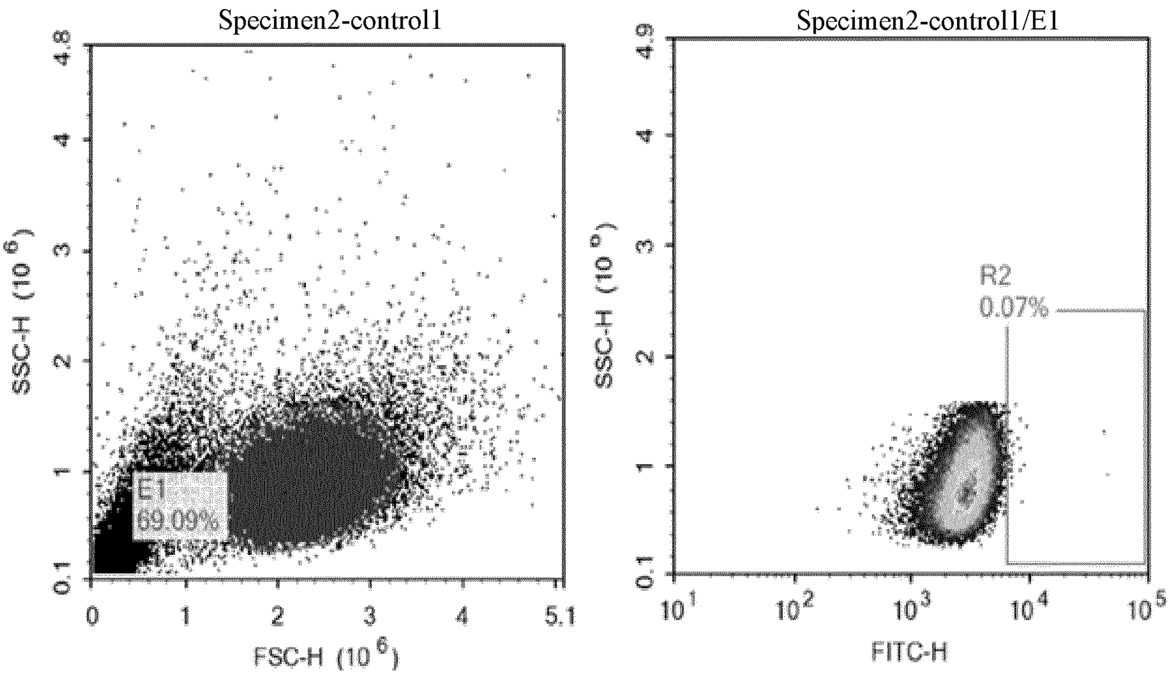
Specimen2-control1
SSC-H (10 6)
FSC-H (10 6)
E1
69.09%
Specimen2-control1/E1
SSC-H (10 6)
FITC-H
R2
0.07%

Fig. 4a

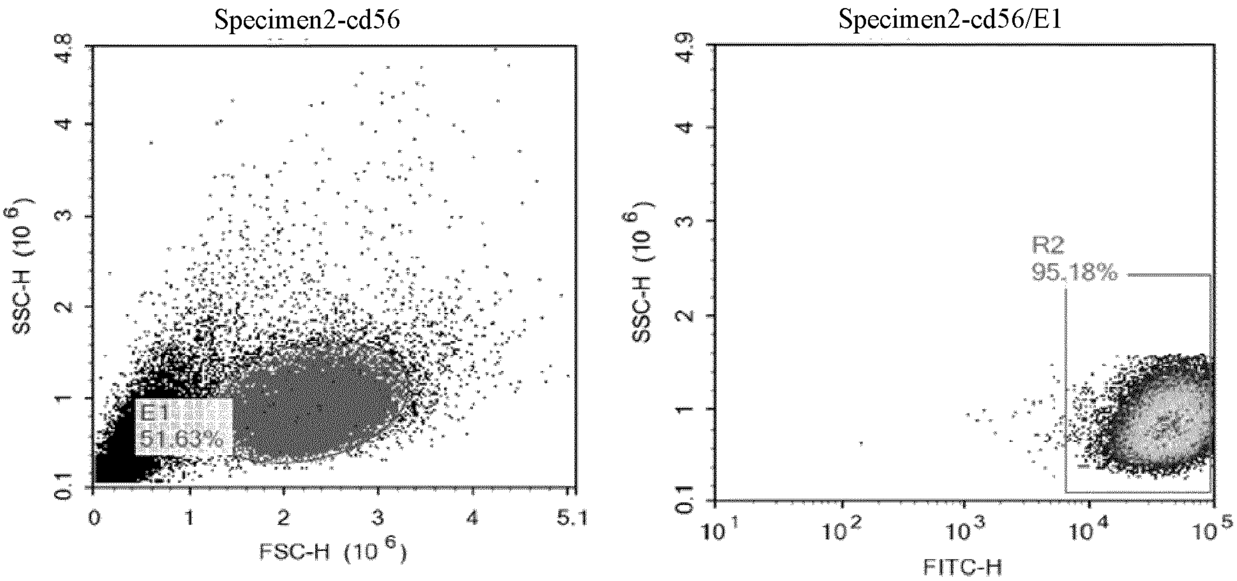
Specimen2-cd56
SSC-H (10 6)
FSC-H (10 6)
E1
51.63%
Specimen2-cd56/E1
SSC-H (10 6)
FITC-H
R2
95.18%

Fig. 4b

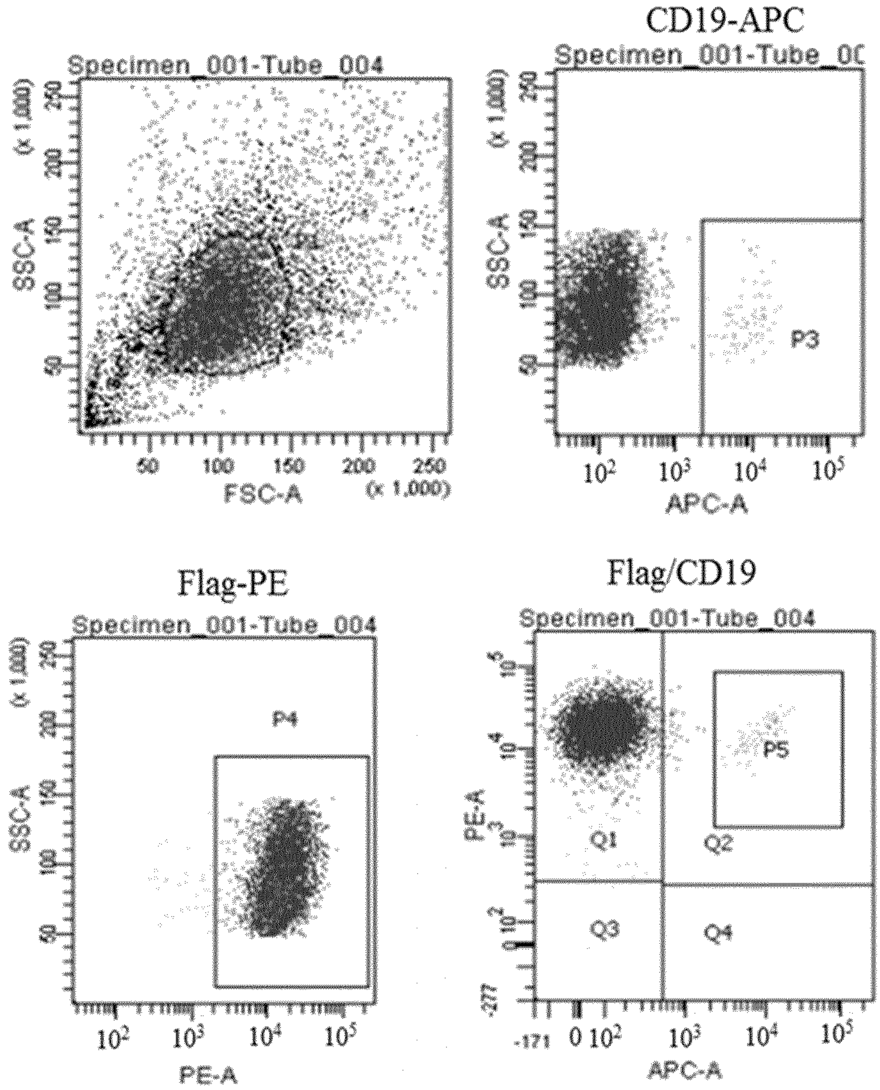
CD19-APC
Specimen_001-Tube_004
SSC-A
FSC-A
P3
APC-A
Flag-PE
P4
PE-A
Flag/CD19
Q1
Q2
Q3
Q4
P5

Fig. 5

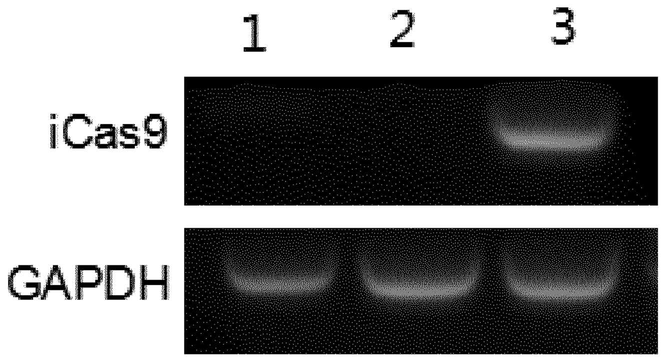
1
2
3
iCas9
GAPDH

Fig. 6

| ID | Chr | Start | End | SupportReads | Copynumber | Func.refGene | Gene.refGene |
|---|---|---|---|---|---|---|---|
| 1 | chr2 | 235229542 | 235229546 | LTR2:4;LTR1:8 | LTR2:0.15;LTR1:0.3 | intergenic | SPP2;ARL4C |
| 2 | chr10 | 56989308 | 56989314 | LTR2:11;LTR1:4 | LTR2:0.94;LTR1:0.34 | intergenic | PCDH15;MTRNR2L5 |

Fig. 7

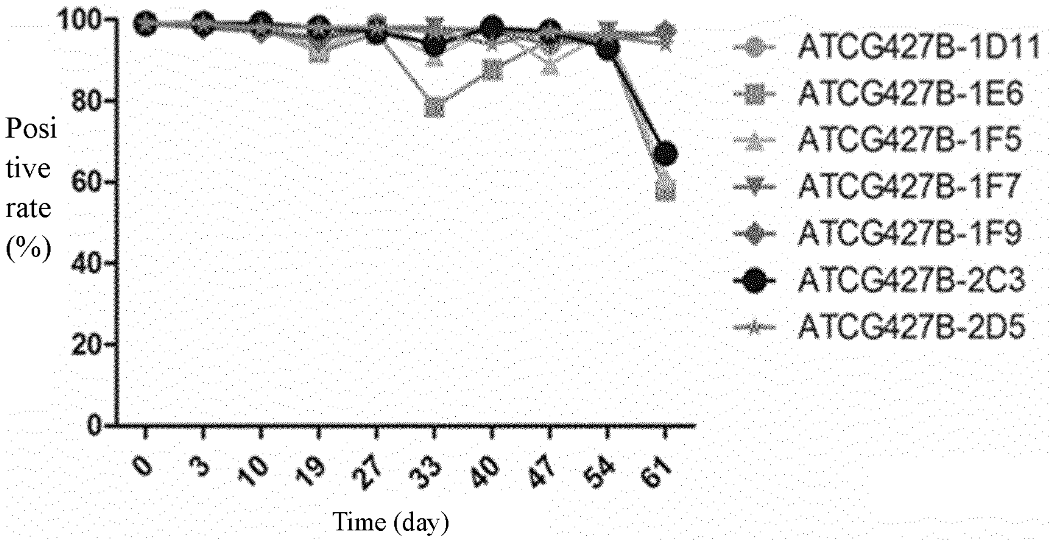


Fig. 8

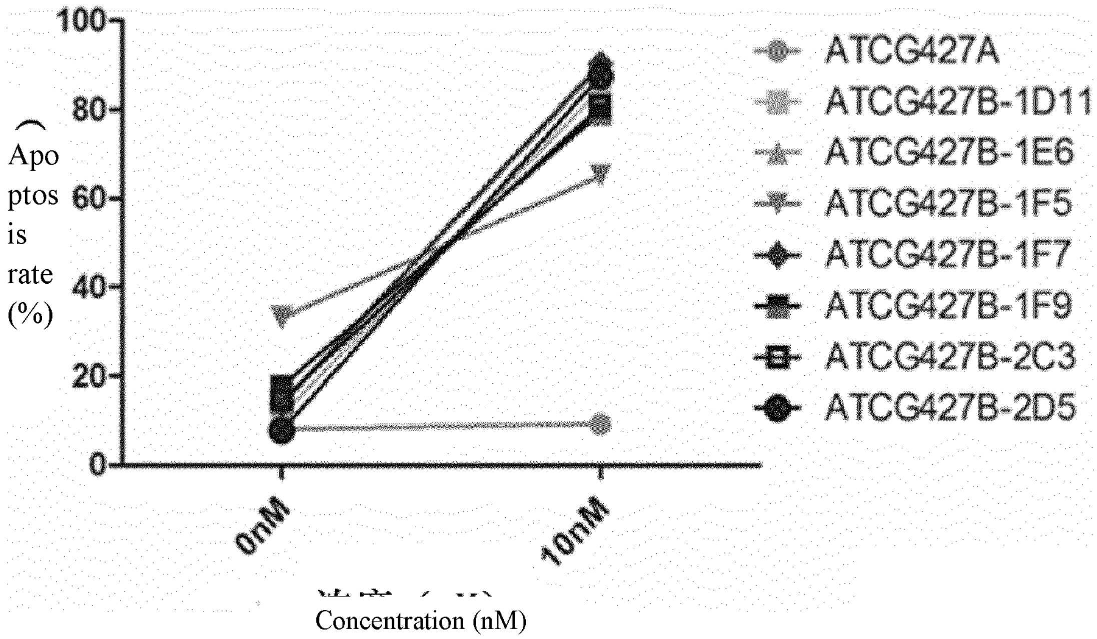


Concentration (nM)

Fig. 9

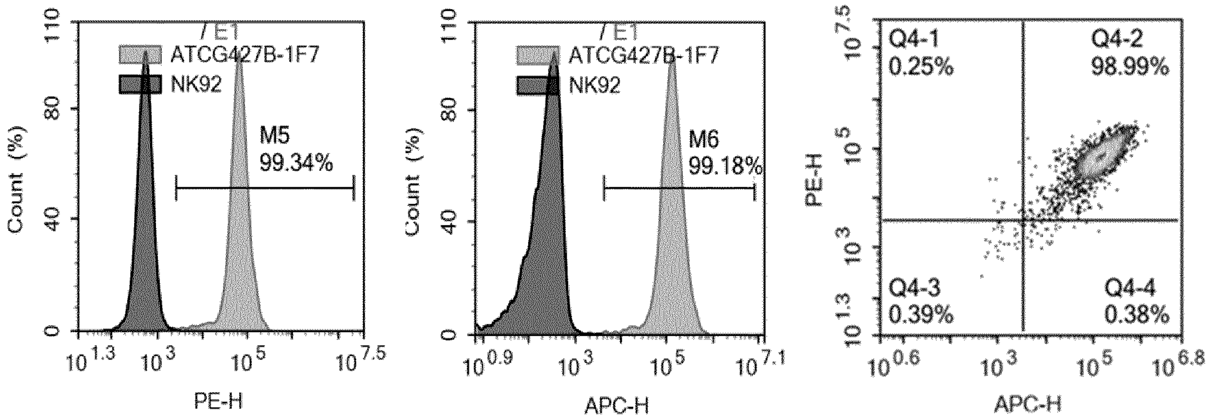


Fig. 10a

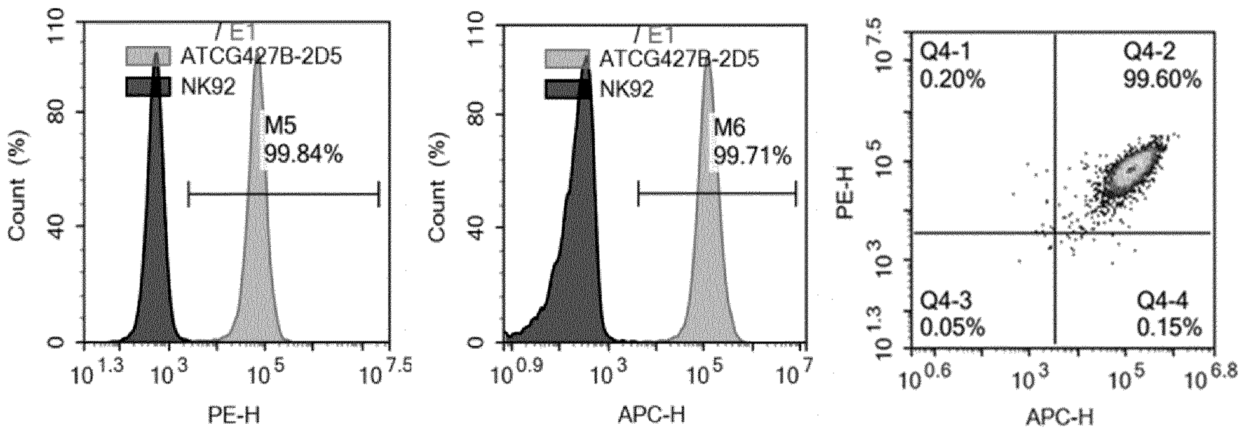


Fig. 10b

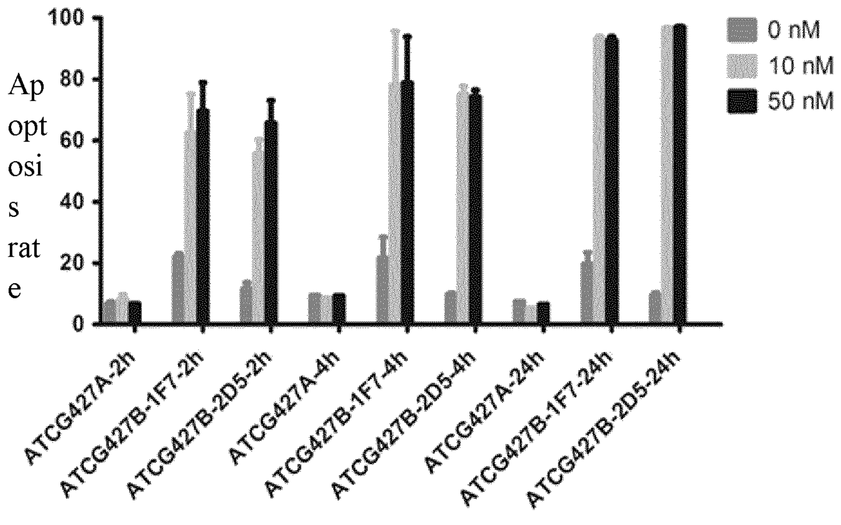


Fig. 11

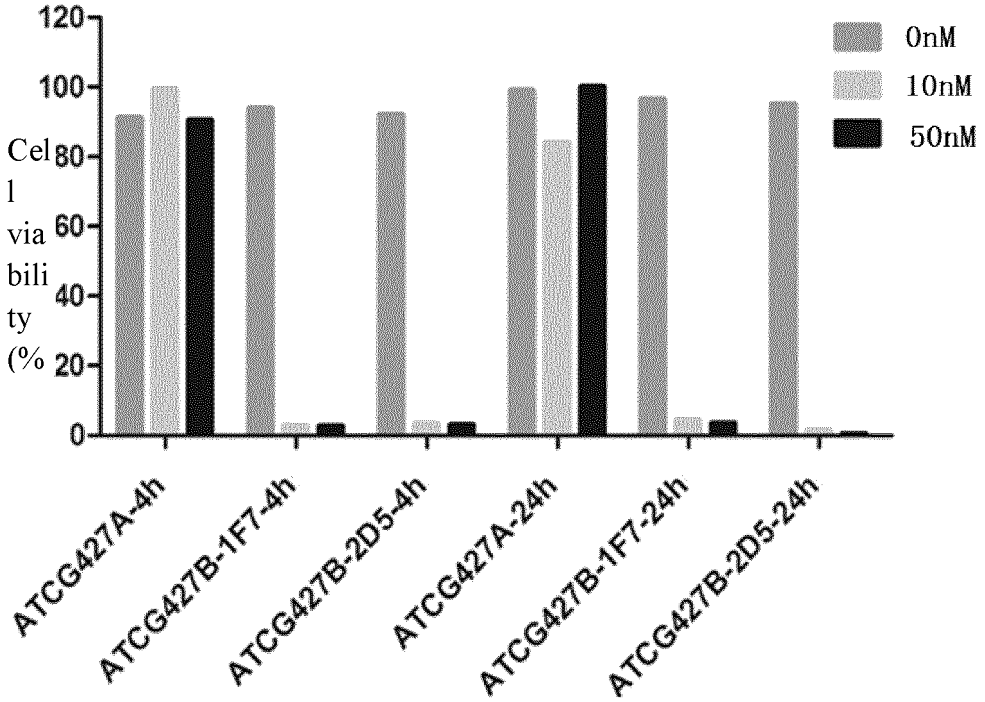
120
100
80
60
40
20
0
Cell viability (%
0nM
10nM
50nM
ATCG427A-4h
ATCG427B-1F7-4h
ATCG427B-2D5-4h
ATCG427A-24h
ATCG427B-1F7-24h
ATCG427B-2D5-24h

Fig. 12

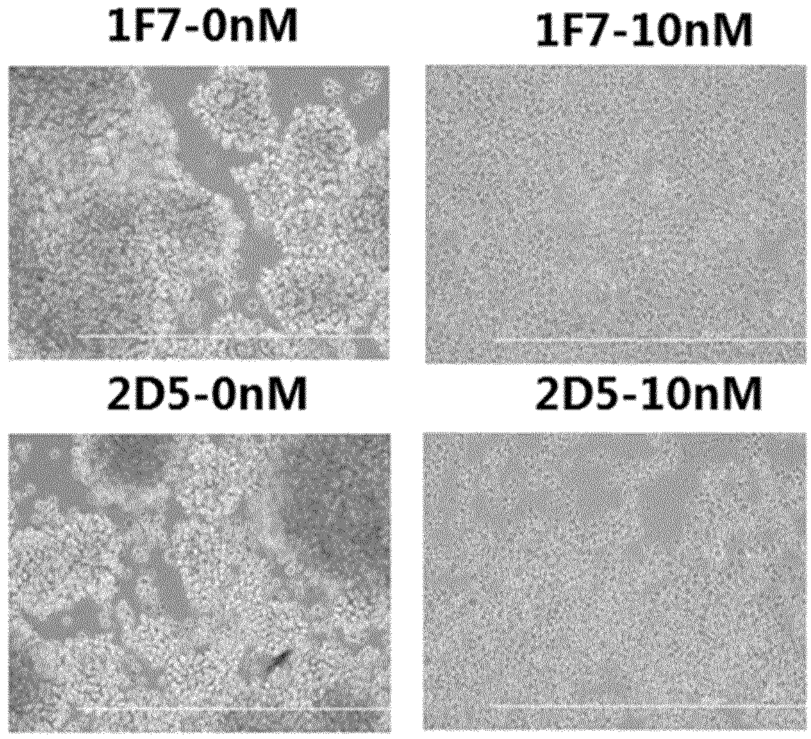
1F7-0nM
1F7-10nM
2D5-0nM
2D5-10nM

Fig. 13

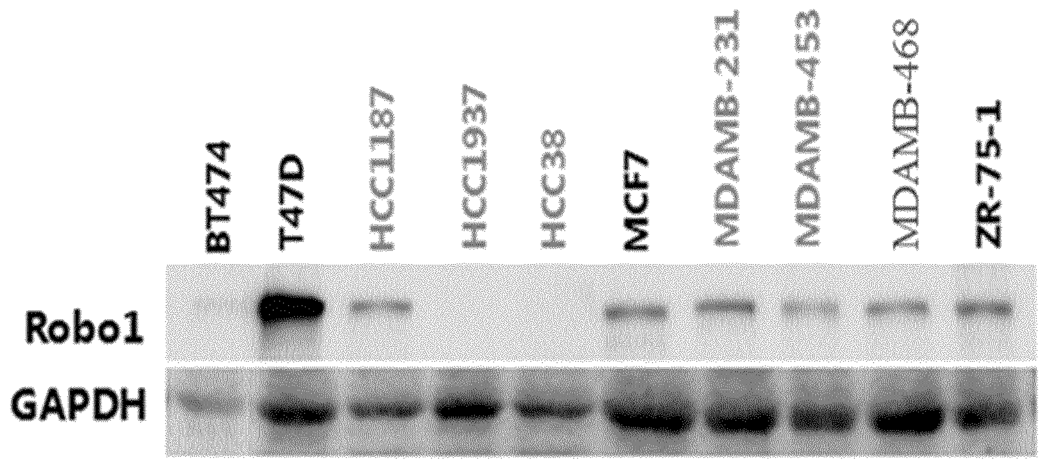


Fig. 14a

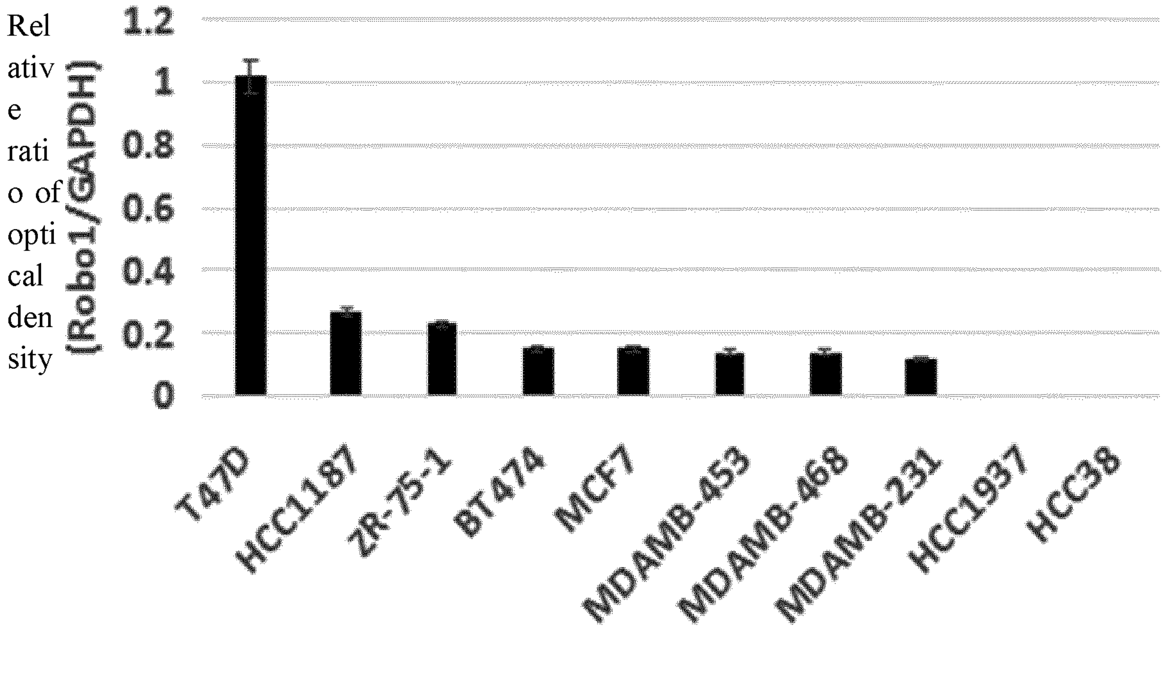


Fig. 14b

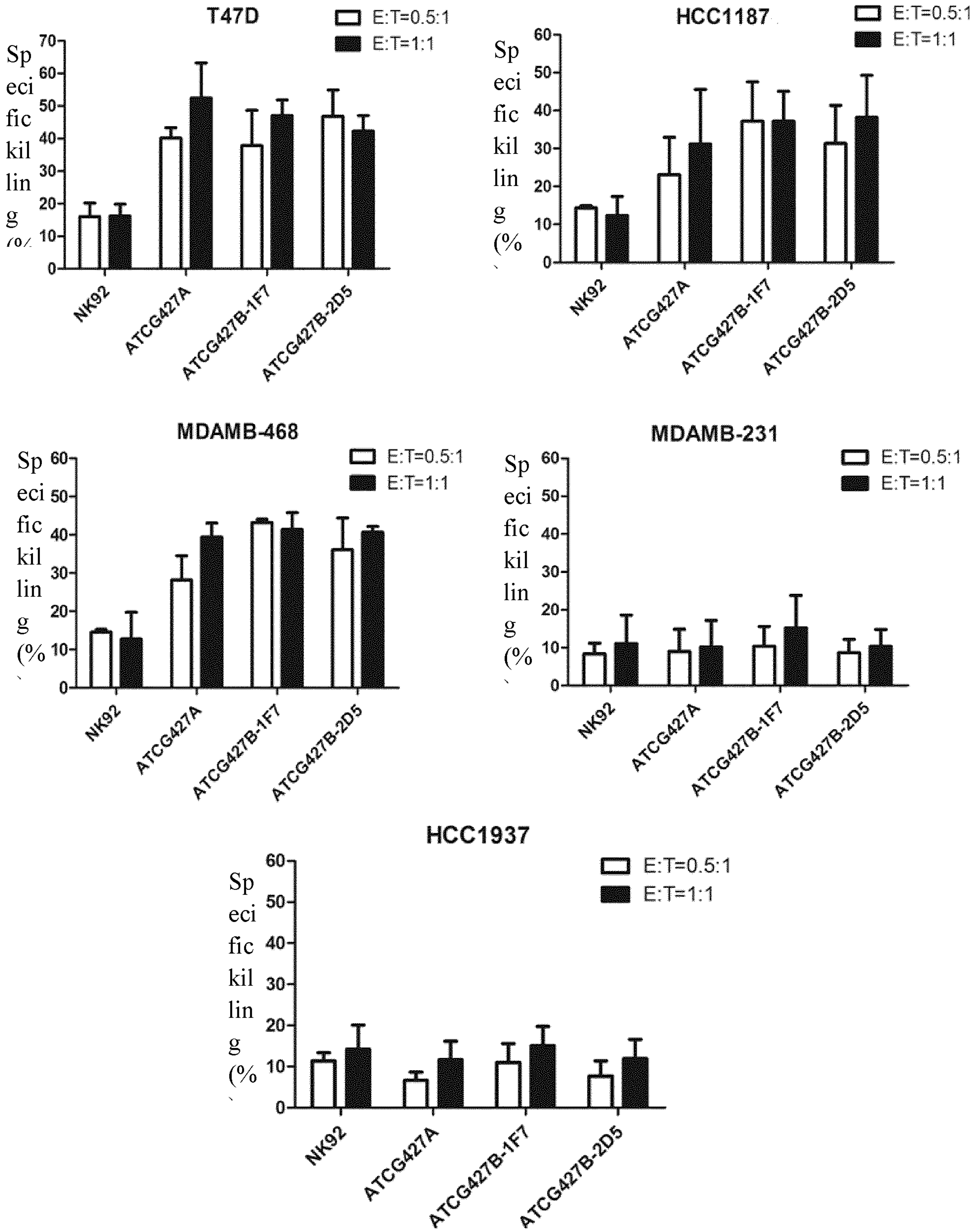
T47D
HCC1187
MDAMB-468
MDAMB-231
HCC1937
E:T=0.5:1
E:T=1:1
Specific killing (%)
NK92
ATCG427A
ATCG427B-1F7
ATCG427B-2D5

Fig. 15

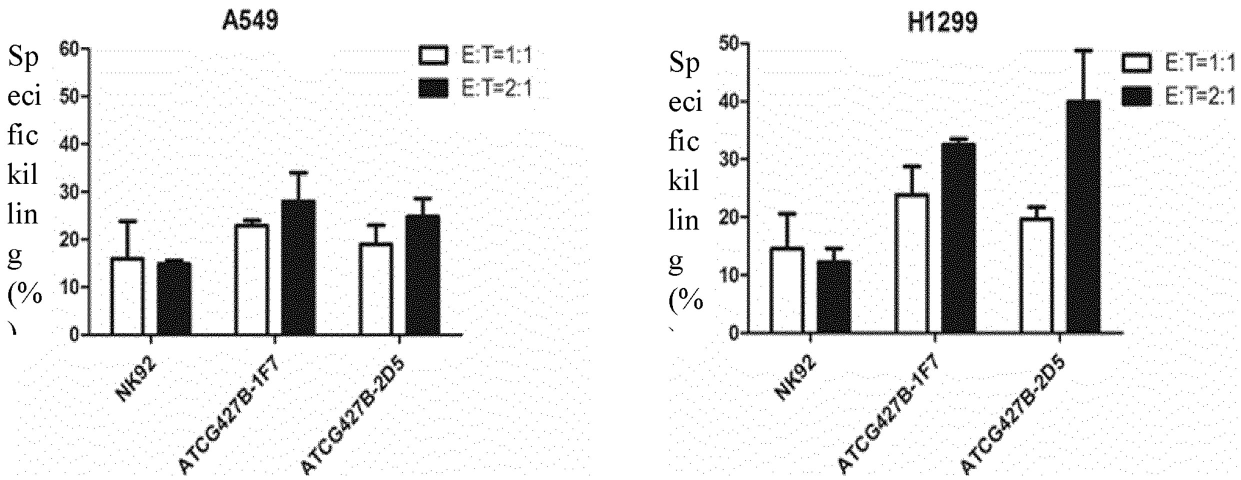


Fig. 16a

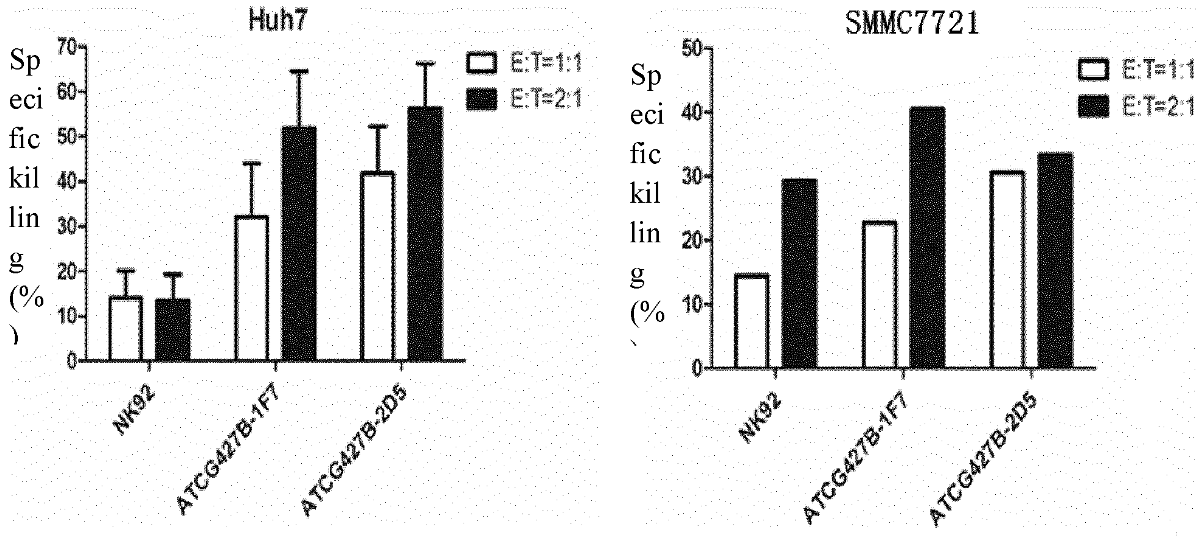


Fig. 16b

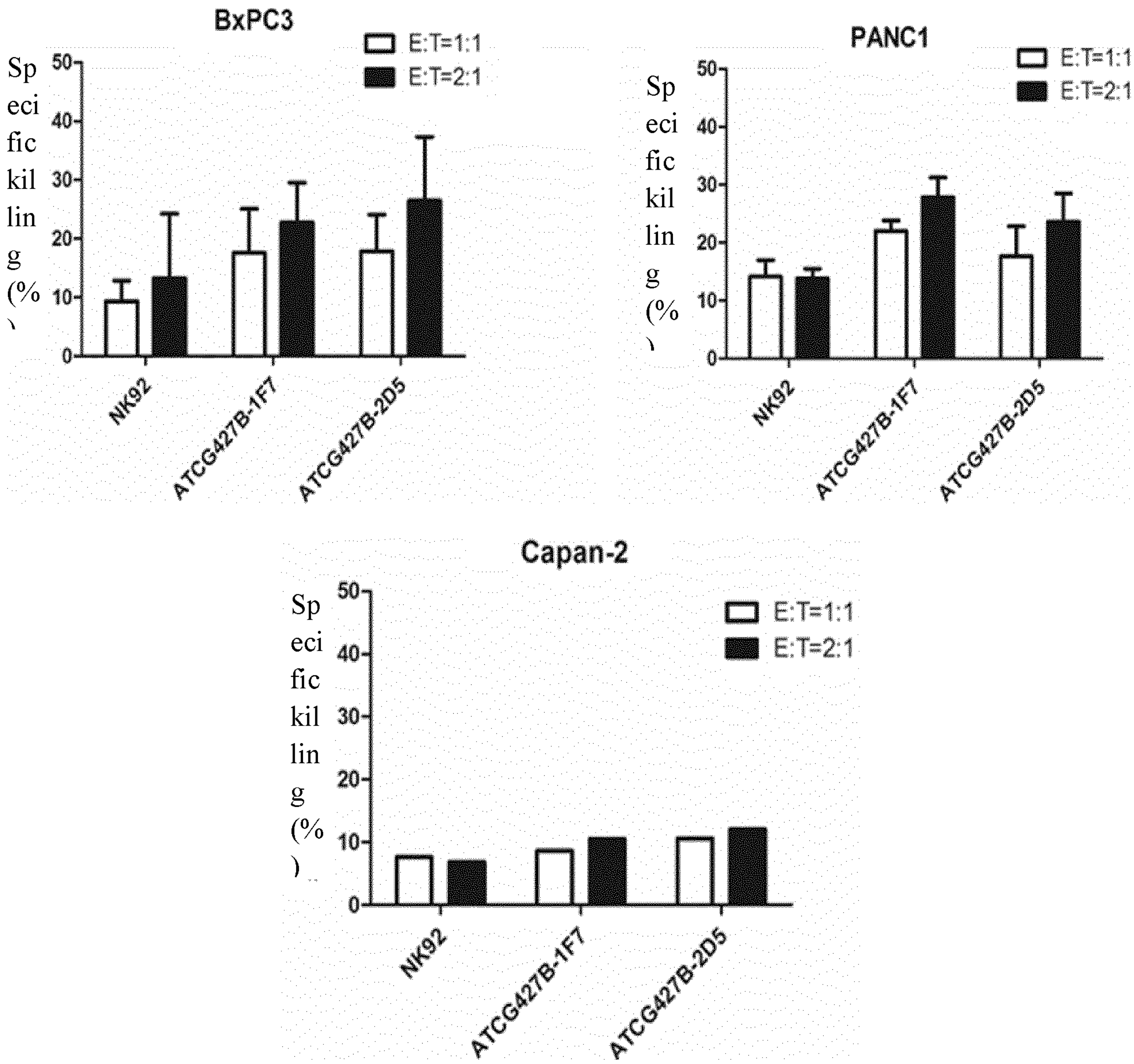
BxPC3
E:T=1:1
E:T=2:1
Specific killing (%)
0
10
20
30
40
50
NK92
ATCG427B-1F7
ATCG427B-2D5
PANC1
E:T=1:1
E:T=2:1
Specific killing (%)
0
10
20
30
40
50
NK92
ATCG427B-1F7
ATCG427B-2D5
Capan-2
E:T=1:1
E:T=2:1
Specific killing (%)
0
10
20
30
40
50
NK92
ATCG427B-1F7
ATCG427B-2D5

Fig. 16c

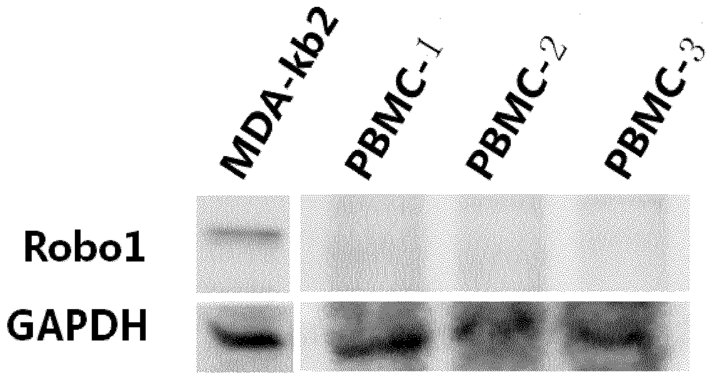


Fig. 17a

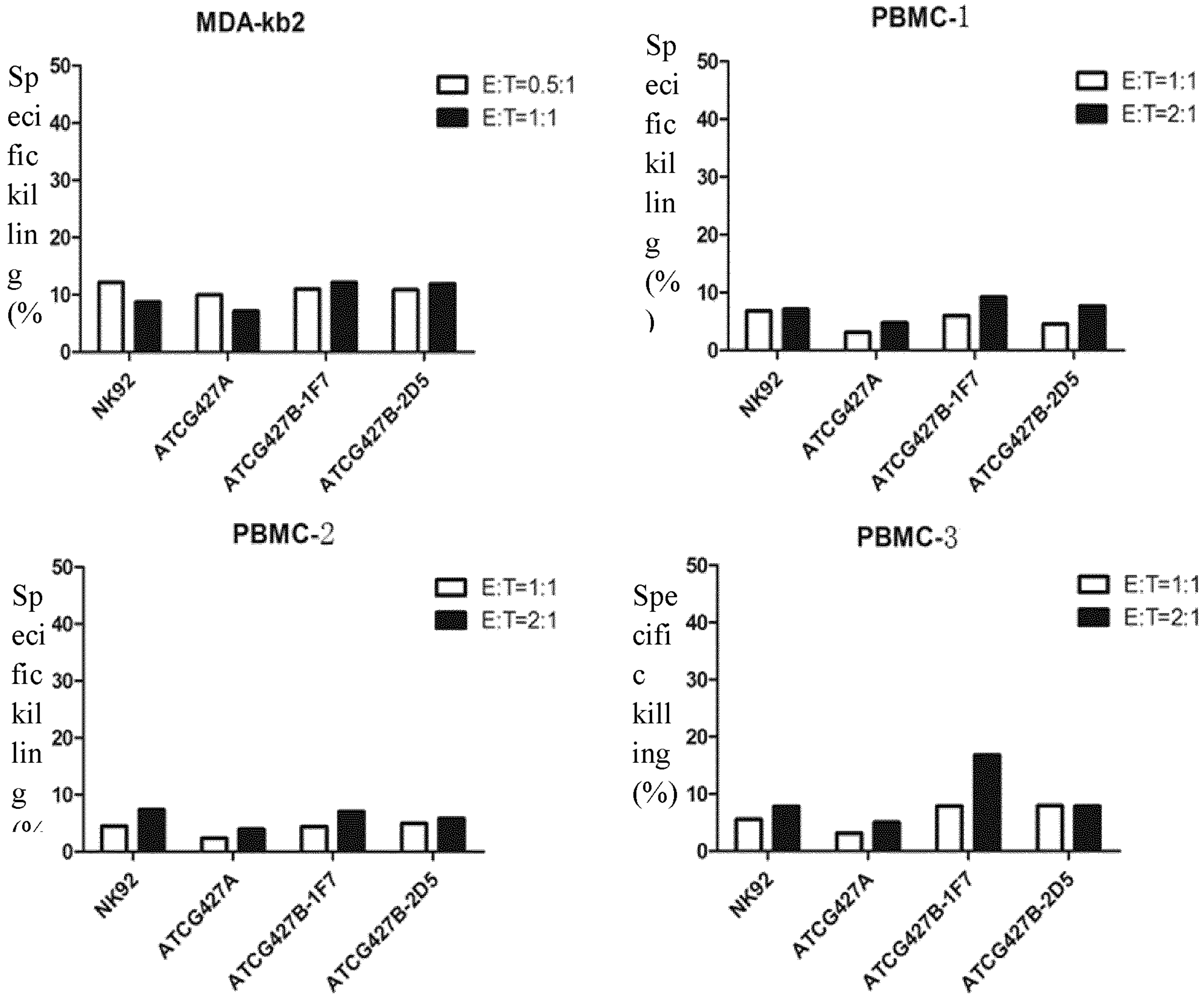


Fig. 17b

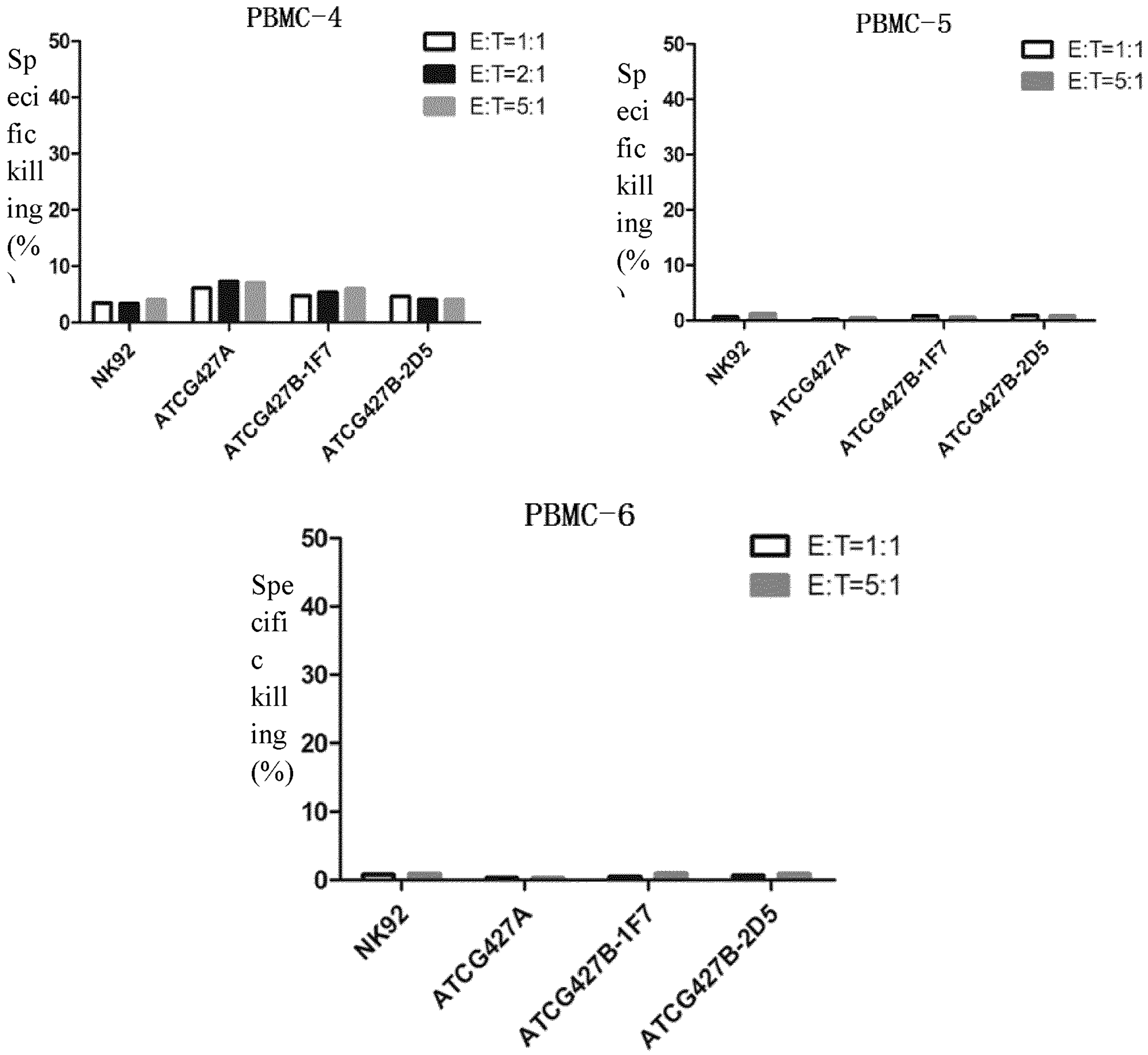
PBMC-4
Specific killing (%)
E:T=1:1
E:T=2:1
E:T=5:1
NK92
ATCG427A
ATCG427B-1F7
ATCG427B-2D5
PBMC-5
Specific killing (%)
E:T=1:1
E:T=5:1
NK92
ATCG427A
ATCG427B-1F7
ATCG427B-2D5
PBMC-6
Specific killing (%)
E:T=1:1
E:T=5:1
NK92
ATCG427A
ATCG427B-1F7
ATCG427B-2D5

Fig. 18

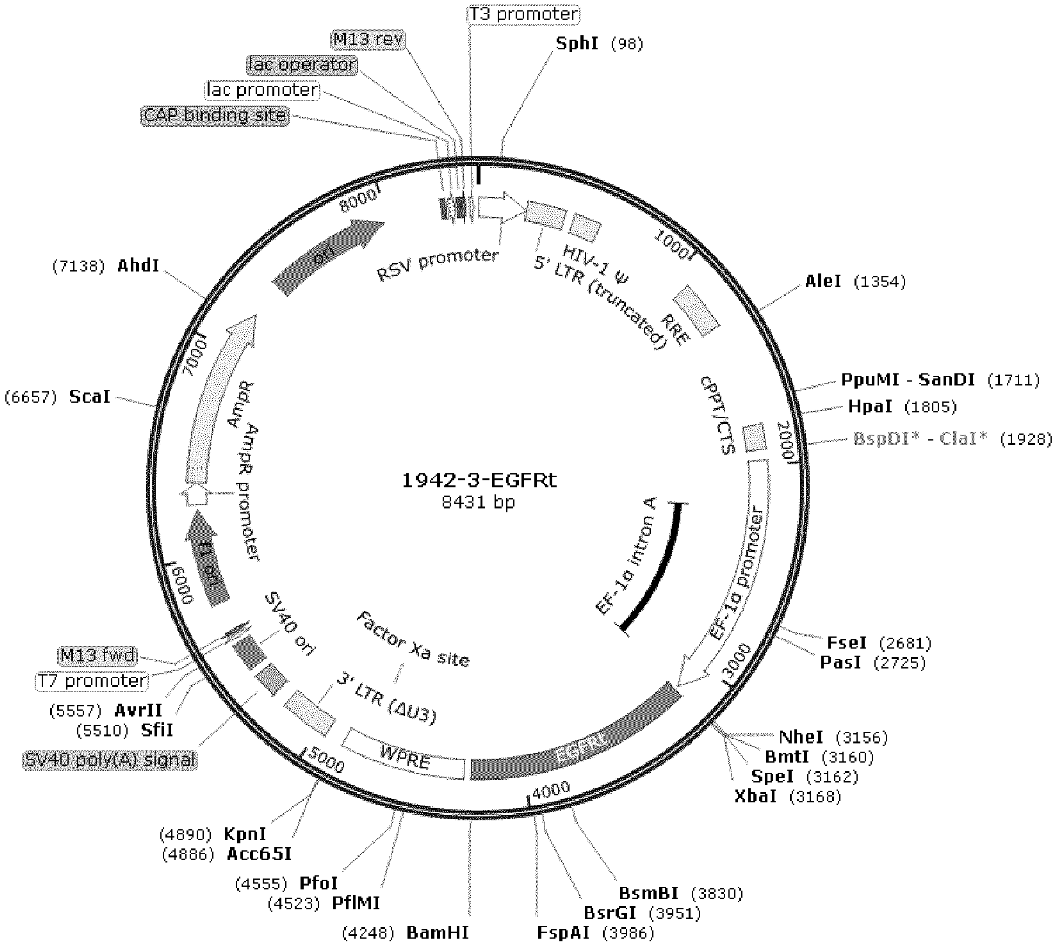
1942-3-EGFRt
8431 bp
T3 promoter
M13 rev
lac operator
lac promoter
CAP binding site
SphI (98)
AleI (1354)
PpuMI - SanDI (1711)
HpaI (1805)
BspDI* - ClaI* (1928)
FseI (2681)
PasI (2725)
NheI (3156)
BmtI (3160)
SpeI (3162)
XbaI (3168)
BsmBI (3830)
BsrGI (3951)
FspAI (3986)
(4248) BamHI
(4555) PfoI
(4523) PflMI
(4890) KpnI
(4886) Acc65I
(5557) AvrII
(5510) SfiI
SV40 poly(A) signal
M13 fwd
T7 promoter
(6657) ScaI
(7138) AhdI
ori
RSV promoter
HIV-1 ψ
5' LTR (truncated)
RRE
cPPT/CTS
EF-1α intron A
EF-1α promoter
EGFRt
WPRE
3' LTR (ΔU3)
Factor Xa site
SV40 ori
f1 ori
AmpR promoter
AmpR
1000
2000
3000
4000
5000
6000
7000
8000

Fig. 19

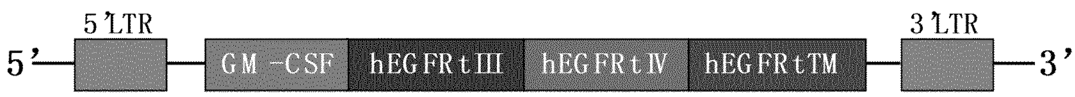
5'LTR
3'LTR
5'
3'
GM-CSF
hEGFRtIII
hEGFRtIV
hEGFRtTM

Fig. 20

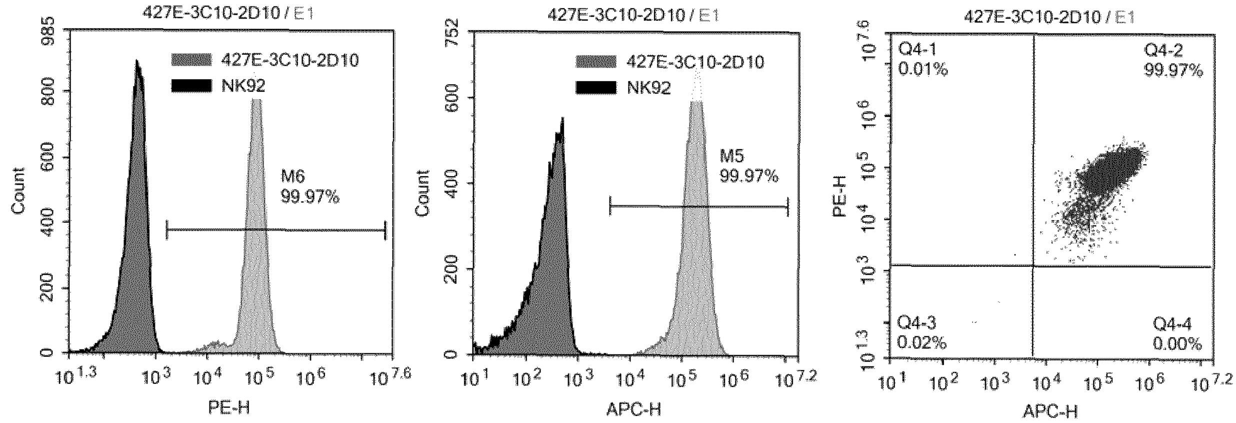


Fig. 21a

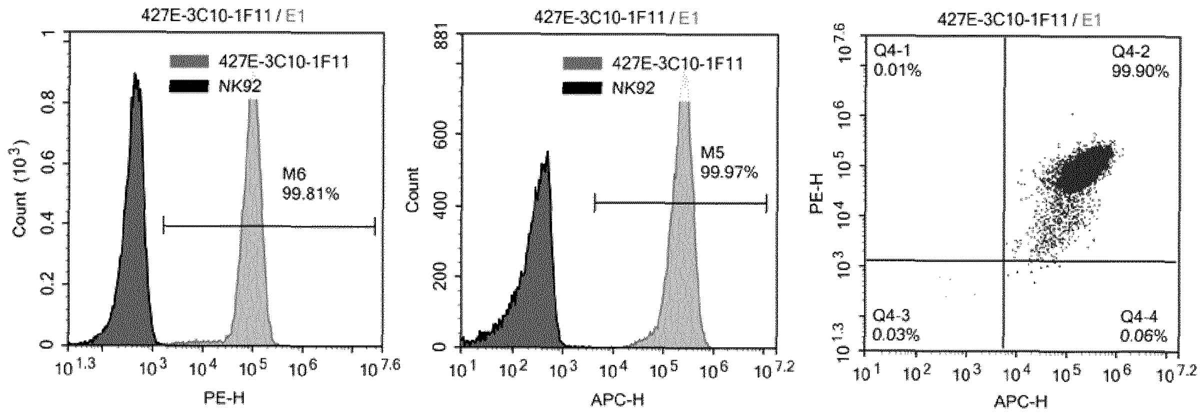


Fig. 21b

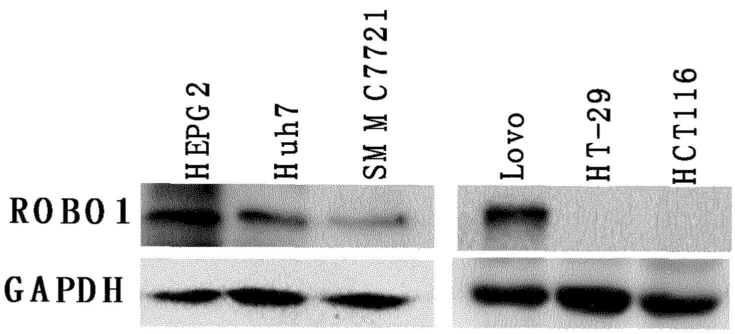


Fig. 22

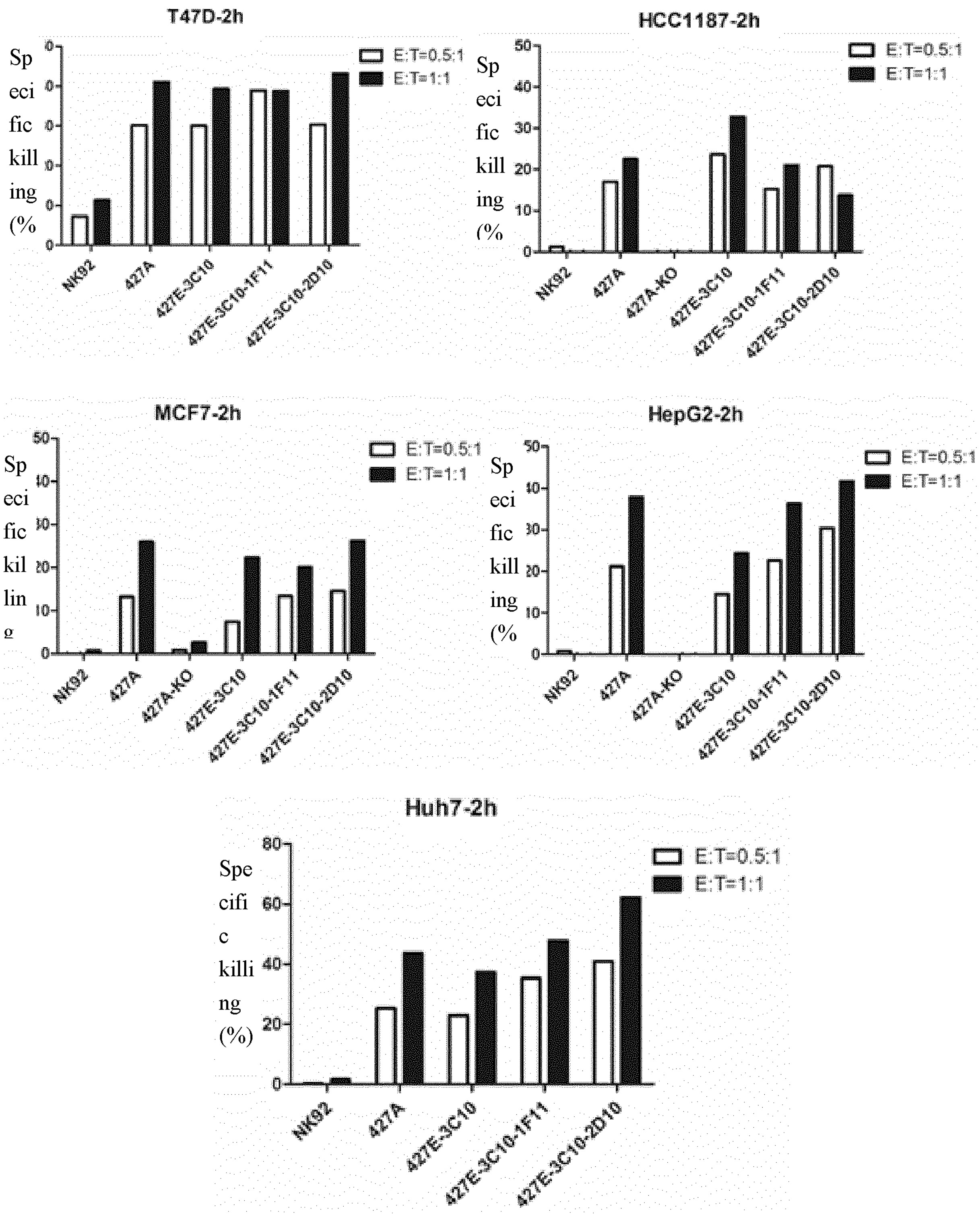
T47D-2h
Sp
eci
fic
kill
ing
(%
E:T=0.5:1
E:T=1:1
NK92
427A
427E-3C10
427E-3C10-1F11
427E-3C10-2D10
HCC1187-2h
Sp
eci
fic
kill
ing
(%
0
10
20
30
40
50
E:T=0.5:1
E:T=1:1
NK92
427A
427A-KO
427E-3C10
427E-3C10-1F11
427E-3C10-2D10
MCF7-2h
Sp
eci
fic
kil
lin
g
0
10
20
30
40
50
E:T=0.5:1
E:T=1:1
NK92
427A
427A-KO
427E-3C10
427E-3C10-1F11
427E-3C10-2D10
HepG2-2h
Sp
eci
fic
kill
ing
(%
0
10
20
30
40
50
E:T=0.5:1
E:T=1:1
NK92
427A
427A-KO
427E-3C10
427E-3C10-1F11
427E-3C10-2D10
Huh7-2h
Spe
cifi
c
killi
ng
(%)
0
20
40
60
80
E:T=0.5:1
E:T=1:1
NK92
427A
427E-3C10
427E-3C10-1F11
427E-3C10-2D10

Fig. 23a

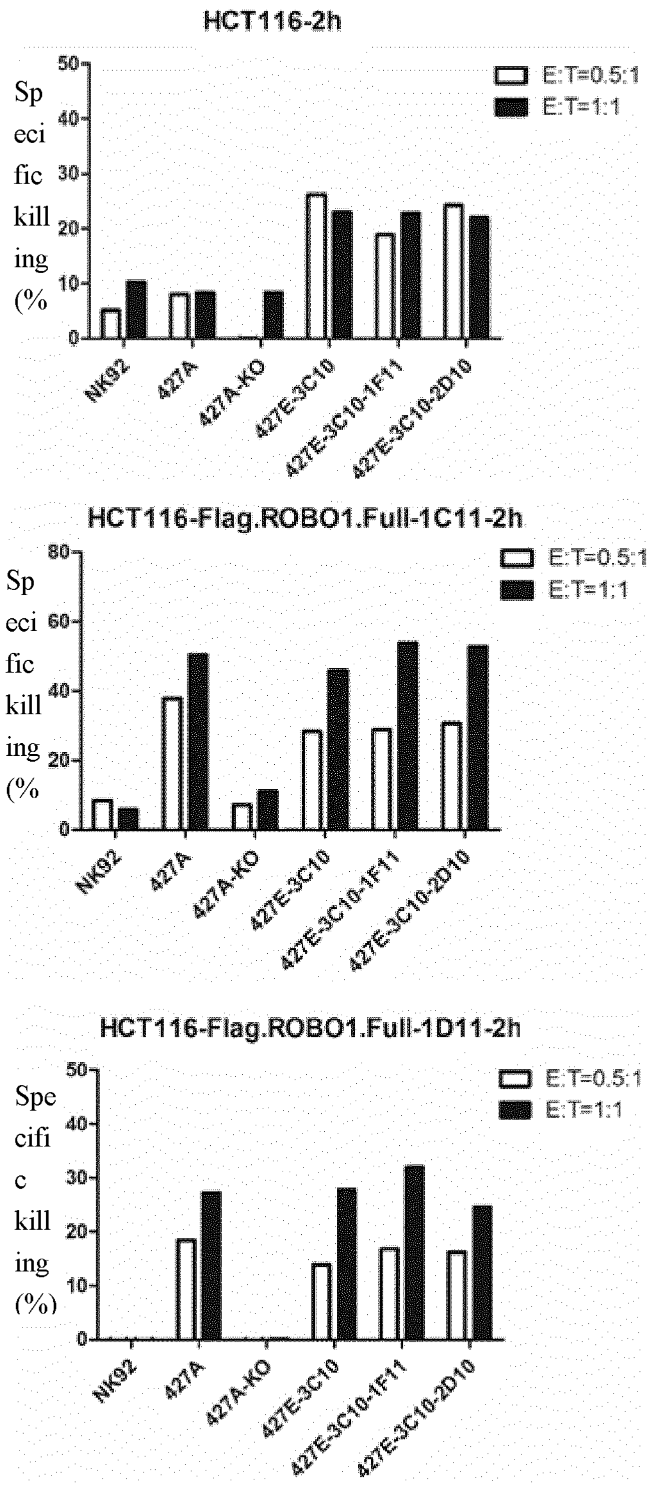
HCT116-2h
Specific killing (%
E:T=0.5:1
E:T=1:1
0
10
20
30
40
50
NK92
427A
427A-KO
427E-3C10
427E-3C10-1F11
427E-3C10-2D10
HCT116-Flag.ROBO1.Full-1C11-2h
Specific killing (%
E:T=0.5:1
E:T=1:1
0
20
40
60
80
NK92
427A
427A-KO
427E-3C10
427E-3C10-1F11
427E-3C10-2D10
HCT116-Flag.ROBO1.Full-1D11-2h
Specific killing (%)
E:T=0.5:1
E:T=1:1
0
10
20
30
40
50
NK92
427A
427A-KO
427E-3C10
427E-3C10-1F11
427E-3C10-2D10

Fig. 23b

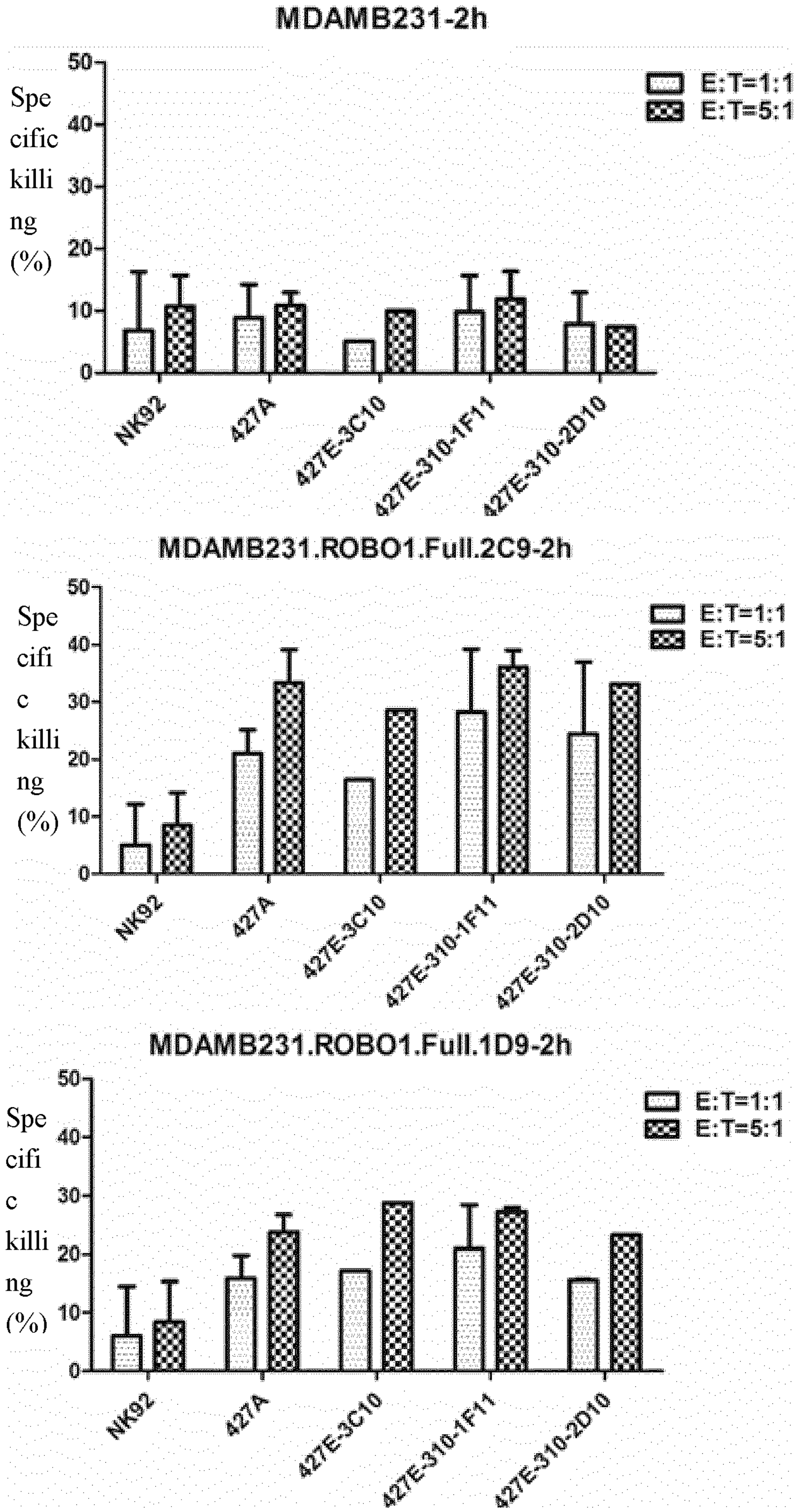
MDAMB231-2h
Specific killing (%)
50
40
30
20
10
0
E:T=1:1
E:T=5:1
NK92
427A
427E-3C10
427E-310-1F11
427E-310-2D10
MDAMB231.ROBO1.Full.2C9-2h
Specific killing (%)
50
40
30
20
10
0
E:T=1:1
E:T=5:1
NK92
427A
427E-3C10
427E-310-1F11
427E-310-2D10
MDAMB231.ROBO1.Full.1D9-2h
Specific killing (%)
50
40
30
20
10
0
E:T=1:1
E:T=5:1
NK92
427A
427E-3C10
427E-310-1F11
427E-310-2D10

Fig. 23c

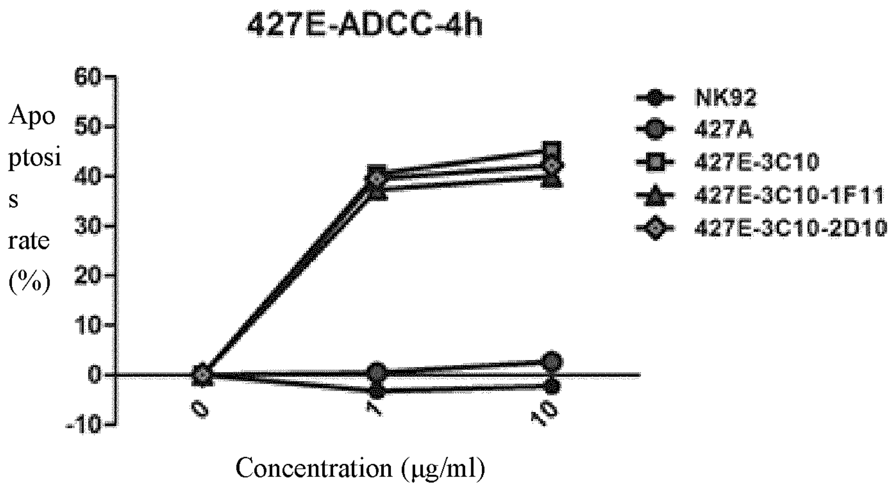
427E-ADCC-4h
Apoptosis rate (%)
60
50
40
30
20
10
0
-10
0
1
10
Concentration (μg/ml)
NK92
427A
427E-3C10
427E-3C10-1F11
427E-3C10-2D10

Fig. 24

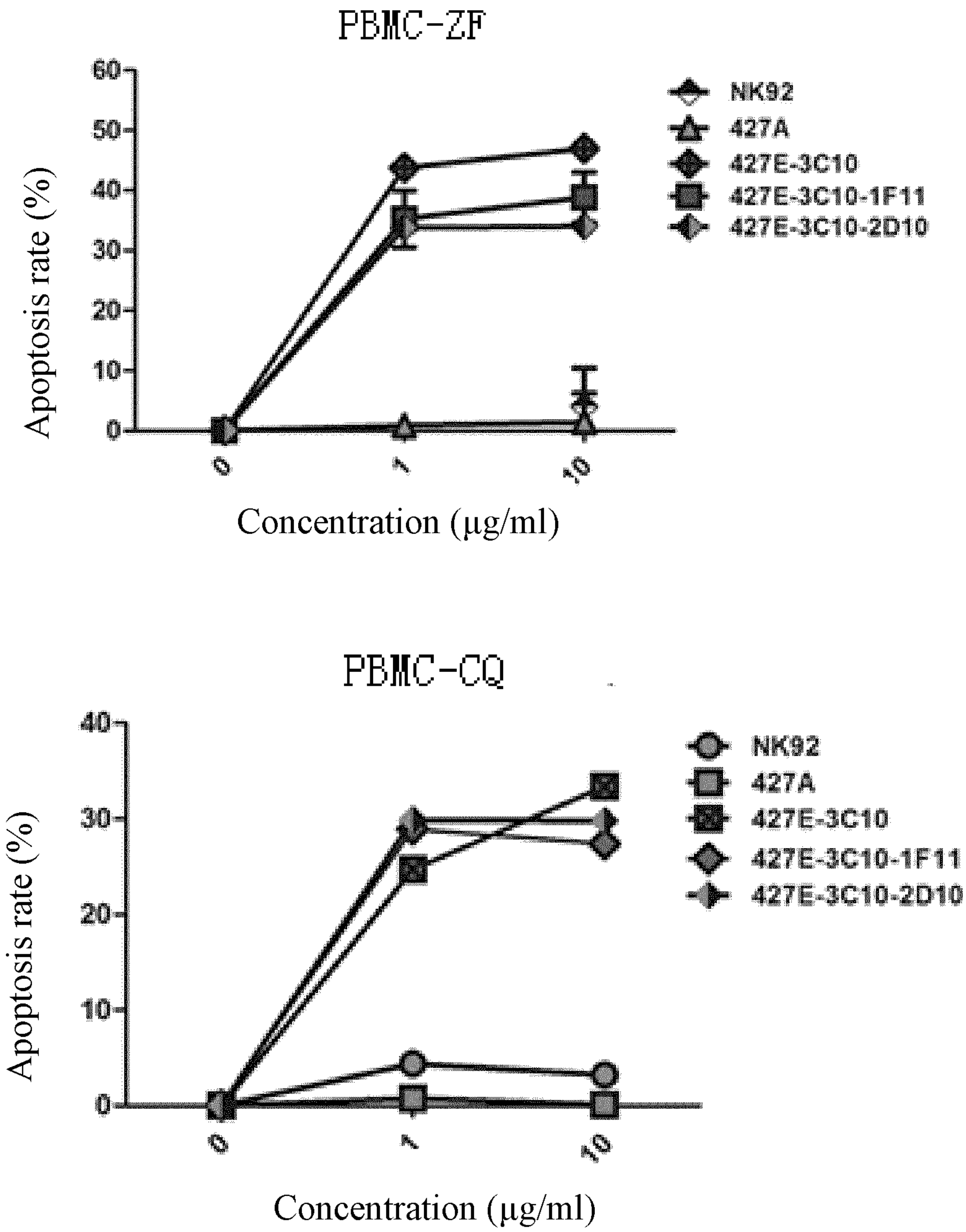
PBMC-ZF
Apoptosis rate (%)
60
50
40
30
20
10
0
0
1
10
Concentration (μg/ml)
NK92
427A
427E-3C10
427E-3C10-1F11
427E-3C10-2D10
PBMC-CQ
Apoptosis rate (%)
40
30
20
10
0
0
1
10
Concentration (μg/ml)
NK92
427A
427E-3C10
427E-3C10-1F11
427E-3C10-2D10

Fig. 25

**INTERNATIONAL SEARCH REPORT**

International application No.
**PCT/CN2020/077582**

**A. CLASSIFICATION OF SUBJECT MATTER**

C12N 15/62(2006.01)i; C07K 19/00(2006.01)i; C12N 15/867(2006.01)i; C12N 5/10(2006.01)i; A61K 35/17(2015.01)i; A61P 35/00(2006.01)i

According to International Patent Classification (IPC) or to both national classification and IPC

**B. FIELDS SEARCHED**

Minimum documentation searched (classification system followed by classification symbols)

C12N; C07K; A61K; A61P

Documentation searched other than minimum documentation to the extent that such documents are included in the fields searched

Electronic data base consulted during the international search (name of data base and, where practicable, search terms used)

Databases: CNABS, CNTXT, SIPOABS, DWPI, WOTXT, CNKI, 百度学术, BAIDU SCHOLAR, PUBMED, GENBANK +DDBJ+EMBL, 中国专利生物序列检索系统, CHINA PATENT BIOLOGICAL SEQUENCE SEARCH SYSTEM; Search terms: 自杀基因, 嵌合抗原受体, 雷帕霉素, ROBO1, suicide gene, chimeric antigen receptor, CAR, CART, CAR-T, CAR T, CD20, RQR8, iCaspase9, EGFRt, sirolimus, rapamycin, search for SEQ ID NO: 7 and 13

**C. DOCUMENTS CONSIDERED TO BE RELEVANT**

| Category* | Citation of document, with indication, where appropriate, of the relevant passages | Relevant to claim No. |
|---|---|---|
| A | CN 108977453 A (ASCLEPIUS (SUZHOU) BIOTECHNOLOGY CO., LTD.) 11 December 2018 (2018-12-11) see claims 1-27 | 1-28 |
| A | CN 107365798 A (SHANDONG QILU CELL THERAPY ENGINEERING AND TECHNOLOGY CO., LTD.) 21 November 2017 (2017-11-21) see claims 1-10 | 1-28 |
| A | US 2013071414 A1 (DOTTI, G. et al.) 21 March 2013 (2013-03-21) see entire document | 1-28 |

☐ Further documents are listed in the continuation of Box C.  ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07 April 2020** | **17 April 2020** |

| Name and mailing address of the ISA/CN | Authorized officer |
|---|---|
| **China National Intellectual Property Administration**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/077582**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **29-34**
because they relate to subject matter not required to be searched by this Authority, namely:
[1] A treatment method for human or animal diseases

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/077582**

| Patent document cited in search report | Publication date (day/month/year) | Patent family member(s) | Publication date (day/month/year) |
|---|---|---|---|
| CN 108977453 A | 11 December 2018 | WO 2018218710 A1 | 06 December 2018 |
| CN 107365798 A | 21 November 2017 | None | |
| US 2013071414 A1 | 21 March 2013 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ANNA SCHERBAKOVA.** *A Mathematical Model of Natural Killer Cell Activity*

Sequence Listing

<110> ASCLEPIUS (SUZHOU) TECHNOLOGY COMPANY GROUP CO., LTD.

<120> ROBO1 CAR-NK CELL CARRYING SUICIDE GENE, PREPARATION METHOD AND APPLICATION THEREOF

<130> 1062

<160> 13

<170> PatentIn version 3.5

<210> 1

<211> 324

<212> DNA

<213> Artificial sequence

<220>

<223> SEQ ID NO: 1

<400> 1

atgggagtgc aggtggaaac catctcccca ggagacgggc gcaccttccc caagcgcggc 60

cagacctgcg tggtgcacta caccgggatg cttgaagatg gaaagaaagt tgattcctcc 120

cgggacagaa acaagccctt taagtttatg ctaggcaagc aggaggtgat ccgaggctgg 180

gaagaagggg ttgcccagat gagtgtgggt cagagagcca aactgactat atctccagat 240

tatgcctatg gtgccactgg gcacccaggc atcatcccac cacatgccac tctcgtcttc 300

gatgtggagc ttctaaaact ggaa 324

<210> 2

<211> 846

<212> DNA

<213> Artificial sequence

<220>

<223> SEQ ID NO: 2

<400> 2

ggatttggtg atgtcggtgc tcttgagagt ttgaggggaa atgcagattt ggcttacatc 60
ctgagcatgg agccctgtgg ccactgcctc attatcaaca atgtgaactt ctgccgtgag 120
tccgggctcc gcacccgcac tggctccaac atcgactgtg agaagttgcg gcgtcgcttc 180
tcctcgctgc atttcatggt ggaggtgaag ggcgacctga ctgccaagaa aatggtgctg 240
gctttgctgg agctggcgca gcaggaccac ggtgctctgg actgctgcgt ggtggtcatt 300
ctctctcacg gctgtcaggc cagccacctg cagttcccag ggctgtcta cggcacagat 360
ggatgccctg tgtcggtcga gaagattgtg aacatcttca atgggaccag ctgccccagc 420
ctgggaggga agcccaagct ctttttcatc caggcctgtg gtggggagca gaaagaccat 480
gggtttgagg tggcctccac ttcccctgaa gacgagtccc ctggcagtaa ccccgagcca 540
gatgccaccc cgttccagga aggtttgagg accttcgacc agctggacgc catatctagt 600
ttgcccacac ccagtgacat ctttgtgtcc tactctactt tcccaggttt tgtttcctgg 660
agggacccca agagtggctc ctggtacgtt gagaccctgg acgacatctt tgagcagtgg 720
gctcactctg aagacctgca gtccctcctg cttagggtcg ctaatgctgt ttcggtgaaa 780
gggatttata aacagatgcc tggttgcttt aatttcctcc ggaaaaaact tttctttaaa 840
acatca 846

<210> 3
<211> 24
<212> DNA
<213> Artificial sequence

<220>
<223> SEQ ID NO: 3

<400> 3

tccggaggag gatccggagt cgac 24

<210> 4

<211> 1032

<212> DNA

<213> Artificial sequence

<220>

<223> SEQ ID NO: 4

<400> 4

atgccacctc ctcgcctcct cttcttcctc ctcttcctca cccccatgga agtcaggccc 60

gaggaacctc tagtggtgaa ggtggaagag ggagataacg ctgtgctgca gtgcctcaag 120

gggacctcag atggccccac tcagcagctg acctggtctc gggagtcccc gcttaaaccc 180

ttcttaaaac tcagcctggg gctgccaggc ctgggaatcc acatgaggcc cctggccatc 240

tggcttttca tcttcaacgt ctctcaacag atgggggcct tctacctgtg ccagccgggg 300

ccccctctg agaaggcctg gcagcctggc tggacagtca atgtggaggg cagcggggag 360

ctgttccggt ggaatgtttc ggacctaggt ggcctgggct gtggcctgaa gaacaggtcc 420

tcagagggcc ccagctcccc ttccgggaag ctcatgagcc ccaagctgta tgtgtgggcc 480

aaagaccgcc ctgagatctg ggagggagag cctccgtgtc tcccaccgag ggacagcctg 540

aaccagagcc tcagccagga cctcaccatg gcccctggct ccacactctg gctgtcctgt 600

ggggtacccc ctgactctgt gtccaggggc cccctctcct ggacccatgt gcaccccaag 660

gggcctaagt cattgctgag cctagagctg aaggacgatc gcccggccag agatatgtgg 720

gtaatggaga cgggtctgtt gttgccccgg gccacagctc aagacgctgg aaagtattat 780

tgtcaccgtg gcaacctgac catgtcattc cacctggaga tcactgctcg gccagtacta 840

tggcactggc tgctgaggac tggtggctgg aaggtctcag ctgtgacttt ggcttatctg 900

atcttctgcc tgtgttccct tgtgggcatt cttcatcttc aaagagccct ggtcctgagg 960

aggaaaagaa agcgaatgac tgaccccacc aggagattct tcaaagtgac gcctccccca 1020

ggaagcgggt ga 1032

<210> 5

<211> 69

<212> DNA

<213> Artificial sequence

<220>

<223> SEQ ID NO: 5

<400> 5

gaattcggca gtggagaggg cagaggaagt ctgctaacat gcggtgacgt cgaggagaat 60

cctggccca 69

<210> 6

<211> 2295

<212> DNA

<213> Artificial sequence

<220>

<223> SEQ ID NO: 6

<400> 6

atgggagtgc aggtggaaac catctcccca ggagacgggc gcaccttccc caagcgcggc 60

cagacctgcg tggtgcacta caccgggatg cttgaagatg gaaagaaagt tgattcctcc 120

cgggacagaa acaagccctt taagtttatg ctaggcaagc aggaggtgat ccgaggctgg 180

gaagaagggg ttgcccagat gagtgtgggt cagagagcca aactgactat atctccagat 240

tatgcctatg gtgccactgg gcacccaggc atcatcccac cacatgccac tctcgtcttc 300

gatgtggagc ttctaaaact ggaatccgga ggaggatccg gagtcgacgg atttggtgat 360

gtcggtgctc ttgagagttt gaggggaaat gcagatttgg cttacatcct gagcatggag 420

ccctgtggcc actgcctcat tatcaacaat gtgaacttct gccgtgagtc cgggctccgc 480

acccgcactg gctccaacat cgactgtgag aagttgcggc gtcgcttctc ctcgctgcat 540

ttcatggtgg aggtgaaggg cgacctgact gccaagaaaa tggtgctggc tttgctggag 600

ctggcgcagc aggaccacgg tgctctggac tgctgcgtgg tggtcattct ctctcacggc 660

tgtcaggcca gccacctgca gttcccaggg gctgtctacg gcacagatgg atgccctgtg 720
tcggtcgaga agattgtgaa catcttcaat gggaccagct gcccccagcct gggagggaag 780
cccaagctct ttttcatcca ggcctgtggt ggggagcaga aagaccatgg gtttgaggtg 840
gcctccactt cccctgaaga cgagtcccct ggcagtaacc ccgagccaga tgccaccccg 900
ttccaggaag gtttgaggac cttcgaccag ctggacgcca tatctagttt gcccacaccc 960
agtgacatct ttgtgtccta ctctactttc ccaggttttg tttcctggag ggacccccaag 1020
agtggctcct ggtacgttga gaccctggac gacatctttg agcagtgggc tcactctgaa 1080
gacctgcagt ccctcctgct tagggtcgct aatgctgttt cggtgaaagg gatttataaa 1140
cagatgcctg gttgctttaa tttcctccgg aaaaaacttt tctttaaaac atcagaattc 1200
ggcagtggag agggcagagg aagtctgcta acatgcggtg acgtcgagga gaatcctggc 1260
ccaatgccac ctcctcgcct cctcttcttc ctcctcttcc tcacccccat ggaagtcagg 1320
cccgaggaac ctctagtggt gaaggtggaa gagggagata acgctgtgct gcagtgcctc 1380
aaggggacct cagatggccc cactcagcag ctgacctggt ctcgggagtc cccgcttaaa 1440
cccttcttaa aactcagcct ggggctgcca ggcctgggaa tccacatgag gcccctggcc 1500
atctggcttt tcatcttcaa cgtctctcaa cagatggggg gcttctacct gtgccagccg 1560
gggccccct ctgagaaggc ctggcagcct ggctggacag tcaatgtgga gggcagcggg 1620
gagctgttcc ggtggaatgt ttcggaccta ggtggcctgg gctgtggcct gaagaacagg 1680
tcctcagagg gccccagctc cccttccggg aagctcatga gccccaagct gtatgtgtgg 1740
gccaaagacc gccctgagat ctgggaggga gagcctccgt gtctcccacc gagggacagc 1800
ctgaaccaga gcctcagcca ggacctcacc atggcccctg gctccacact ctggctgtcc 1860
tgtggggtac cccctgactc tgtgtccagg ggcccccctct cctggaccca tgtgcacccc 1920
aaggggccta agtcattgct gagcctagag ctgaaggacg atcgcccggc cagagatatg 1980
tgggtaatgg agacgggtct gttgttgccc cgggccacag ctcaagacgc tggaaagtat 2040
tattgtcacc gtggcaacct gaccatgtca ttccacctgg agatcactgc tcggccagta 2100
ctatggcact ggctgctgag gactggtggc tggaaggtct cagctgtgac tttggcttat 2160
ctgatcttct gcctgtgttc ccttgtgggc attcttcatc ttcaaagagc cctggtcctg 2220
aggaggaaaa gaaagcgaat gactgacccc accaggagat tcttcaaagt gacgcctccc 2280
ccaggaagcg ggtga 2295

<210> 7

<211> 9624

<212> DNA

<213> Artificial sequence

<220>

<223> SEQ ID NO: 7

<400> 7

agcttaatgt agtcttatgc aatactcttg tagtcttgca acatggtaac gatgagttag 60

caacatgcct tacaaggaga gaaaaagcac cgtgcatgcc gattggtgga agtaaggtgg 120

tacgatcgtg ccttattagg aaggcaacag acgggtctga catggattgg acgaaccact 180

gaattgccgc attgcagaga tattgtattt aagtgcctag ctcgatacat aaacgggtct 240

ctctggttag accagatctg agcctgggag ctctctggct aactagggaa cccactgctt 300

aagcctcaat aaagcttgcc ttgagtgctt caagtagtgt gtgcccgtct gttgtgtgac 360

tctggtaact agagatccct cagacccttt tagtcagtgt ggaaaatctc tagcagtggc 420

gcccgaacag ggacttgaaa gcgaaaggga aaccagagga gctctctcga cgcaggactc 480

ggcttgctga agcgcgcacg gcaagaggcg aggggcggcg actggtgagt acgccaaaaa 540

ttttgactag cggaggctag aaggagagag atgggtgcga gagcgtcagt attaagcggg 600

ggagaattag atcgcgatgg gaaaaaattc ggttaaggcc agggggaaag aaaaaatata 660

aattaaaaca tatagtatgg gcaagcaggg agctagaacg attcgcagtt aatcctggcc 720

tgttagaaac atcagaaggc tgtagacaaa tactgggaca gctacaacca tcccttcaga 780

caggatcaga agaacttaga tcattatata atacagtagc aaccctctat tgtgtgcatc 840

aaaggataga gataaaagac accaaggaag ctttagacaa gatagaggaa gagcaaaaca 900

aaagtaagac caccgcacag caagcggccg ctgatcttca gacctggagg aggagatatg 960

agggacaatt ggagaagtga attatataaa tataaagtag taaaaattga accattagga 1020

gtagcaccca ccaaggcaaa gagaagagtg gtgcagagag aaaaaagagc agtgggaata 1080

ggagctttgt tccttgggtt cttgggagca gcaggaagca ctatgggcgc agcgtcaatg 1140

acgctgacgg tacaggccag acaattattg tctggtatag tgcagcagca gaacaatttg 1200

ctgagggcta ttgaggcgca acagcatctg ttgcaactca cagtctgggg catcaagcag 1260

ctccaggcaa gaatcctggc tgtggaaaga tacctaaagg atcaacagct cctggggatt 1320

tggggttgct ctggaaaact catttgcacc actgctgtgc cttggaatgc tagttggagt 1380
aataaatctc tggaacagat ttggaatcac acgacctgga tggagtggga cagagaaatt 1440
aacaattaca caagcttaat acactcctta attgaagaat cgcaaaacca gcaagaaaag 1500
aatgaacaag aattattgga attagataaa tgggcaagtt tgtggaattg gtttaacata 1560
acaaattggc tgtggtatat aaaattattc ataatgatag taggaggctt ggtaggttta 1620
agaatagttt ttgctgtact ttctatagtg aatagagtta ggcagggata ttcaccatta 1680
tcgtttcaga cccacctccc aaccccgagg ggacccgaca ggcccgaagg aatagaagaa 1740
gaaggtggag agagagacag agacagatcc attcgattag tgaacggatc tcgacggtat 1800
cggttaactt ttaaaagaaa aggggggatt ggggggtaca gtgcagggga aagaatagta 1860
gacataatag caacagacat acaaactaaa gaattacaaa aacaaattac aaaaattcaa 1920
aattttatcg atcacgagac tagcctcgag aagcttgata tcgaattcca ccgtgaggct 1980
ccggtgcccg tcagtgggca gagcgcacat cgcccacagt ccccgagaag ttggggggag 2040
gggtcggcaa ttgaaccggt gcctagagaa ggtggcgcgg ggtaaactgg gaaagtgatg 2100
tcgtgtactg gctccgcctt tttcccgagg gtgggggaga accgtatata agtgcagtag 2160
tcgccgtgaa cgttcttttt cgcaacgggt ttgccgccag aacacaggta agtgccgtgt 2220
gtggttcccg cgggcctggc ctctttacgg gttatggccc ttgcgtgcct tgaattactt 2280
ccacctggct gcagtacgtg attcttgatc ccgagcttcg ggttggaagt gggtgggaga 2340
gttcgaggcc ttgcgcttaa ggagcccctt cgcctcgtgc ttgagttgag gcctggcctg 2400
ggcgctgggg ccgccgcgtg cgaatctggt ggcaccttcg cgcctgtctc gctgctttcg 2460
ataagtctct agccatttaa aatttttgat gacctgctgc gacgcttttt ttctggcaag 2520
atagtcttgt aaatgcgggc caagatctgc acactggtat ttcggttttt ggggccgcgg 2580
gcggcgacgg ggcccgtgcg tcccagcgca catgttcggc gaggcggggc ctgcgagcgc 2640
ggccaccgag aatcggacgg gggtagtctc aagctggccg gcctgctctg gtgcctggcc 2700
tcgcgccgcc gtgtatcgcc ccgccctggg cggcaaggct ggcccggtcg gcaccagttg 2760
cgtgagcgga aagatggccg cttcccggcc ctgctgcagg gagctcaaaa tggaggacgc 2820
ggcgctcggg agagcgggcg ggtgagtcac ccacacaaag gaaaagggcc tttccgtcct 2880
cagccgtcgc ttcatgtgac tccacggagt accgggcgcc gtccaggcac ctcgattagt 2940
tctcgagctt ttggagtacg tcgtctttag gttgggggga ggggttttat gcgatggagt 3000
ttccccacac tgagtgggtg gagactgaag ttaggccagc ttggcacttg atgtaattct 3060
ccttggaatt tgcccttttt gagtttggat cttggttcat tctcaagcct cagacagtgg 3120

ttcaaagttt ttttcttcca tttcaggtgt cgtgagctag cactagttct agaatgggag 3180

tgcaggtgga aaccatctcc ccaggagacg ggcgcacctt ccccaagcgc ggccagacct 3240

gcgtggtgca ctacaccggg atgcttgaag atggaaagaa agttgattcc tcccgggaca 3300

gaaacaagcc ctttaagttt atgctaggca agcaggaggt gatccgaggc tgggaagaag 3360

gggttgccca gatgagtgtg ggtcagagag ccaaactgac tatatctcca gattatgcct 3420

atggtgccac tgggcaccca ggcatcatcc caccacatgc cactctcgtc ttcgatgtgg 3480

agcttctaaa actggaatcc ggaggaggat ccggagtcga cggatttggt gatgtcggtg 3540

ctcttgagag tttgagggga aatgcagatt tggcttacat cctgagcatg gagccctgtg 3600

gccactgcct cattatcaac aatgtgaact tctgccgtga gtccgggctc cgcacccgca 3660

ctggctccaa catcgactgt gagaagttgc ggcgtcgctt ctcctcgctg catttcatgg 3720

tggaggtgaa gggcgacctg actgccaaga aaatggtgct ggctttgctg gagctggcgc 3780

agcaggacca cggtgctctg gactgctgcg tggtggtcat tctctctcac ggctgtcagg 3840

ccagccacct gcagttccca ggggctgtct acggcacaga tggatgccct gtgtcggtcg 3900

agaagattgt gaacatcttc aatgggacca gctgccccag cctgggaggg aagcccaagc 3960

tctttttcat ccaggcctgt ggtggggagc agaaagacca tgggtttgag gtggcctcca 4020

cttcccctga agacgagtcc cctggcagta accccgagcc agatgccacc ccgttccagg 4080

aaggtttgag gaccttcgac cagctggacg ccatatctag tttgcccaca cccagtgaca 4140

tctttgtgtc ctactctact ttcccaggtt ttgtttcctg gagggacccc aagagtggct 4200

cctggtacgt tgagaccctg gacgacatct ttgagcagtg ggctcactct gaagacctgc 4260

agtccctcct gcttagggtc gctaatgctg tttcggtgaa agggatttat aaacagatgc 4320

ctggttgctt taatttcctc cggaaaaaac ttttctttaa aacatcagaa ttcggcagtg 4380

gagagggcag aggaagtctg ctaacatgcg gtgacgtcga ggagaatcct ggcccaatgc 4440

cacctcctcg cctcctcttc ttcctcctct tcctcacccc catggaagtc aggcccgagg 4500

aacctctagt ggtgaaggtg gaagagggag ataacgctgt gctgcagtgc ctcaagggga 4560

cctcagatgg ccccactcag cagctgacct ggtctcggga gtccccgctt aaacccttct 4620

taaaactcag cctggggctg ccaggcctgg gaatccacat gaggcccctg gccatctggc 4680

ttttcatctt caacgtctct caacagatgg ggggcttcta cctgtgccag ccggggcccc 4740

cctctgagaa ggcctggcag cctggctgga cagtcaatgt ggagggcagc ggggagctgt 4800

tccggtggaa tgtttcggac ctaggtggcc tgggctgtgg cctgaagaac aggtcctcag 4860

agggccccag ctccccttcc gggaagctca tgagccccaa gctgtatgtg tgggccaaag 4920

accgccctga gatctgggag ggagagcctc cgtgtctccc accgagggac agcctgaacc 4980
agagcctcag ccaggacctc accatggccc ctggctccac actctggctg tcctgtgggg 5040
taccccctga ctctgtgtcc aggggccccc tctcctggac ccatgtgcac cccaaggggc 5100
ctaagtcatt gctgagccta gagctgaagg acgatcgccc ggccagagat atgtgggtaa 5160
tggagacggg tctgttgttg ccccgggcca cagctcaaga cgctggaaag tattattgtc 5220
accgtggcaa cctgaccatg tcattccacc tggagatcac tgctcggcca gtactatggc 5280
actggctgct gaggactggt ggctggaagg tctcagctgt gactttggct tatctgatct 5340
tctgcctgtg ttcccttgtg ggcattcttc atcttcaaag agccctggtc ctgaggagga 5400
aaagaaagcg aatgactgac cccaccagga gattcttcaa agtgacgcct cccccaggaa 5460
gcgggtgagt cgacaatcaa cctctggatt acaaaatttg tgaaagattg actggtattc 5520
ttaactatgt tgctcctttt acgctatgtg gatacgctgc tttaatgcct ttgtatcatg 5580
ctattgcttc ccgtatggct ttcattttct cctccttgta taaatcctgg ttgctgtctc 5640
tttatgagga gttgtggccc gttgtcaggc aacgtggcgt ggtgtgcact gtgtttgctg 5700
acgcaacccc cactggttgg ggcattgcca ccacctgtca gctcctttcc gggactttcg 5760
ctttccccct ccctattgcc acggcggaac tcatcgccgc ctgccttgcc cgctgctgga 5820
caggggctcg gctgttgggc actgacaatt ccgtggtgtt gtcggggaag ctgacgtcct 5880
ttccatggct gctcgcctgt gttgccacct ggattctgcg cgggacgtcc ttctgctacg 5940
tcccttcggc cctcaatcca gcggaccttc cttcccgcgg cctgctgccg gctctgcggc 6000
ctcttccgcg tcttcgcctt cgccctcaga cgagtcggat ctccctttgg gccgcctccc 6060
cgcctggaat tcgagctcgg tacctttaag accaatgact tacaaggcag ctgtagatct 6120
tagccacttt ttaaaagaaa aggggggact ggaagggcta attcactccc aacgaagaca 6180
agatctgctt tttgcttgta ctgggtctct ctggttagac cagatctgag cctgggagct 6240
ctctggctaa ctagggaacc cactgcttaa gcctcaataa agcttgcctt gagtgcttca 6300
agtagtgtgt gcccgtctgt tgtgtgactc tggtaactag agatccctca gacccttta 6360
gtcagtgtgg aaaatctcta gcagtagtag ttcatgtcat cttattattc agtatttata 6420
acttgcaaag aaatgaatat cagagagtga gaggaacttg tttattgcag cttataatgg 6480
ttacaaataa agcaatagca tcacaaattt cacaaataaa gcattttttt cactgcattc 6540
tagttgtggt ttgtccaaac tcatcaatgt atcttatcat gtctggctct agctatcccg 6600
cccctaactc cgcccatccc gcccctaact ccgcccagtt ccgcccattc tccgccccat 6660
ggctgactaa ttttttttat ttatgcagag gccgaggccg cctcggcctc tgagctattc 6720

cagaagtagt gaggaggctt ttttggaggc ctagggacgt acccaattcg ccctatagtg 6780
agtcgtatta cgcgcgctca ctggccgtcg ttttacaacg tcgtgactgg gaaaaccctg 6840
gcgttaccca acttaatcgc cttgcagcac atccccctttt cgccagctgg cgtaatagcg 6900
aagaggcccg caccgatcgc ccttcccaac agttgcgcag cctgaatggc gaatgggacg 6960
cgccctgtag cggcgcatta agcgcggcgg gtgtggtggt tacgcgcagc gtgaccgcta 7020
cacttgccag cgccctagcg cccgctcctt tcgctttctt cccttccttt ctcgccacgt 7080
tcgccggctt tccccgtcaa gctctaaatc gggggctccc tttagggttc cgatttagtg 7140
ctttacggca cctcgacccc aaaaaacttg attagggtga tggttcacgt agtgggccat 7200
cgccctgata gacggttttt cgccctttga cgttggagtc cacgttcttt aatagtggac 7260
tcttgttcca aactggaaca acactcaacc ctatctcggt ctattctttt gatttataag 7320
ggattttgcc gatttcggcc tattggttaa aaaatgagct gatttaacaa aaatttaacg 7380
cgaattttaa caaaatatta acgcttacaa tttaggtggc acttttcggg gaaatgtgcg 7440
cggaacccct atttgtttat ttttctaaat acattcaaat atgtatccgc tcatgagaca 7500
ataaccctga taaatgcttc aataatattg aaaaaggaag agtatgagta ttcaacattt 7560
ccgtgtcgcc cttattccct tttttgcggc attttgcctt cctgtttttg ctcacccaga 7620
aacgctggtg aaagtaaaag atgctgaaga tcagttgggt gcacgagtgg gttacatcga 7680
actggatctc aacagcggta agatccttga gagttttcgc cccgaagaac gttttccaat 7740
gatgagcact tttaaagttc tgctatgtgg cgcggtatta tcccgtattg acgccgggca 7800
agagcaactc ggtcgccgca tacactattc tcagaatgac ttggttgagt actcaccagt 7860
cacagaaaag catcttacgg atggcatgac agtaagagaa ttatgcagtg ctgccataac 7920
catgagtgat aacactgcgg ccaacttact tctgacaacg atcggaggac cgaaggagct 7980
aaccgctttt ttgcacaaca tgggggatca tgtaactcgc cttgatcgtt gggaaccgga 8040
gctgaatgaa gccataccaa acgacgagcg tgacaccacg atgcctgtag caatggcaac 8100
aacgttgcgc aaactattaa ctggcgaact acttactcta gcttcccggc aacaattaat 8160
agactggatg gaggcggata aagttgcagg accacttctg cgctcggccc ttccggctgg 8220
ctggtttatt gctgataaat ctggagccgg tgagcgtggg tctcgcggta tcattgcagc 8280
actggggcca gatggtaagc cctcccgtat cgtagttatc tacacgacgg ggagtcaggc 8340
aactatggat gaacgaaata gacagatcgc tgagataggt gcctcactga ttaagcattg 8400
gtaactgtca gaccaagttt actcatatat actttagatt gatttaaaac ttcattttta 8460
atttaaaagg atctaggtga agatcctttt tgataatctc atgaccaaaa tcccttaacg 8520

tgagttttcg ttccactgag cgtcagaccc cgtagaaaag atcaaaggat cttcttgaga 8580

tcctttttt ctgcgcgtaa tctgctgctt gcaaacaaaa aaaccaccgc taccagcggt 8640

ggtttgtttg ccggatcaag agctaccaac tctttttccg aaggtaactg gcttcagcag 8700

agcgcagata ccaaatactg ttcttctagt gtagccgtag ttaggccacc acttcaagaa 8760

ctctgtagca ccgcctacat acctcgctct gctaatcctg ttaccagtgg ctgctgccag 8820

tggcgataag tcgtgtctta ccgggttgga ctcaagacga tagttaccgg ataaggcgca 8880

gcggtcgggc tgaacggggg gttcgtgcac acagcccagc ttggagcgaa cgacctacac 8940

cgaactgaga tacctacagc gtgagctatg agaaagcgcc acgcttcccg aagggagaaa 9000

ggcggacagg tatccggtaa gcggcagggt cggaacagga gagcgcacga gggagcttcc 9060

agggggaaac gcctggtatc tttatagtcc tgtcgggttt cgccacctct gacttgagcg 9120

tcgatttttg tgatgctcgt caggggggcg gagcctatgg aaaaacgcca gcaacgcggc 9180

ctttttacgg ttcctggcct tttgctggcc ttttgctcac atgttctttc ctgcgttatc 9240

ccctgattct gtggataacc gtattaccgc ctttgagtga gctgataccg ctcgccgcag 9300

ccgaacgacc gagcgcagcg agtcagtgag cgaggaagcg gaagagcgcc caatacgcaa 9360

accgcctctc cccgcgcgtt ggccgattca ttaatgcagc tggcacgaca ggtttcccga 9420

ctggaaagcg ggcagtgagc gcaacgcaat taatgtgagt tagctcactc attaggcacc 9480

ccaggcttta cactttatgc ttccggctcg tatgttgtgt ggaattgtga gcggataaca 9540

atttcacaca ggaaacagct atgaccatga ttacgccaag cgcgcaatta accctcacta 9600

aagggaacaa aagctggagc tgca 9624

<210> 8

<211> 480

<212> PRT

<213> Artificial sequence

<220>

<223> SEQ ID NO: 8

<400> 8

Met Ala Leu Pro Val Thr Ala Leu Leu Leu Pro Leu Ala Leu Leu Leu

1 5 10 15

His Ala Ala Arg Pro Ile Gln Met Thr Gln Thr Thr Ser Ser Leu Ser

20 25 30

Ala Ser Leu Gly Asp Arg Val Thr Ile Ser Cys Arg Ala Ser Gln Asp

35 40 45

Ile Ser Asn Phe Leu Asn Trp Tyr Gln Gln Lys Pro Asp Gly Thr Val

50 55 60

Lys Leu Leu Ile Tyr Tyr Thr Ser Arg Leu His Ser Gly Val Pro Ser

65 70 75 80

Arg Phe Ser Gly Ser Gly Ser Gly Thr Asp Phe Ser Leu Thr Ile Ser

85 90 95

Lys Leu Glu Gln Glu Asp Ile Ala Thr Tyr Phe Cys Gln Gln Gly Asn

100 105 110

Thr Leu Pro Leu Thr Phe Gly Ala Gly Thr Lys Leu Glu Leu Lys Gly

115 120 125

Gly Gly Gly Ser Gly Gly Gly Gly Ser Gly Gly Gly Gly Ser Leu Gln

130 135 140

Gln Ser Gly Pro Glu Leu Val Lys Pro Gly Ala Ser Val Lys Ile Ser

145 150 155 160

Cys Lys Ala Ser Gly Tyr Thr Phe Thr Asp Tyr Tyr Met Asn Trp Val

165 170 175

Lys Leu Ser His Gly Lys Ser Leu Glu Trp Ile Gly Asp Ile Val Pro

180 185 190

Asn Asn Gly Asp Thr Thr Tyr Asn Gln Asn Phe Arg Gly Lys Ala Thr

195 200 205

Leu Thr Val Asp Lys Ser Ser Ser Thr Ala Tyr Met Glu Leu Arg Ser

210 215 220

Leu Thr Ser Glu Asp Ser Ala Val Tyr Tyr Cys Ala Arg Phe Ser Asn

225 230 235 240

Tyr Val Tyr Pro Phe Asp Tyr Trp Gly Gln Gly Thr Thr Ile Thr Val

245 250 255

Ser Thr Thr Thr Pro Ala Pro Arg Pro Pro Thr Pro Ala Pro Thr Ile

260 265 270

Ala Ser Gln Pro Leu Ser Leu Arg Pro Glu Ala Cys Arg Pro Ala Ala

275 280 285

Gly Gly Ala Val His Thr Arg Gly Leu Asp Phe Ala Cys Asp Ile Tyr

290 295 300

Ile Trp Ala Pro Leu Ala Gly Thr Cys Gly Val Leu Leu Leu Ser Leu

305 310 315 320

Val Ile Thr Leu Tyr Cys Lys Arg Gly Arg Lys Lys Leu Leu Tyr Ile

325 330 335

Phe Lys Gln Pro Phe Met Arg Pro Val Gln Thr Thr Gln Glu Glu Asp

340 345 350

Gly Cys Ser Cys Arg Phe Pro Glu Glu Glu Glu Gly Gly Cys Glu Leu

355 360 365

Arg Val Lys Phe Ser Arg Ser Ala Asp Ala Pro Ala Tyr Gln Gln Gly

370 375 380

Gln Asn Gln Leu Tyr Asn Glu Leu Asn Leu Gly Arg Arg Glu Glu Tyr

385 390 395 400

Asp Val Leu Asp Lys Arg Arg Gly Arg Asp Pro Glu Met Gly Gly Lys

405 410 415

Pro Arg Arg Lys Asn Pro Gln Glu Gly Leu Tyr Asn Glu Leu Gln Lys

420 425 430

Asp Lys Met Ala Glu Ala Tyr Ser Glu Ile Gly Met Lys Gly Glu Arg

435 440 445

Arg Arg Gly Lys Gly His Asp Gly Leu Tyr Gln Gly Leu Ser Thr Ala

450 455 460

Thr Lys Asp Thr Tyr Asp Ala Leu His Met Gln Ala Leu Pro Pro Arg

465 470 475 480

<210> 9

<211> 480

<212> PRT

<213> Artificial sequence

<220>

<223> SEQ ID NO: 9

<400> 9

Met Ala Leu Pro Val Thr Ala Leu Leu Leu Pro Leu Ala Leu Leu Leu
1 5 10 15

His Ala Ala Arg Pro Ile Gln Met Thr Gln Thr Thr Ser Ser Leu Ser
20 25 30

Ala Ser Leu Gly Asp Arg Val Thr Ile Ser Cys Arg Ala Ser Gln Asp
35 40 45

Ile Ser Asn Phe Leu Asn Trp Tyr Gln Gln Lys Pro Asp Gly Thr Val
50 55 60

Lys Leu Leu Ile Tyr Ala Thr Ser Arg Leu His Ser Gly Val Pro Ser
65 70 75 80

Arg Phe Ser Gly Ser Gly Ser Gly Thr Asp Phe Ser Leu Thr Ile Ser
85 90 95

Lys Leu Glu Gln Glu Asp Ile Ala Thr Tyr Phe Cys Gln Gln Gly Asn
100 105 110

Thr Leu Pro Leu Thr Phe Gly Ala Gly Thr Lys Leu Glu Leu Lys Gly
115 120 125

Gly Gly Gly Ser Gly Gly Gly Gly Ser Gly Gly Gly Gly Ser Leu Gln
130 135 140

Gln Ser Gly Pro Glu Leu Val Lys Pro Gly Ala Ser Val Lys Ile Ser
145 150 155 160

Cys Lys Ala Ser Gly Tyr Thr Phe Thr Asp Tyr Tyr Met Asn Trp Val

165 170 175

Lys Leu Ser His Gly Lys Ser Leu Glu Trp Ile Gly Asp Ile Val Pro

180 185 190

Asn Asn Gly Asp Thr Thr Tyr Asn Gln Asn Phe Arg Gly Lys Ala Thr

195 200 205

Leu Thr Val Asp Lys Ser Ser Ser Thr Ala Tyr Met Glu Leu Arg Ser

210 215 220

Leu Thr Ser Glu Asp Ser Ala Val Tyr Tyr Cys Ala Arg Phe Ser Asn

225 230 235 240

Tyr Val Tyr Pro Phe Asp Tyr Trp Gly Gln Gly Thr Thr Ile Thr Val

245 250 255

Ser Thr Thr Thr Pro Ala Pro Arg Pro Pro Thr Pro Ala Pro Thr Ile

260 265 270

Ala Ser Gln Pro Leu Ser Leu Arg Pro Glu Ala Cys Arg Pro Ala Ala

275 280 285

Gly Gly Ala Val His Thr Arg Gly Leu Asp Phe Ala Cys Asp Ile Tyr

290 295 300

Ile Trp Ala Pro Leu Ala Gly Thr Cys Gly Val Leu Leu Leu Ser Leu

305 310 315 320

Val Ile Thr Leu Tyr Cys Lys Arg Gly Arg Lys Lys Leu Leu Tyr Ile

325 330 335

Phe Lys Gln Pro Phe Met Arg Pro Val Gln Thr Thr Gln Glu Glu Asp

340 345 350

Gly Cys Ser Cys Arg Phe Pro Glu Glu Glu Glu Gly Gly Cys Glu Leu

355 360 365

Arg Val Lys Phe Ser Arg Ser Ala Asp Ala Pro Ala Tyr Gln Gln Gly

370 375 380

Gln Asn Gln Leu Tyr Asn Glu Leu Asn Leu Gly Arg Arg Glu Glu Tyr

385 390 395 400

Asp Val Leu Asp Lys Arg Arg Gly Arg Asp Pro Glu Met Gly Gly Lys

405 410 415

Pro Arg Arg Lys Asn Pro Gln Glu Gly Leu Tyr Asn Glu Leu Gln Lys

420 425 430

Asp Lys Met Ala Glu Ala Tyr Ser Glu Ile Gly Met Lys Gly Glu Arg

435 440 445

Arg Arg Gly Lys Gly His Asp Gly Leu Tyr Gln Gly Leu Ser Thr Ala

450 455 460

Thr Lys Asp Thr Tyr Asp Ala Leu His Met Gln Ala Leu Pro Pro Arg

465 470 475 480

<210> 10

<211> 1440

<212> DNA

<213> Artificial sequence

<220>

<223> SEQ ID NO: 10

<400> 10

atggccctgc ctgtgacagc cctgctgctg cctctggctc tgctgctgca tgccgctaga 60

cccatccaga tgacacagac tacatcctcc ctgtctgcct ctctgggaga cagagtcacc 120

atcagttgca gggcaagtca ggacattagc aattttttaa actggtatca gcagaaacca 180

gatggaactg ttaaactcct gatctactac acatcaagat tacattctgg agtcccatca 240

aggttcagtg gcagtgggtc tggaacagat ttttctctca ccattagcaa actggagcaa 300

gaagatattg ccacttactt ttgccaacag ggtaatacgc ttccacttac gttcggcgct 360

gggacaaagt tggaacttaa aggtggtggt ggttctggcg gcggcggctc cggaggagga 420

ggatcgctgc aacagtctgg acctgagttg gtgaagcctg gggcttcagt gaagatttcc 480

tgcaaggctt ctggatacac attcactgac tactacatga attgggtgaa gcttagccat 540

ggaaagagcc ttgagtggat tggagatatt gttcctaaca atggtgatac tacttacaac 600

cagaatttca gaggcaaggc cacattgact gtagacaagt cctccagcac agcctacatg 660

gagctccgca gcctgacatc tgaggactct gcagtctatt actgtgcaag attcagtaat 720
tacgtttacc cttttgacta ctggggccaa ggcaccacta tcacagtctc caccacgacg 780
ccagcgccgc gaccaccaac accggcgccc accatcgcgt cgcagcccct gtccctgcgc 840
ccagaggcgt gccggccagc ggcggggggc gcagtgcaca cgagggggct ggacttcgcc 900
tgtgatatct acatctgggc gcccttggcc gggacttgtg gggtccttct cctgtcactg 960
gttatcaccc tttactgcaa acggggcaga aagaaactcc tgtatatatt caaacaacca 1020
tttatgagac cagtacaaac tactcaagag gaagatggct gtagctgccg atttccagaa 1080
gaagaagaag gaggatgtga actgagagtg aagttcagca ggagcgcaga cgcccccgcg 1140
taccagcagg gccagaacca gctctataac gagctcaatc taggacgaag agaggagtac 1200
gatgttttgg acaagagacg tggccgggac cctgagatgg ggggaaagcc gagaaggaag 1260
aaccctcagg aaggcctgta caatgaactg cagaaagata agatggcgga ggcctacagt 1320
gagattggga tgaaaggcga gcgccggagg ggcaaggggc acgatggcct ttaccagggt 1380
ctcagtacag ccaccaagga cacctacgac gcccttcaca tgcaggccct gccccctcgc 1440

<210> 11
<211> 1440
<212> DNA
<213> Artificial sequence

<220>
<223> SEQ ID NO: 11

<400> 11
atggccctgc ctgtgacagc cctgctgctg cctctggctc tgctgctgca tgccgctaga 60
cccatccaga tgacacagac tacatcctcc ctgtctgcct ctctgggaga cagagtcacc 120
atcagttgca gggcaagtca ggacattagc aattttttaa actggtatca gcagaaacca 180
gatggaactg ttaaactcct gatctacgcc acatcaagat tacattctgg agtcccatca 240
aggttcagtg gcagtgggtc tggaacagat ttttctctca ccattagcaa actggagcaa 300
gaagatattg ccacttactt ttgccaacag ggtaatacgc ttccacttac gttcggcgct 360
gggacaaagt tggaacttaa aggtggtggt ggttctggcg gcggcggctc cggaggagga 420

ggatcgctgc aacagtctgg acctgagttg gtgaagcctg gggcttcagt gaagatttcc 480
tgcaaggctt ctggatacac attcactgac tactacatga attgggtgaa gcttagccat 540
ggaaagagcc ttgagtggat tggagatatt gttcctaaca atggtgatac tacttacaac 600
cagaatttca gaggcaaggc cacattgact gtagacaagt cctccagcac agcctacatg 660
gagctccgca gcctgacatc tgaggactct gcagtctatt actgtgcaag attcagtaat 720
tacgtttacc cttttgacta ctggggccaa ggcaccacta tcacagtctc caccacgacg 780
ccagcgccgc gaccaccaac accggcgccc accatcgcgt cgcagcccct gtccctgcgc 840
ccagaggcgt gccggccagc ggcggggggc gcagtgcaca cgagggggct ggacttcgcc 900
tgtgatatct acatctgggc gcccttggcc gggacttgtg gggtccttct cctgtcactg 960
gttatcaccc tttactgcaa acggggcaga aagaaactcc tgtatatatt caaacaacca 1020
tttatgagac cagtacaaac tactcaagag gaagatggct gtagctgccg atttccagaa 1080
gaagaagaag gaggatgtga actgagagtg aagttcagca ggagcgcaga cgcccccgcg 1140
taccagcagg gccagaacca gctctataac gagctcaatc taggacgaag agaggagtac 1200
gatgttttgg acaagagacg tggccgggac cctgagatgg ggggaaagcc gagaaggaag 1260
aaccctcagg aaggcctgta caatgaactg cagaaagata agatggcgga ggcctacagt 1320
gagattggga tgaaaggcga gcgccggagg ggcaaggggc acgatggcct ttaccagggt 1380
ctcagtacag ccaccaagga cacctacgac gcccttcaca tgcaggccct gccccctcgc 1440

<210> 12
<211> 1086
<212> DNA
<213> Artificial sequence

<220>
<223> SEQ ID NO: 12

<400> 12
tctagaatgt tgctgcttgt aacttctctc cttctttgcg agttgcccca tcctgcgttc 60
ctccttattc ccaggaaggt atgcaatggg atcggtatag gagagttcaa ggattccctt 120
tctatcaacg ctacgaatat aaagcacttc aagaactgta cgtccatcag tggagacctg 180

catatattgc cggtggcgtt ccgaggggac agttttaccc acacgccccc tctcgaccca 240
caggagctgg atatcttgaa gaccgtgaag gagataactg gctttcttct cattcaggcg 300
tggccggaaa ataggacaga cttgcacgcc tttgaaaact tggaaattat acgagggcgg 360
acaaaacaac acggtcaatt cagcctggcc gttgtatccc tcaatatcac tagcttgggt 420
ctccgaagtc tgaaagaaat aagtgacggg gacgttataa tttctgggaa caagaacctc 480
tgctacgcaa acacaataaa ctggaaaaaa ttgtttggaa ctagcgggca gaaaactaag 540
atcattagta acagaggcga gaatagttgc aaagccaccg gacaagtgtg ccatgcactt 600
tgcagccccg agggttgttg gggccctgaa ccacgggatt gcgtgtcatg cagaaacgtc 660
tcacgaggtc gcgagtgtgt cgacaaatgt aacctgcttg aaggggagcc tcgcgaattc 720
gtagaaaaca gcgagtgcat tcaatgccac ccagagtgtc tcccccaggc catgaacatc 780
acctgtacag gacgggggcc agataactgt attcaatgcg cacactatat agatggacca 840
cattgtgtga aaacatgtcc cgcaggggtc atgggtgaga acaacacgct cgtttggaaa 900
tatgcagatg ccgggcatgt atgccacctc tgtcacccga actgcactta tgggtgcact 960
gggcccggcc tggaaggatg ccccaccaac ggacccaaga ttccctccat agcgaccgga 1020
atggttggag ccttgcttct tcttctggta gtggcgctcg ggatcgggtt gttcatgtaa 1080
ggatcc 1086

<210> 13
<211> 8431
<212> DNA
<213> Artificial sequence

<220>
<223> SEQ ID NO: 13

<400> 13
agcttaatgt agtcttatgc aatactcttg tagtcttgca acatggtaac gatgagttag 60
caacatgcct tacaaggaga gaaaaagcac cgtgcatgcc gattggtgga agtaaggtgg 120
tacgatcgtg ccttattagg aaggcaacag acgggtctga catggattgg acgaaccact 180
gaattgccgc attgcagaga tattgtattt aagtgcctag ctcgatacat aaacgggtct 240

ctctggttag accagatctg agcctgggag ctctctggct aactagggaa cccactgctt 300
aagcctcaat aaagcttgcc ttgagtgctt caagtagtgt gtgcccgtct gttgtgtgac 360
tctggtaact agagatccct cagacccttt tagtcagtgt ggaaaatctc tagcagtggc 420
gcccgaacag ggacttgaaa gcgaaaggga aaccagagga gctctctcga cgcaggactc 480
ggcttgctga agcgcgcacg gcaagaggcg aggggcggcg actggtgagt acgccaaaaa 540
ttttgactag cggaggctag aaggagagag atgggtgcga gagcgtcagt attaagcggg 600
ggagaattag atcgcgatgg gaaaaaattc ggttaaggcc agggggaaag aaaaaatata 660
aattaaaaca tatagtatgg gcaagcaggg agctagaacg attcgcagtt aatcctggcc 720
tgttagaaac atcagaaggc tgtagacaaa tactgggaca gctacaacca tcccttcaga 780
caggatcaga agaacttaga tcattatata atacagtagc aaccctctat tgtgtgcatc 840
aaaggataga gataaaagac accaaggaag ctttagacaa gatagaggaa gagcaaaaca 900
aaagtaagac caccgcacag caagcggccg ctgatcttca gacctggagg aggagatatg 960
agggacaatt ggagaagtga attatataaa tataaagtag taaaaattga accattagga 1020
gtagcaccca ccaaggcaaa gagaagagtg gtgcagagag aaaaaagagc agtgggaata 1080
ggagctttgt tccttgggtt cttgggagca gcaggaagca ctatgggcgc agcgtcaatg 1140
acgctgacgg tacaggccag acaattattg tctggtatag tgcagcagca gaacaatttg 1200
ctgagggcta ttgaggcgca acagcatctg ttgcaactca cagtctgggg catcaagcag 1260
ctccaggcaa gaatcctggc tgtggaaaga tacctaaagg atcaacagct cctggggatt 1320
tggggttgct ctggaaaact catttgcacc actgctgtgc cttggaatgc tagttggagt 1380
aataaatctc tggaacagat ttggaatcac acgacctgga tggagtggga cagagaaatt 1440
aacaattaca caagcttaat acactcctta attgaagaat cgcaaaacca gcaagaaaag 1500
aatgaacaag aattattgga attagataaa tgggcaagtt tgtggaattg gtttaacata 1560
acaaattggc tgtggtatat aaaattattc ataatgatag taggaggctt ggtaggttta 1620
agaatagttt ttgctgtact ttctatagtg aatagagtta ggcagggata ttcaccatta 1680
tcgtttcaga cccacctccc aaccccgagg ggacccgaca ggcccgaagg aatagaagaa 1740
gaaggtggag agagagacag agacagatcc attcgattag tgaacggatc tcgacggtat 1800
cggttaactt ttaaaagaaa aggggggatt ggggggtaca gtgcagggga aagaatagta 1860
gacataatag caacagacat acaaactaaa gaattacaaa aacaaattac aaaaattcaa 1920
aattttatcg atcacgagac tagcctcgag aagcttgata tcgaattcca ccgtgaggct 1980
ccggtgcccg tcagtgggca gagcgcacat cgcccacagt ccccgagaag ttggggggag 2040

gggtcggcaa ttgaaccggt gcctagagaa ggtggcgcgg ggtaaactgg gaaagtgatg 2100
tcgtgtactg gctccgcctt tttcccgagg gtgggggaga accgtatata agtgcagtag 2160
tcgccgtgaa cgttcttttt cgcaacgggt ttgccgccag aacacaggta agtgccgtgt 2220
gtggttcccg cgggcctggc ctctttacgg gttatggccc ttgcgtgcct tgaattactt 2280
ccacctggct gcagtacgtg attcttgatc ccgagcttcg ggttggaagt gggtgggaga 2340
gttcgaggcc ttgcgcttaa ggagcccctt cgcctcgtgc ttgagttgag gcctggcctg 2400
ggcgctgggg ccgccgcgtg cgaatctggt ggcaccttcg cgcctgtctc gctgctttcg 2460
ataagtctct agccatttaa aatttttgat gacctgctgc gacgcttttt ttctggcaag 2520
atagtcttgt aaatgcgggc caagatctgc acactggtat ttcggttttt ggggccgcgg 2580
gcggcgacgg ggcccgtgcg tcccagcgca catgttcggc gaggcggggc ctgcgagcgc 2640
ggccaccgag aatcggacgg gggtagtctc aagctggccg gcctgctctg gtgcctggcc 2700
tcgcgccgcc gtgtatcgcc ccgccctggg cggcaaggct ggcccggtcg gcaccagttg 2760
cgtgagcgga aagatggccg cttcccggcc ctgctgcagg gagctcaaaa tggaggacgc 2820
ggcgctcggg agagcgggcg ggtgagtcac ccacacaaag gaaaagggcc tttccgtcct 2880
cagccgtcgc ttcatgtgac tccacggagt accgggcgcc gtccaggcac ctcgattagt 2940
tctcgagctt ttggagtacg tcgtctttag gttgggggga ggggttttat gcgatggagt 3000
ttccccacac tgagtgggtg gagactgaag ttaggccagc ttggcacttg atgtaattct 3060
ccttggaatt tgcccttttt gagtttggat cttggttcat tctcaagcct cagacagtgg 3120
ttcaaagttt ttttcttcca tttcaggtgt cgtgagctag cactagttct agaatgttgc 3180
tgcttgtaac ttctctcctt ctttgcgagt tgccccatcc tgcgttcctc cttattccca 3240
ggaaggtatg caatgggatc ggtataggag agttcaagga ttccctttct atcaacgcta 3300
cgaatataaa gcacttcaag aactgtacgt ccatcagtgg agacctgcat atattgccgg 3360
tggcgttccg aggggacagt tttacccaca cgcccccctct cgacccacag gagctggata 3420
tcttgaagac cgtgaaggag ataactggct ttcttctcat tcaggcgtgg ccggaaaata 3480
ggacagactt gcacgccttt gaaaacttgg aaattatacg agggcggaca aaacaacacg 3540
gtcaattcag cctggccgtt gtatccctca atatcactag cttgggtctc cgaagtctga 3600
aagaaataag tgacggggac gttataattt ctgggaacaa gaacctctgc tacgcaaaca 3660
caataaactg gaaaaaattg tttggaacta gcgggcagaa aactaagatc attagtaaca 3720
gaggcgagaa tagttgcaaa gccaccggac aagtgtgcca tgcactttgc agccccgagg 3780
gttgttgggg ccctgaacca cgggattgcg tgtcatgcag aaacgtctca cgaggtcgcg 3840

agtgtgtcga caaatgtaac ctgcttgaag gggagcctcg cgaattcgta gaaaacagcg 3900

agtgcattca atgccaccca gagtgtctcc cccaggccat gaacatcacc tgtacaggac 3960

gggggccaga taactgtatt caatgcgcac actatataga tggaccacat tgtgtgaaaa 4020

catgtcccgc aggggtcatg ggtgagaaca acacgctcgt ttggaaatat gcagatgccg 4080

ggcatgtatg ccacctctgt cacccgaact gcacttatgg gtgcactggg cccggcctgg 4140

aaggatgccc caccaacgga cccaagattc cctccatagc gaccggaatg gttggagcct 4200

tgcttcttct tctggtagtg gcgctcggga tcgggttgtt catgtaagga tccaccggtc 4260

gccaccagcg gccgcgtcga caatcaacct ctggattaca aaatttgtga aagattgact 4320

ggtattctta actatgttgc tccttttacg ctatgtggat acgctgcttt aatgcctttg 4380

tatcatgcta ttgcttcccg tatggctttc attttctcct ccttgtataa atcctggttg 4440

ctgtctcttt atgaggagtt gtggcccgtt gtcaggcaac gtggcgtggt gtgcactgtg 4500

tttgctgacg caacccccac tggttggggc attgccacca cctgtcagct cctttccggg 4560

actttcgctt tccccctccc tattgccacg gcggaactca tcgccgcctg ccttgcccgc 4620

tgctggacag gggctcggct gttgggcact gacaattccg tggtgttgtc ggggaagctg 4680

acgtcctttc catggctgct cgcctgtgtt gccacctgga ttctgcgcgg gacgtccttc 4740

tgctacgtcc cttcggccct caatccagcg gaccttcctt cccgcggcct gctgccggct 4800

ctgcggcctc ttccgcgtct tcgccttcgc cctcagacga gtcggatctc cctttgggcc 4860

gcctccccgc ctggaattcg agctcggtac ctttaagacc aatgacttac aaggcagctg 4920

tagatcttag ccactttta aaagaaaagg ggggactgga agggctaatt cactcccaac 4980

gaagacaaga tctgcttttt gcttgtactg ggtctctctg gttagaccag atctgagcct 5040

gggagctctc tggctaacta gggaacccac tgcttaagcc tcaataaagc ttgccttgag 5100

tgcttcaagt agtgtgtgcc cgtctgttgt gtgactctgg taactagaga tccctcagac 5160

ccttttagtc agtgtggaaa atctctagca gtagtagttc atgtcatctt attattcagt 5220

atttataact tgcaaagaaa tgaatatcag agagtgagag gaacttgttt attgcagctt 5280

ataatggtta caaataaagc aatagcatca caaatttcac aaataaagca tttttttcac 5340

tgcattctag ttgtggtttg tccaaactca tcaatgtatc ttatcatgtc tggctctagc 5400

tatcccgccc ctaactccgc ccatcccgcc cctaactccg cccagttccg cccattctcc 5460

gccccatggc tgactaattt tttttattta tgcagaggcc gaggccgcct cggcctctga 5520

gctattccag aagtagtgag gaggcttttt tggaggccta gggacgtacc caattcgccc 5580

tatagtgagt cgtattacgc gcgctcactg gccgtcgttt tacaacgtcg tgactgggaa 5640

aaccctggcg ttacccaact taatcgcctt gcagcacatc ccccttttcgc cagctggcgt 5700
aatagcgaag aggcccgcac cgatcgccct tcccaacagt tgcgcagcct gaatggcgaa 5760
tgggacgcgc cctgtagcgg cgcattaagc gcggcgggtg tggtggttac gcgcagcgtg 5820
accgctacac ttgccagcgc cctagcgccc gctcctttcg ctttcttccc ttcctttctc 5880
gccacgttcg ccggctttcc ccgtcaagct ctaaatcggg ggctcccttt agggttccga 5940
tttagtgctt tacggcacct cgaccccaaa aaacttgatt agggtgatgg ttcacgtagt 6000
gggccatcgc cctgatagac ggtttttcgc cctttgacgt tggagtccac gttctttaat 6060
agtggactct tgttccaaac tggaacaaca ctcaacccta tctcggtcta ttcttttgat 6120
ttataaggga ttttgccgat ttcggcctat tggttaaaaa atgagctgat ttaacaaaaa 6180
tttaacgcga attttaacaa aatattaacg cttacaattt aggtggcact tttcggggaa 6240
atgtgcgcgg aacccctatt tgtttatttt tctaaataca ttcaaatatg tatccgctca 6300
tgagacaata accctgataa atgcttcaat aatattgaaa aaggaagagt atgagtattc 6360
aacatttccg tgtcgccctt attccctttt ttgcggcatt ttgccttcct gtttttgctc 6420
acccagaaac gctggtgaaa gtaaaagatg ctgaagatca gttgggtgca cgagtgggtt 6480
acatcgaact ggatctcaac agcggtaaga tccttgagag ttttcgcccc gaagaacgtt 6540
ttccaatgat gagcactttt aaagttctgc tatgtggcgc ggtattatcc cgtattgacg 6600
ccgggcaaga gcaactcggt cgccgcatac actattctca gaatgacttg gttgagtact 6660
caccagtcac agaaaagcat cttacggatg gcatgacagt aagagaatta tgcagtgctg 6720
ccataaccat gagtgataac actgcggcca acttacttct gacaacgatc ggaggaccga 6780
aggagctaac cgcttttttg cacaacatgg gggatcatgt aactcgcctt gatcgttggg 6840
aaccggagct gaatgaagcc ataccaaacg acgagcgtga caccacgatg cctgtagcaa 6900
tggcaacaac gttgcgcaaa ctattaactg gcgaactact tactctagct tcccggcaac 6960
aattaataga ctggatggag gcggataaag ttgcaggacc acttctgcgc tcggcccttc 7020
cggctggctg gtttattgct gataaatctg gagccggtga gcgtgggtct cgcggtatca 7080
ttgcagcact ggggccagat ggtaagccct cccgtatcgt agttatctac acgacgggga 7140
gtcaggcaac tatggatgaa cgaaatagac agatcgctga gataggtgcc tcactgatta 7200
agcattggta actgtcagac caagtttact catatatact ttagattgat ttaaaacttc 7260
atttttaatt taaaaggatc taggtgaaga tcctttttga taatctcatg accaaaatcc 7320
cttaacgtga gttttcgttc cactgagcgt cagaccccgt agaaaagatc aaaggatctt 7380
cttgagatcc tttttttctg cgcgtaatct gctgcttgca aacaaaaaaa ccaccgctac 7440

cagcggtggt ttgtttgccg gatcaagagc taccaactct ttttccgaag gtaactggct 7500
tcagcagagc gcagatacca aatactgttc ttctagtgta gccgtagtta ggccaccact 7560
tcaagaactc tgtagcaccg cctacatacc tcgctctgct aatcctgtta ccagtggctg 7620
ctgccagtgg cgataagtcg tgtcttaccg ggttggactc aagacgatag ttaccggata 7680
aggcgcagcg gtcgggctga acggggggtt cgtgcacaca gcccagcttg gagcgaacga 7740
cctacaccga actgagatac ctacagcgtg agctatgaga aagcgccacg cttcccgaag 7800
ggagaaaggc ggacaggtat ccggtaagcg gcagggtcgg aacaggagag cgcacgaggg 7860
agcttccagg gggaaacgcc tggtatcttt atagtcctgt cgggtttcgc cacctctgac 7920
ttgagcgtcg atttttgtga tgctcgtcag gggggcggag cctatggaaa aacgccagca 7980
acgcggcctt tttacggttc ctggcctttt gctggccttt tgctcacatg ttctttcctg 8040
cgttatcccc tgattctgtg gataaccgta ttaccgcctt tgagtgagct gataccgctc 8100
gccgcagccg aacgaccgag cgcagcgagt cagtgagcga ggaagcggaa gagcgcccaa 8160
tacgcaaacc gcctctcccc gcgcgttggc cgattcatta atgcagctgg cacgacaggt 8220
ttcccgactg gaaagcgggc agtgagcgca acgcaattaa tgtgagttag ctcactcatt 8280
aggcacccca ggctttacac tttatgcttc cggctcgtat gttgtgtgga attgtgagcg 8340
gataacaatt tcacacagga aacagctatg accatgatta cgccaagcgc gcaattaacc 8400
ctcactaaag ggaacaaaag ctggagctgc a 8431